# EUROPEAN PATENT APPLICATION

(11) **EP 4 810 493 A1**
(43) Date of publication of application: **23.09.2026**
(21) Application number: 24890827.9
(22) Date of filing: 15.11.2024
(51) Int. Cl.: C07D 471/04, A61K 31/519, A61P 25/16, A61P 25/18, A61P 25/28, A61P 25/14

(54) **HETEROCYCLIC CARBONYL DERIVATIVE MODULATOR, PREPARATION METHOD THEREFOR, AND USE THEREOF**

(30) Priority: 17.11.2023 CN 202311545334; 19.12.2023 CN 202311762719; 16.01.2024 CN 202410062656; 06.02.2024 CN 202410173024; 01.03.2024 CN 202410239475; 26.04.2024 CN 202410520784; 21.06.2024 CN 202410817211; 19.07.2024 CN 202410980405; 10.09.2024 CN 202411268934; 17.10.2024 CN 202411458343
(71) Applicant: Shanghai Hansoh Biomedical Co., Ltd., Shanghai 201203 (CN); Jiangsu Hansoh Pharmaceutical Group Co., Ltd., Lianyungang, Jiangsu 222069 (CN)
(72) Inventor: XIAO, Hualing, Shanghai 201203 (CN); CHENG, Bao, Shanghai 201203 (CN); LIU, Yong, Shanghai 201203 (CN); FAN, Bobo, Shanghai 201203 (CN); SU, Yidong, Shanghai 201203 (CN)
(74) Representative: De Clercq & Partners
(86) International application number: PCT/CN2024/132360
(87) International publication number: WO 2025/103475

(57) **Abstract**

The present invention relates to a heterocyclic carbonyl derivative modulator, a preparation method therefor, and the use thereof. Specifically, the present invention relates to a compound represented by general formula (II-B), a preparation method therefor, a pharmaceutical composition containing said compound, and a use thereof as a modulator in the treatment of Alzheimer's disease, schizophrenia, pain, addiction, and sleep disorders, wherein each substituent in general formula (II-B) is as defined in the description.

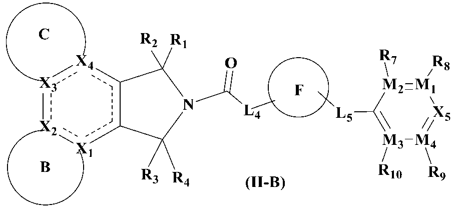

## Description

### Technical Field

The present invention belongs to the field of biomedicine, and specifically relates to a heterocyclic carbonyl derivative modulator, and a preparation method therefor and the use thereof.

### Background Art

The objective of the present invention is to provide compounds that are useful for treating or preventing such diseases and to improve therapeutic properties.

Schizophrenia is a severe, complex, and debilitating mental health disorder characterised by a range of symptoms, including delusions, hallucinations, disorganised speech or behaviour, slowed speech, and blunted affect. Schizophrenia is also often accompanied by severe cognitive impairments, which further limit the patient's ability to work and maintain interpersonal relationships. According to statistics from the World Health Organisation, schizophrenia affects more than 23 million people worldwide, with a lifetime prevalence of 3.8‰-8.4‰. Onset is most common in late adolescence, and the main challenges in treatment are low medication adherence and high relapse rates. Only 20% of patients report good treatment outcomes. Medication adherence is poor, with an adherence rate of approximately 60% within 18 months and a discontinuation rate of 74%. Among patients who discontinue treatment, the relapse rate is as high as 77% at one year and 90% at two years. Compared with the general population, the life expectancy of patients with schizophrenia is reduced by 10 to 25 years.

Muscarinic receptors, which belong to the acetylcholine receptor family, are typical GPCRs and have five subtypes (M1-5). Among them, the G protein-coupled M4 subtype is mainly expressed in the cerebral cortex, striatum, hypothalamus, and hippocampus. A 2018 genome-wide association study on schizophrenia published in *Nature genetics* (11,260 cases and 24,542 controls) showed a significant correlation between mutations in the M4 gene and the disease.

Cholinergic neuronal transmission is crucial for cognitive function, and its receptor antagonists can cause severe memory impairment. In schizophrenia, excessively high dopamine levels in the striatum and nucleus accumbens are thought to be associated with the disease; therefore, blocking the dopamine D2 receptor is the current effective treatment strategy. Some newly developed M4-specific positive allosteric modulators (PAMs) enhance the action of the endogenous agonist acetylcholine, revealing that muscarinic acetylcholine receptors are involved in controlling key cognitive synapses in the hippocampus and dopamine release in the striatum.

In recent studies, M4 PAMs have been shown to reduce striatal dopamine release in wild-type mice following amphetamine treatment. Further in vivo studies demonstrated that the M4 PAM VU0467154 improved learning deficits induced by the NMDA receptor antagonist MK-801.

The PAM CVL-231 targeting the M4 receptor is currently in Phase II clinical trials, and its Phase Ib clinical trial showed overall good tolerability. After six weeks of dosing, both the 30 mg once-daily and the 20 mg twice-daily regimens demonstrated significant antipsychotic activity. Compared with the placebo group, the once-daily 30 mg group showed a mean reduction of 19.5 points in the Positive and Negative Syndrome Scale (PANSS) total score from baseline, while the twice-daily 20 mg group showed a mean reduction of 17.9 points from baseline, which were both statistically and clinically significant.

Activating M4 receptors in the striatum and hippocampus with specific PAMs can significantly improve the hyperdopaminergic state in the striatum and the hyperactive state in the hippocampus, thereby providing treatment for the psychiatric symptoms and cognitive impairments associated with schizophrenia. Therefore, modulating M4 receptor activity is an effective strategy for treating or preventing schizophrenia mediated by M4 dysregulation.

### Summary of the Invention

In another aspect, the present invention provides a compound represented by general formula (II-A1) or (II-A2), or a stereoisomer or pharmaceutically acceptable salt thereof: 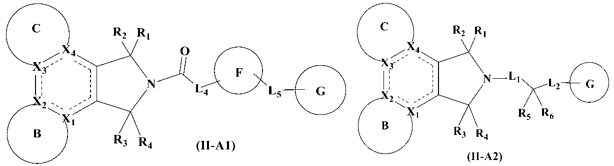 wherein:
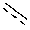 is a single bond or a double bond;
each X₁ is independently selected from a bond, C, CR₁₁, CR₁₁R₂₂ or N;
each X₂ is independently selected from a bond, C, CR₂₂, CR₁₁R₂₂ or N;
each X₃ is independently selected from a bond, C, CR₃₃, CR₁₁R₂₂ or N;
each X₄ is independently selected from a bond, C, CR₄₄, CR₁₁R₂₂ or N;
each L₄ or L₅ is independently selected from a bond, C(R₅R₆), N(R₅), O or S;
L₁ is selected from C(CF₃)R₆₆, C(O), C(=S), C(=NR₆₆) or S(O)ₙ₂;
L₂ is selected from C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene;
each R₁₁, R₂₂, R₃₃, R₄₄ or R₆₆ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
ring B or ring C is present or absent, and when present, each is independently selected from cycloalkyl, heterocyclyl, aryl or heteroaryl, wherein the cycloalkyl, heterocyclyl, aryl or heteroaryl is optionally substituted with one or more substituents selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
at least one of ring B and ring C is present;
each ring F or ring G is independently selected from cycloalkyl, heterocyclyl, aryl or heteroaryl, wherein the cycloalkyl, heterocyclyl, aryl or heteroaryl may optionally be further substituted with a substituent;
each R₁, R₂, R₃, R₄, R₅ or R₆ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, oxo, thio, halogen, amino, hydroxyl, cyano, nitro, alkyl, alkenyl, alkynyl, deuteroalkyl, haloalkyl, alkoxy, haloalkoxy, hydroxyalkyl or cyano-substituted alkyl;
alternatively, R₁ and R₂, R₃ and R₄, or R₅ and R₆, together with the carbon atom to which they are each attached, form cycloalkyl or heterocyclyl, wherein the cycloalkyl or heterocyclyl is optionally substituted with one or more substituents selected from deuterium, oxo, thio, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
n2 is 1 or 2.
In a preferred embodiment of the present invention,
X₁ is selected from C, CR₁₁ or N;
X₂ is selected from C, CR₂₂ or N;
X₃ is selected from C, CR₃₃ or N;
X₄ is selected from C, CR₄₄ or N.

In a preferred embodiment of the present invention,
each ring F or ring G is independently selected from cycloalkyl, heterocyclyl, aryl or heteroaryl, wherein the cycloalkyl, heterocyclyl, aryl or heteroaryl may optionally be further substituted with one or more substituents selected from R₅₅, R₇, R₈, R₉ or R₁₀;
each R₅₅, R₇, R₈, R₉ or R₁₀ is independently selected from hydrogen, deuterium, halogen, amino, hydroxyl, cyano, nitro, alkyl, alkenyl, alkynyl, deuteroalkyl, haloalkyl, alkoxy, haloalkoxy, deuterated alkoxy, hydroxyalkyl, cyano-substituted alkyl, cycloalkyl, heterocyclyl, aryl, heteroaryl, -O(CRₐₐR_{bb})ₙ₁R₃₁, -N(CRₐₐR_{bb})ₙ₁R₃₁, -S(CRₐₐR_{bb})ₙ₁R₃₁, -OCR₃₁R₃₂R₃₃, -(CRₐₐR_{bb})ₙ₁C(O)R₃₁, N=S=OR₃₁R₃₂, N(R₃₁)=S=OR₃₂-, -(CH₂)ₐ₁C(O)NR₃₁R₃₂, -(CH₂)ₙ₁P(O)R₃₁R₃₂, -(CH₂)ₙ₁P(O)₂R₃₁R₃₂, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
each R₃₁, R₃₂, R₃₃, Rₐₐ, R_{bb} or R_{cc} is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
m1 is 0, 1 or 2;
n1 is 0, 1 or 2.

In a preferred embodiment of the present invention, the compound is further represented by general formula (II-B), (II-C), (II-D) or (II-E): 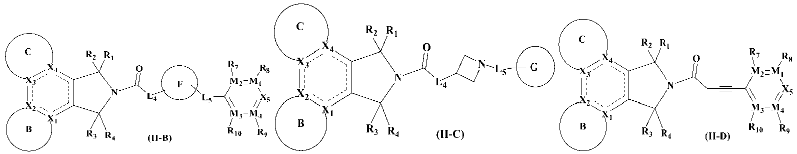 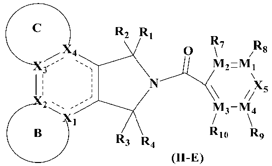
X₅ is selected from CR₅₅ or N;
each M₁, M₂, M₃ or M₄ is independently selected from C or N;
when M₁, M₂, M₃ or M₄ is N, R₇, R₈, R₉ or R₁₀ is absent; each R₅₅, R₇, R₈, R₉ or R₁₀ is independently selected from hydrogen, deuterium, halogen, amino, hydroxyl, cyano, nitro, alkyl, alkenyl, alkynyl, deuteroalkyl, haloalkyl, alkoxy, haloalkoxy, deuterated alkoxy, hydroxyalkyl, cyano-substituted alkyl, cycloalkyl, heterocyclyl, aryl, heteroaryl, -O(CRₐₐR_{bb})ₙ₁R₃₁, -N(CRₐₐR_{bb})ₙ₁R₃₁, - S(CRₐₐR_{bb})ₙ₁R₃₁, -OCR₃₁R₃₂R₃₃, -(CRₐₐR_{bb})ₙ₁C(O)R₃₁, N=S=OR₃₁R₃₂, N(R₃₁)=S=OR₃₂-, -(CH₂)ₙ₁C(O)NR₃₁R₃₂, -(CH₂)ₙ₁P(O)R₃₁R₃₂, -(CH₂)ₙ₁P(O)₂R₃₁R₃₂, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
each R₃₁, R₃₂, R₃₃, Rₐₐ, R_{bb} or R_{cc} is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
m1 is 0, 1 or 2;
n1 is 0, 1 or 2.

In certain embodiments of the present invention,
each R₅₅, R₇, R₈, R₉ or R₁₀ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl, heteroaryl, -(CRₐₐR_{bb})ₙ₁C(O)R₃₁, N=S=OR₃₁R₃₂, N(R₃₁)=S=OR₃₂-, -(CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl may optionally be further substituted with one or more substituents selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
each R₃₁, R₃₂, R₃₃, Rₐₐ, R_{bb} or R_{cc} is independently selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from hydrogen, deuterium, hydroxyl, halogen, cyano, amino, alkyl, haloalkyl or deuteroalkyl;
m1 is 0, 1 or 2;
n1 is 0, 1 or 2.

In certain embodiments of the present invention,
each X₅, M₁, M₂, M₃ or M₄ is independently selected from CR₅₅ or N;
each R₅₅, R₇, R₈, R₉ or R₁₀ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl, heteroaryl, -(CRₐₐR_{bb})ₙ₁C(O)R₃₁, N=S=OR₃₁R₃₂, N(R₃₁)=S=OR₃₂-, -(CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁(NR₃₁)C(O)R₃₂, - (CRₐₐR_{bb})ₙ₁S(O)(=NR_{cc})R₃₂ or -(CRₐₐR_{bb})ₙ₁S(O)ₘ₁R₃₂, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl may optionally be further substituted with one or more substituents selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
each R₃₁, R₃₂, Rₐₐ, R_{bb} or R_{cc} is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from hydrogen, deuterium, hydroxyl, halogen, cyano, amino, alkyl, haloalkyl or deuteroalkyl;
m1 is 0, 1 or 2;
n1 is 0, 1 or 2;
ring B, ring C, X₁-X₄, R₁, R₂, R₃, and R₄ are as defined in general formula (II-A1) or (II-A2).

In a further preferred embodiment of the present invention, the compound is not selected from the following structures:

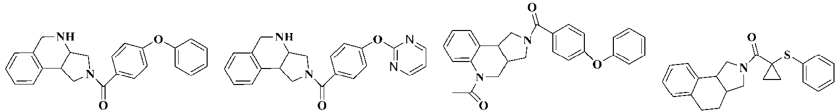

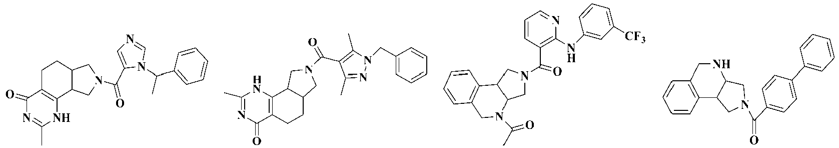

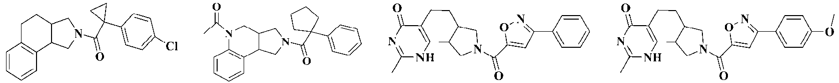

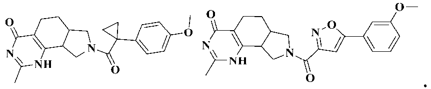

In certain embodiments of the present invention, the compound is further represented by general formula (II) or (II-1): 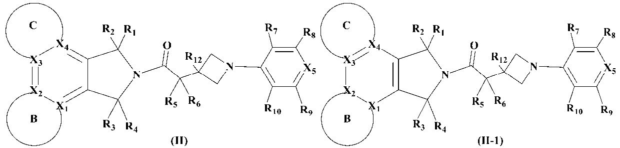 wherein:
each X₁ is independently selected from a bond, C, CR₁₁, CR₁₁R₂₂ or N;
each X₂ is independently selected from a bond, C, CR₂₂, CR₁₁R₂₂ or N;
each X₃ is independently selected from a bond, C, CR₃₃ or N;
each X₄ is independently selected from a bond, C, CR₄₄ or N;
each R₁₁, R₂₂, R₃₃, R₄₄, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, hydroxyl, halogen, cyano, amino, C₁₋₆ alkyl, C₁₋₆ haloalkyl or C₁₋₆ deuteroalkyl;
each R₅₅, R₇, R₈, R₉, R₁₀ or R₁₂ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, deuterated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -O(CRₐₐR_{bb})ₙ₁R₃₁, -N(CRₐₐR_{bb})ₙ₁R₃₁, -S(CRₐₐR_{bb})ₙ₁R₃₁, -OCR₃₁R₃₂R₃₃, - (CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, - (CH₂)ₙ₁P(O)₂R₃₁R₃₂, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
ring B or ring C is present or absent, and when present, each is independently selected from C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
at least one of ring B and ring C is present;
alternatively, R₁ and R₂, R₃ and R₄, or R₅ and R₆, together with the carbon atom to which they are each attached, form C₃₋₁₂ cycloalkyl or 3- to 12-membered heterocyclyl, wherein the C₃₋₁₂ cycloalkyl or 3- to 12-membered heterocyclyl is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
m1 is 0, 1 or 2; and
n1 is 0, 1 or 2.

In a preferred embodiment of the present invention,
each X₁ is independently selected from C, CR₁₁ or N;
each X₂ is independently selected from C, CR₂₂ or N;
each X₃ is independently selected from C, CR₃₃ or N;
each X₄ is independently selected from C, CR₄₄ or N.

In certain embodiments of the present invention,
each X₁ is independently selected from C, CR₁₁ or N;
each X₂ is independently selected from C, CR₂₂ or N;
each X₃ is independently selected from C, CR₃₃ or N;
each X₄ is independently selected from C, CR₄₄ or N;
each X₅ is independently selected from CR₅₅ or N;
each R₁₁, R₂₂, R₃₃, R₄₄, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, which is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
each R₅₅, R₇, R₈, R₉, R₁₀ or R₁₂ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁(NR₃₁)C(O)R₃₂, - (CRₐₐR_{bb})ₙ₁S(O)(=NR_{cc})R₃₂ or -(CRₐₐR_{bb})ₙ₁S(O)ₘ₁R₃₂, which may optionally be further substituted with one or more substituents selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
ring B or ring C is present or absent, and when present, each is independently selected from C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which may optionally be further substituted with one or more substituents selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
at least one of ring B and ring C is present;
alternatively, R₁ and R₂, R₃ and R₄, or R₅ and R₆, together with the carbon atom to which they are each attached, form C₃₋₁₂ cycloalkyl or 3- to 12-membered heterocyclyl, which may optionally be further substituted with one or more substituents selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₃₁ or R₃₂, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, which is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
m1 is 0, 1 or 2; and
n1 is 0, 1 or 2.

In a preferred embodiment of the present invention, the compound is further represented by general formula (II-A-1), (II-A-2) or (II-A-3): 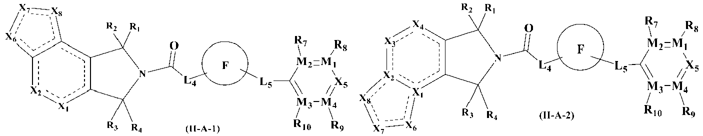 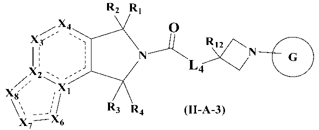 wherein:
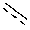 is a single bond or a double bond;
each X₁ is independently selected from a bond, C, CR₁₁, CR₁₁R₂₂ or N;
each X₂ is independently selected from a bond, C, CR₂₂, CR₁₁R₂₂ or N;
each X₃ is independently selected from a bond, C, CR₃₃, CR₁₁R₂₂ or N;
each X₄ is independently selected from a bond, C, CR₄₄, CR₁₁R₂₂ or N;
X₅ is selected from CR₅₅ or N;
each M₁, M₂, M₃ or M₄ is independently selected from C or N;
when M₁, M₂, M₃ or M₄ is N, R₇, R₈, R₉ or R₁₀ is absent;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, (CR₆₆)ₙ₂, N or O;
each L₄ or L₅ is independently selected from a bond, C(R₅R₆), N(R₅), O or S;
each R₁₁, R₂₂, R₃₃, R₄₄, R₆₆, R₇₇, R₈₈, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, hydroxyl, halogen, cyano, amino, C₁₋₆ alkyl, C₁₋₆ haloalkyl or C₁₋₆ deuteroalkyl;
each ring F is independently selected from C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
each ring G is independently selected from C₃₋₁₂ cycloalkyl, 3- to 12-membered heterocyclyl, C₆₋₁₂ aryl or 5- to 14-membered heteroaryl, wherein the C₃₋₁₂ cycloalkyl, 3- to 12-membered heterocyclyl, C₆₋₁₂ aryl or 5- to 14-membered heteroaryl may optionally be further substituted with one or more substituents selected from R₅₅, R₇, R₈, R₉ or R₁₀;
alternatively, R₁ and R₂, R₃ and R₄, or R₅ and R₆, together with the carbon atom to which they are each attached, form C₃₋₁₂ cycloalkyl or 3- to 12-membered heterocyclyl, wherein the C₃₋₁₂ cycloalkyl or 3- to 12-membered heterocyclyl is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₅₅, R₇, R₈, R₉, R₁₀ or R₁₂ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, deuterated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -O(CRₐₐR_{bb})ₙ₁R₃₁, -N(CRₐₐR_{bb})ₙ₁R₃₁, -S(CRₐₐR_{bb})ₙ₁R₃₁, -OCR₃₁R₃₂R₃₃, - (CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, - (CH₂)ₙ₁P(O)₂R₃₁R₃₂, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
m1 is 0, 1 or 2; and
n1 is 0, 1 or 2.

In a preferred embodiment of the present invention,
each X₁ is independently selected from C, CR₁₁ or N;
each X₂ is independently selected from C, CR₂₂ or N;
each X₃ is independently selected from C, CR₃₃ or N;
each X₄ is independently selected from C, CR₄₄ or N.
In a preferred embodiment of the present invention,
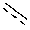 is a single bond or a double bond;
each X₁ is independently selected from C, CR₁₁ or N;
each X₂ is independently selected from C, CR₂₂ or N;
each X₃ is independently selected from C, CR₃₃ or N;
each X₄ is independently selected from C, CR₄₄ or N;
each X₅ is independently selected from CR₅₅ or N;
each M₁, M₂, M₃ or M₄ is independently selected from C or N;
when M₁, M₂, M₃ or M₄ is N, R₇, R₈, R₉ or R₁₀ is absent;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, (CR₆₆)ₙ₂, N or O;
each L₄ or L₅ is independently selected from a bond, C(R₅R₆), N(R₅), O or S;
each R₁₁, R₂₂, R₃₃, R₄₄, R₆₆, R₇₇, R₈₈, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, hydroxyl, halogen, cyano, amino, C₁₋₆ alkyl, C₁₋₆ haloalkyl or C₁₋₆ deuteroalkyl;
each R₅₅, R₇, R₈, R₉, R₁₀ or R₁₂ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl may optionally be further substituted with one or more substituents selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each ring F is independently selected from C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
each ring G is independently selected from C₃₋₁₂ cycloalkyl, 3- to 12-membered heterocyclyl, C₆₋₁₂ aryl or 5- to 14-membered heteroaryl, wherein the C₃₋₁₂ cycloalkyl, 3- to 12-membered heterocyclyl, C₆₋₁₂ aryl or 5- to 14-membered heteroaryl may optionally be further substituted with one or more substituents selected from R₅₅, R₇, R₈, R₉ or R₁₀;
alternatively, R₁ and R₂, R₃ and R₄, or R₅ and R₆, together with the carbon atom to which they are each attached, form C₃₋₁₂ cycloalkyl or 3- to 12-membered heterocyclyl, wherein the C₃₋₁₂ cycloalkyl or 3- to 12-membered heterocyclyl may optionally be further substituted with one or more substituents selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, hydroxyl, halogen, cyano, amino, C₁₋₆ alkyl, C₁₋₆ haloalkyl or C₁₋₆ deuteroalkyl;
m1 is 0, 1 or 2; and
n1 is 0, 1 or 2.

In certain embodiments of the present invention,
each X₁ is independently selected from C, CR₁₁ or N;
each X₂ is independently selected from C, CR₂₂ or N;
each X₃ is independently selected from C, CR₃₃ or N;
each X₄ is independently selected from C, CR₄₄ or N;
each X₅, M₁, M₂, M₃ or M₄ is independently selected from CR₅₅ or N;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, (CR₆₆)ₙ₂, N or O;
each L₄ or L₅ is independently selected from a bond, C(R₅R₆), N(R₅), O or S;
each R₁₁, R₂₂, R₃₃, R₄₄, R₆₆, R₇₇, R₈₈, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
each R₅₅, R₇, R₈, R₉ or R₁₀ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CRₐₐR_{bb})ₙ₁C(O)R₃₁, N=S=OR₃₁R₃₂, - (CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁(NR₃₁)C(O)R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl may optionally be further substituted with one or more substituents selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each ring F is independently selected from C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S may optionally be further substituted with one or more substituents selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
alternatively, R₁ and R₂, R₃ and R₄, or R₅ and R₆, together with the carbon atom to which they are each attached, form C₃₋₁₂ cycloalkyl or 3- to 12-membered heterocyclyl, wherein the C₃₋₁₂ cycloalkyl or 3- to 12-membered heterocyclyl is optionally substituted with one or more substituents selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₃₁, R₃₂, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
m1 is 0, 1 or 2; and
n1 is 0, 1 or 2.

In a preferred embodiment of the present invention, the compound is further represented by general formula (II-A-a1), (II-A-a2), (II-A-a3), (II-A-a4), (II-A-a5), (II-A-a6), (II-A-a7), (II-A-a8) or (II-A-a9):

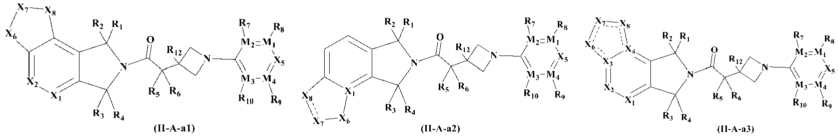

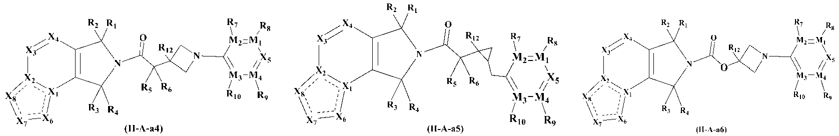

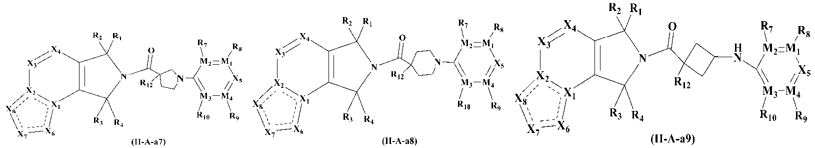

X₁-X₈, M₁-M₄, R₁, R₂, R₃, R₄, R₅, R₆, R₇, R₈, R₉, R₁₀ or R₁₂ is as defined in general formula (II-A-1) or (II-A-2).

In a preferred embodiment of the present invention, the compound is further represented by general formula (II-B-1), (II-B-2) or (II-B-5): 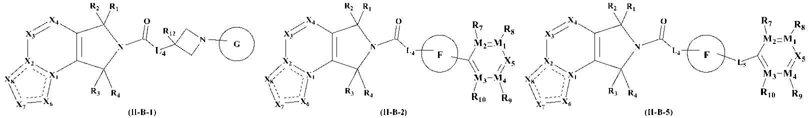
each X₁ is independently selected from C, CR₁₁ or N;
each X₂ is independently selected from C, CR₂₂ or N;
each X₃ is independently selected from CR₃₃ or N;
each X₄ is independently selected from CR₄₄ or N;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, (CR₆₆)ₙ₂, N or O;
each L₄ is independently selected from a bond, C(R₅R₆), N(R₅), O or S;
each R₁₁, R₂₂, R₃₃, R₄₄, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
each ring F is independently selected from C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S may optionally be further substituted with one or more substituents selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
each ring G is independently selected from C₃₋₁₂ cycloalkyl, 3- to 12-membered heterocyclyl, C₆₋₁₂ aryl or 5- to 14-membered heteroaryl, wherein the C₃₋₁₂ cycloalkyl, 3- to 12-membered heterocyclyl, C₆₋₁₂ aryl or 5- to 14-membered heteroaryl may optionally be further substituted with one or more substituents selected from R₅₅, R₇, R₈, R₉ or R₁₀;
alternatively, R₁ and R₂, R₃ and R₄, or R₅ and R₆, together with the carbon atom to which they are each attached, form C₃₋₁₂ cycloalkyl or 3- to 12-membered heterocyclyl, wherein the C₃₋₁₂ cycloalkyl or 3- to 12-membered heterocyclyl may optionally be further substituted with one or more substituents selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₅₅, R₇, R₈, R₉ or R₁₀ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -OCR₃₁R₃₂R₃₃, -(CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl may optionally be further substituted with one or more substituents selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
m1 is 0, 1 or 2; and
n1 is 0, 1 or 2.

In a further preferred embodiment of the present invention, the compound is further represented by general formula (II-B-a1), (II-B-a2), (II-B-a3), (II-B-a4), (II-B-a5), (II-B-a6), (II-B-a7), (II-B-a8), (II-B-a9) or (II-B-a10): 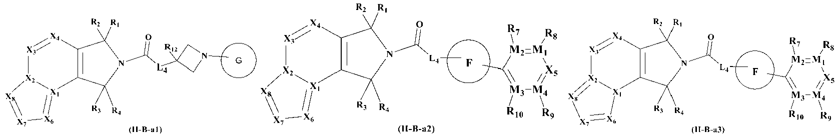 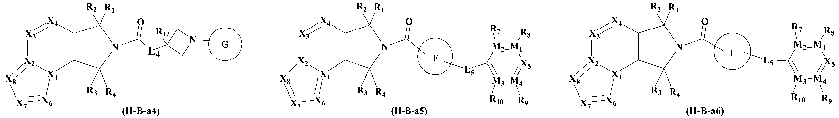 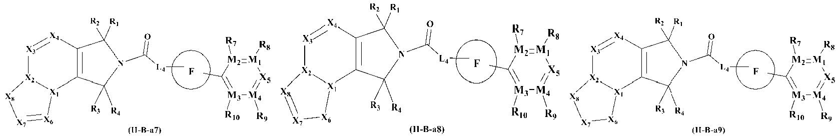 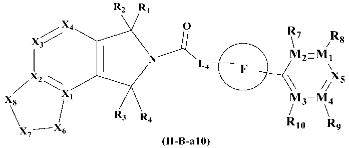
each ring F is independently selected from C₃₋₈ cycloalkyl, 3- to 8-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
each ring G is independently selected from C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S may optionally be further substituted with one or more substituents selected from R₅₅, R₇, R₈, R₉ or R₁₀.

In a further preferred embodiment of the present invention, the compound is further represented by general formula (II-B-3), (II-B-4), (II-8), (II-9), (II-10), (II-11), (II-12), (II-13), (II-14), (II-15), (II-16), (II-17) or (II-18): 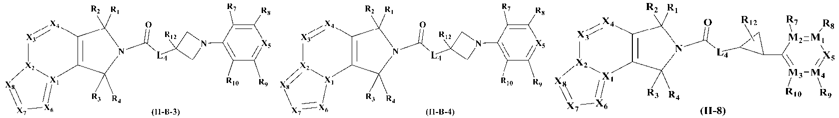 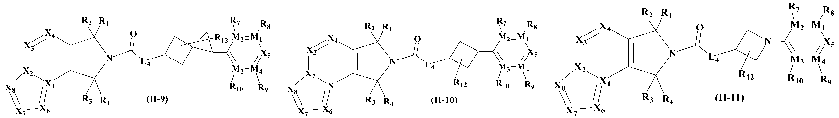 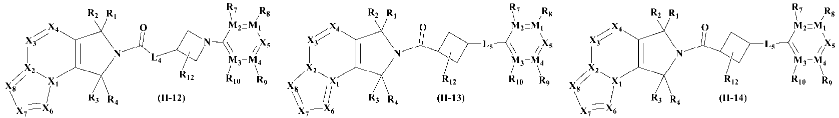 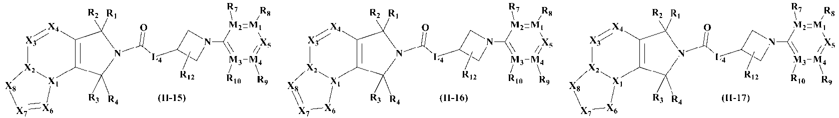 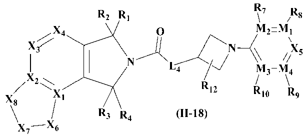 wherein:
each X₁ is independently selected from C, CR₁₁ or N;
each X₂ is independently selected from C, CR₂₂ or N;
each X₃ is independently selected from C, CR₃₃ or N;
each X₄ is independently selected from C, CR₄₄ or N;
each X₅ is independently selected from CR₅₅ or N;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, (CR₆₆)ₙ₂, N or O;
each L₄ is independently selected from a bond, C(R₅R₆), N(R₅), O or S;
each R₁₁, R₂₂, R₃₃, R₄₄, R₆₆, R₇₇, R₈₈, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S is optionally substituted with one or more substituents selected from deuterium, hydroxyl, halogen, cyano, amino, C₁₋₆ alkyl, C₁₋₆ haloalkyl or C₁₋₆ deuteroalkyl;
alternatively, R₁ and R₂, R₃ and R₄, or R₅ and R₆, together with the carbon atom to which they are each attached, form C₃₋₁₀ cycloalkyl or 3- to 10-membered heterocyclyl containing 1 to 3 heteroatoms selected from N, O or S, wherein the C₃₋₁₀ cycloalkyl or 3- to 10-membered heterocyclyl containing 1 to 3 heteroatoms selected from N, O or S is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
each R₅₅, R₇, R₈, R₉ or R₁₀ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, deuterated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, -O(CRₐₐR_{bb})ₙ₁R₃₁, -N(CRₐₐR_{bb})ₙ₁R₃₁, -S(CRₐₐR_{bb})ₙ₁R₃₁, -OCR₃₁R₃₂R₃₃, - (CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, - (CH₂)ₙ₁P(O)₂R₃₁R₃₂, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
m1 is 0, 1 or 2; and
n1 is 0, 1 or 2.

In a preferred embodiment of the present invention,
each R₅₅, R₇, R₈, R₉ or R₁₀ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, - OCR₃₁R₃₂R₃₃, -(CRₐₐR_{bb})ₙ₁C(O)R₃₁, N=S=OR₃₁R₃₂, -(CH₂)ₙ₁C(O)NR₃₁R₃₂, - (CH₂)ₙ₁P(O)R₃₁R₃₂, -(CH₂)ₙ₁P(O)₂R₃₁R₃₂, -(CRₐₐR_{bb})ₙ₁(NR₃₁)C(O)R₃₂, - (CRₐₐR_{bb})ₙ₁C(O)NR₃₁R₃₂, -(CRₐₐR_{bb})ₙ₁(NR₃₁)S(O)ₘ₁R₃₂, - (CRₐₐR_{bb})ₙ₁S(O)ₘ₁NR₃₁R₃₂, -(CRₐₐR_{bb})ₙ₁S(O)(=NR_{cc})R₃₂ or -(CRₐₐR_{bb})ₙ₁S(O)ₘ₁R₃₂, wherein the amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, hydroxyl, halogen, cyano, amino, C₁₋₆ alkyl, C₁₋₆ haloalkyl or C₁₋₆ deuteroalkyl;
m1 is 0, 1 or 2; and
n1 is 0, 1 or 2.

In a preferred embodiment of the present invention, the compound is further represented by general formula (II-1), (II-2), (II-3), (II-4), (II-5), (II-6) or (II-7): 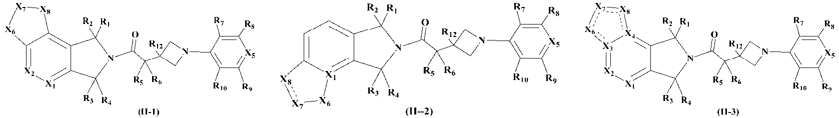 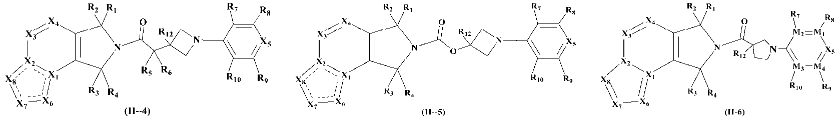 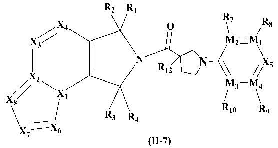
each X₁ is independently selected from C, CR₁₁ or N;
each X₂ is independently selected from C, CR₂₂ or N;
each X₃ is independently selected from C, CR₃₃ or N;
each X₄ is independently selected from C, CR₄₄ or N;
each X₅ is independently selected from CR₅₅ or N; each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, (CR₆₆)ₙ₂, N or O;
each L₄ is independently selected from a bond, C(R₅R₆), N(R₅), O or S;
each R₁₁, R₂₂, R₃₃, R₄₄, R₆₆, R₇₇, R₈₈, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S is optionally substituted with one or more substituents selected from deuterium, hydroxyl, halogen, cyano, amino, C₁₋₆ alkyl, C₁₋₆ haloalkyl or C₁₋₆ deuteroalkyl;
alternatively, R₁ and R₂, R₃ and R₄, or R₅ and R₆, together with the carbon atom to which they are each attached, form C₃₋₁₀ cycloalkyl or 3- to 10-membered heterocyclyl containing 1 to 3 heteroatoms selected from N, O or S, wherein the C₃₋₁₀ cycloalkyl or 3- to 10-membered heterocyclyl containing 1 to 3 heteroatoms selected from N, O or S is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
each R₅₅, R₇, R₈, R₉ or R₁₀ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, deuterated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, -O(CRₐₐR_{bb})ₙ₁R₃₁, -N(CRₐₐR_{bb})ₙ₁R₃₁, -S(CRₐₐR_{bb})ₙ₁R₃₁, -OCR₃₁R₃₂R₃₃, - (CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, - (CH₂)ₙ₁P(O)₂R₃₁R₃₂, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
m1 is 0, 1 or 2; and
n1 is 0, 1 or 2.

In a further preferred embodiment of the present invention,
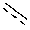 is a single bond or a double bond;
each X₁ is independently selected from C, CR₁₁ or N;
each X₂ is independently selected from C, CR₂₂ or N;
each X₃ is independently selected from C, CR₃₃ or N;
each X₄ is independently selected from C, CR₄₄ or N;
each X₅ is independently selected from CR₅₅ or N;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, CR₆₆, N or O;
each R₁₁, R₂₂, R₃₃, R₄₄, R₆₆, R₇₇, R₈₈, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
each R₅₅, R₇, R₈, R₉, R₁₀ or R₁₂ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl may optionally be further substituted with one or more substituents selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
alternatively, R₁ and R₂, R₃ and R₄, or R₅ and R₆, together with the carbon atom to which they are each attached, form C₃₋₁₂ cycloalkyl or 3- to 12-membered heterocyclyl, wherein the C₃₋₁₂ cycloalkyl or 3- to 12-membered heterocyclyl may optionally be further substituted with one or more substituents selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
n2 is 0, 1 or 2.

In certain embodiments of the present invention,
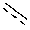 is a single bond or a double bond;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, CR₆₆, N or O;
each R₆₆, R₇₇ or R₈₈ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, which is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
n2 is 0, 1 or 2;
X₁, X₂, and R₁-R₁₁ are as defined in general formula (II).

In a preferred embodiment of the present invention,
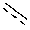 is a single bond or a double bond;
each X₁ is independently selected from C, CR₁₁ or N;
each X₂ is independently selected from C, CR₂₂ or N;
each X₃ is independently selected from C, CR₃₃ or N;
each X₄ is independently selected from C, CR₄₄ or N;
each X₅ is independently selected from CR₅₅ or N;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, CR₆₆, N or O;
each R₁₁, R₂₂, R₃₃, R₄₄, R₆₆, R₇₇, R₈₈, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
each R₅₅, R₇, R₈, R₉, R₁₀ or R₁₂ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁(NR₃₁)C(O)R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl may optionally be further substituted with one or more substituents selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
alternatively, R₁ and R₂, R₃ and R₄, or R₅ and R₆, together with the carbon atom to which they are each attached, form C₃₋₁₂ cycloalkyl or 3- to 12-membered heterocyclyl, wherein the C₃₋₁₂ cycloalkyl or 3- to 12-membered heterocyclyl may optionally be further substituted with one or more substituents selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₃₁, R₃₂, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from hydrogen, deuterium, hydroxyl, halogen, cyano, amino, C₁₋₆ alkyl, C₁₋₆ haloalkyl or C₁₋₆ deuteroalkyl;
n2 is 0, 1 or 2.

In a further preferred embodiment of the present invention, the compound is further represented by general formula (II-1a), (II-2a), (II-3a), (II-4a), (II-5a) or (II-6a):

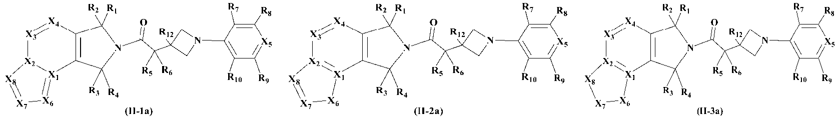

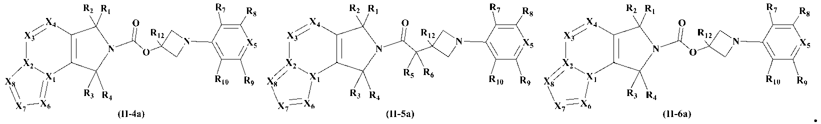

In a further preferred embodiment of the present invention, the compound is further represented by general formula (II-1b), (II-2b), (II-3b), (II-4b), (II-5b), (II-6b), (II-7b), (II-8b), (II-9b), (II-10b), (II-11b), (II-12b), (II-13b), (II-14b), (II-15b), (II-16b), (II-17b), (II-18b) or (II-19b):

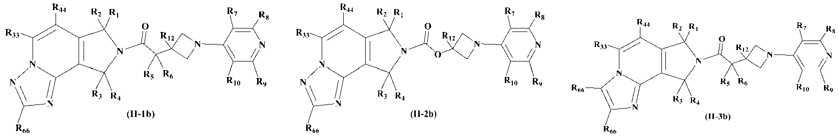

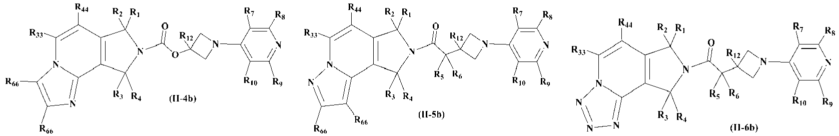

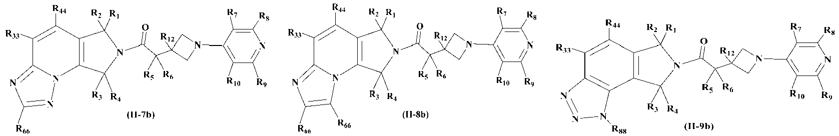

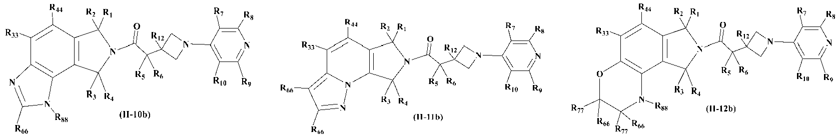

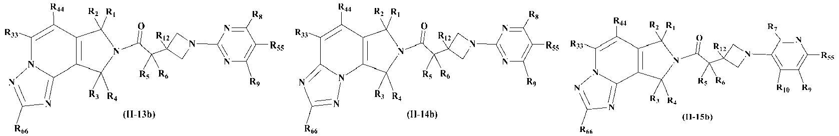

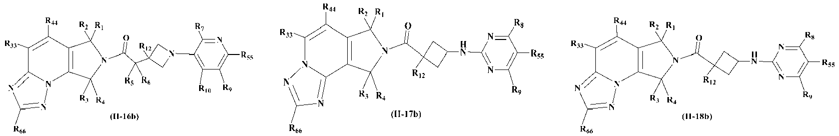

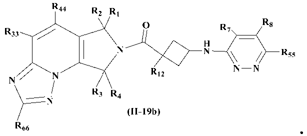

The present invention further provides a compound represented by general formula (VIII-A), or a stereoisomer or pharmaceutically acceptable salt thereof: 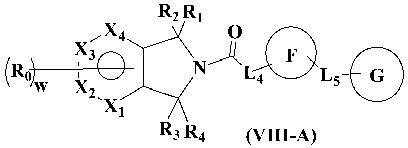 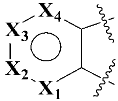 is cycloalkyl, heterocyclyl, aromatic ring or heteroaryl;
X₁ is selected from CR₁₁ or N; X₂ is selected from CR₂₂ or N;
X₃ is selected from CR₃₃ or N; X₄ is selected from CR₄₄ or N;
each L₄ or L₅ is independently selected from a bond, C(R₅R₆), N(R₅), O or S;
each R₀, R₁₁, R₂₂, R₃₃, R₄₄, R₁, R₂, R₃, R₄, R₅ or R₆ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
furthermore, any two of the groups R₁, R₂, R₃, R₄, R₁₁, R₂₂, R₃₃ and R₄₄, together with the atom to which they are each attached, form cycloalkyl, heterocyclyl, aryl or heteroaryl, wherein the cycloalkyl, heterocyclyl, aryl or heteroaryl is optionally substituted with one or more substituents selected from deuterium, oxo, thio, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
ring F or ring G is present or absent, and when present, each is independently selected from cycloalkyl, heterocyclyl, aryl or heteroaryl, wherein the cycloalkyl, heterocyclyl, aryl or heteroaryl may optionally be further substituted with one or more substituents selected from R₅₅, R₇, R₈, R₉ or R₁₀;
each R₅₅, R₇, R₈, R₉ or R₁₀ is independently selected from hydrogen, deuterium, halogen, amino, hydroxyl, cyano, nitro, alkyl, alkenyl, alkynyl, deuteroalkyl, haloalkyl, alkoxy, haloalkoxy, deuterated alkoxy, hydroxyalkyl, cyano-substituted alkyl, cycloalkyl, heterocyclyl, aryl, heteroaryl, -O(CRₐₐR_{bb})ₙ₁R₃₁, -N(CRₐₐR_{bb})ₙ₁R₃₁, -S(CRₐₐR_{bb})ₙ₁R₃₁, -OCR₃₁R₃₂R₃₃, -(CRₐₐR_{bb})ₙ₁C(O)R₃₁, N=S=OR₃₁R₃₂, N(R₃₁)=S=OR₃₂-, -(CH₂)ₙ₁C(O)NR₃₁R₃₂, -(CH₂)ₙ₁P(O)R₃₁R₃₂, -(CH₂)ₙ₁P(O)₂R₃₁R₃₂, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
each R₃₁, R₃₂, R₃₃, Rₐₐ, R_{bb} or R_{cc} is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
m1 is 0, 1 or 2;
n 1 is 0, 1 or 2;
w is 0, 1, 2, 3 or 4.

In a preferred embodiment of the present invention, general formula (VIII-A) is further represented by general formula (VIII-1A), (VIII-1B) or (VIII-1C): 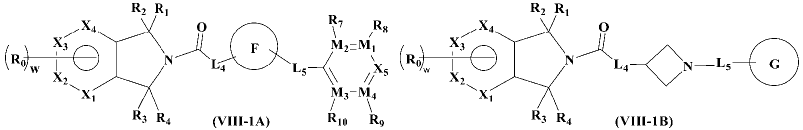 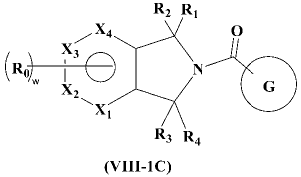
each X₁ is independently selected from CR₁₁ or N; each X₂ is independently selected from CR₂₂ or N;
each X₃ is independently selected from CR₃₃ or N; each X₄ is independently selected from CR₄₄ or N;
each L₄ or L₅ is independently selected from a bond, C(R₅R₆), N(R₅), O or S;
X₅ is selected from CR₅₅ or N;
each M₁, M₂, M₃ or M₄ is independently selected from C or N;
when M₁, M₂, M₃ or M₄ is N, R₇, R₈, R₉ or R₁₀ is absent;
each ring F or ring G is independently selected from C₃₋₁₂ cycloalkyl, 3- to 12-membered heterocyclyl, C₆₋₁₂ aryl or 5- to 12-membered heteroaryl, wherein the C₃₋₁₂ cycloalkyl, 3- to 12-membered heterocyclyl, C₆₋₁₂ aryl or 5- to 12-membered heteroaryl is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₀, R₁₁, R₂₂, R₃₃, R₄₄, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, hydroxyl, halogen, cyano, amino, C₁₋₆ alkyl, C₁₋₆ haloalkyl or C₁₋₆ deuteroalkyl;
each R₅₅, R₇, R₈, R₉ or R₁₀ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, deuterated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -O(CRₐₐR_{bb})ₙ₁R₃₁, -N(CRₐₐR_{bb})ₙ₁R₃₁, -S(CRₐₐR_{bb})ₙ₁R₃₁, -OCR₃₁R₃₂R₃₃, -(CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, -(CH₂)ₙ₁P(O)₂R₃₁R₃₂, - (CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl may optionally be further substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
m1 is 0, 1 or 2; and
n1 is 0, 1 or 2;
provided that
   (1) R₁ and R₄₄, or R₂ and R₄₄, together with the atom to which they are each attached, form C₃₋₁₂ cycloalkyl, 3- to 12-membered heterocyclyl, C₆₋₁₂ aryl or 5- to 12-membered heteroaryl, wherein the C₃₋₁₂ cycloalkyl, 3- to 12-membered heterocyclyl, C₆₋₁₂ aryl or 5- to 12-membered heteroaryl is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
   alternatively, (2) R₃ and R₁₁, or R₄ and R₁₁, together with the atom to which they are each attached, form C₃₋₁₂ cycloalkyl, 3- to 12-membered heterocyclyl, C₆₋₁₂ aryl or 5- to 12-membered heteroaryl, wherein the C₃₋₁₂ cycloalkyl, 3- to 12-membered heterocyclyl, C₆₋₁₂ aryl or 5- to 12-membered heteroaryl is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
   alternatively, (3) R₂₂ and R₃₃, together with the atom to which they are attached, form C₃₋₁₂ cycloalkyl, 3- to 12-membered heterocyclyl, C₆₋₁₂ aryl or 5- to 12-membered heteroaryl, wherein the C₃₋₁₂ cycloalkyl, 3- to 12-membered heterocyclyl, C₆₋₁₂ aryl or 5- to 12-membered heteroaryl is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
   alternatively, (4) R₃₃ and R₄₄, together with the atom to which they are attached, form C₃₋₁₂ cycloalkyl, 3- to 12-membered heterocyclyl, C₆₋₁₂ aryl or 5- to 12-membered heteroaryl, wherein the C₃₋₁₂ cycloalkyl, 3- to 12-membered heterocyclyl, C₆₋₁₂ aryl or 5- to 12-membered heteroaryl is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
   alternatively, (4) R₁₁ and R₂₂, together with the atom to which they are attached, form C₃₋₁₂ cycloalkyl, 3- to 12-membered heterocyclyl, C₆₋₁₂ aryl or 5- to 12-membered heteroaryl, wherein the C₃₋₁₂ cycloalkyl, 3- to 12-membered heterocyclyl, C₆₋₁₂ aryl or 5- to 12-membered heteroaryl is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl.

In the above general formulas described in the present invention,
each R₁₁, R₂₂, R₃₃ or R₄₄ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, hydroxyl, halogen, cyano, amino, C₁₋₃ alkyl, C₁₋₃ haloalkyl or C₁₋₃ deuteroalkyl;
preferably, each R₁₁, R₂₂, R₃₃ or R₄₄ is independently selected from hydrogen, deuterium, fluorine, chlorine, bromine, amino, hydroxyl, cyano, nitro, methyl, ethyl, propyl, isopropyl, fluoromethyl, difluoromethyl, trifluoromethyl, cyclopropyl, cyclobutyl, epoxyethyl, epoxypropyl or epoxybutyl, which is optionally substituted with one or more substituents selected from hydrogen, deuterium, hydroxyl, halogen, cyano, amino, C₁₋₃ alkyl, C₁₋₃ haloalkyl or C₁₋₃ deuteroalkyl.

In the above general formulas described in the present invention,
R₅₅ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, deuterated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, -O(CRₐₐR_{bb})ₙ₁R₃₁, -N(CRₐₐR_{bb})ₙ₁R₃₁, -S(CRₐₐR_{bb})ₙ₁R₃₁, -(CRₐₐR_{bb})ₙ₁C(O)R₃₁, N=S=OR₃₁R₃₂, - (CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, deuterated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
each R₃₁, R₃₂ or R₃₃ is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₆ cycloalkyl, 3- to 6-membered heterocyclyl containing 1 to 3 heteroatoms selected from N, O or S, C₆₋₁₀ aryl or 5- to 8-membered heteroaryl containing 1 to 3 heteroatoms selected from N, O or S, wherein the amino, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₆ cycloalkyl, 3- to 6-membered heterocyclyl containing 1 to 3 heteroatoms selected from N, O or S, C₆₋₁₀ aryl or 5- to 8-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each R₃₁, R₃₂ or R₃₃ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, cyclopropyl, cyclobutyl, cyclopentyl, aminomethyl, methoxy, ethoxy or propoxy, wherein the amino, methylamino, dimethylamino, methyl, ethyl, isopropyl, fluoromethyl, fluoroethyl, difluoromethyl, difluoroethyl, trifluoroethyl, cyclopropyl, cyclobutyl, cyclopentyl, aminomethyl, methoxy, ethoxy or propoxy is optionally substituted with one or more substituents selected from deuterium, hydroxyl, fluorine, chlorine, bromine, cyano, amino, C₁₋₃ alkyl, C₁₋₃ haloalkyl or C₁₋₃ deuteroalkyl;
each Rₐₐ, R_{bb} or R_{cc} is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl, wherein the amino, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each Rₐₐ, R_{bb} or R_{cc} is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy.

In a further preferred embodiment of the present invention,
R₅₅ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, -(CRₐₐR_{bb})ₙ₁C(O)R₃₁, N=S=OR₃₁R₃₂, - (CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S;
each R₃₁, R₃₂ or R₃₃ is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₆ cycloalkyl, 3- to 6-membered heterocyclyl containing 1 to 3 heteroatoms selected from N, O or S, C₆₋₁₀ aryl or 5- to 8-membered heteroaryl containing 1 to 3 heteroatoms selected from N, O or S, which is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each R₃₁, R₃₂ or R₃₃ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, cyclopropyl, cyclobutyl, cyclopentyl, aminomethyl, methoxy, ethoxy or propoxy;
each Rₐₐ, R_{bb} or R_{cc} is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl, which is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each Rₐₐ, R_{bb} or R_{cc} is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy.

In the above general formulas described in the present invention,
each R₁ or R₂ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, hydroxyl, halogen, cyano, amino, C₁₋₃ alkyl, C₁₋₃ haloalkyl or C₁₋₃ deuteroalkyl;
preferably, each R₁ or R₂ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy;
alternatively, R₁ and R₂, together with the carbon atom to which they are attached, form C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S;
preferably, R₁ and R₂, together with the carbon atom to which they are attached, form C₃₋₈ cycloalkyl, 3- to 8-membered heterocyclyl containing 1 to 3 heteroatoms selected from N, O or S, C₆₋₁₀ aryl or 5- to 8-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl.

In the above general formulas described in the present invention,
each R₃ or R₄ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, hydroxyl, halogen, cyano, amino, C₁₋₃ alkyl, C₁₋₃ haloalkyl or C₁₋₃ deuteroalkyl;
preferably, each R₃ or R₄ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy;
alternatively, R₃ and R₄, together with the carbon atom to which they are attached, form C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S;
preferably, R₃ and R₄, together with the carbon atom to which they are attached, form C₃₋₈ cycloalkyl, 3- to 8-membered heterocyclyl containing 1 to 3 heteroatoms selected from N, O or S, C₆₋₁₀ aryl or 5- to 8-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl.

In the above general formulas described in the present invention,
each R₅ or R₆ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, hydroxyl, halogen, cyano, amino, C₁₋₃ alkyl, C₁₋₃ haloalkyl or C₁₋₃ deuteroalkyl;
preferably, each R₅ or R₆ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy;
alternatively, R₅ and R₆, together with the carbon atom to which they are attached, form C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S;
preferably, R₅ and R₆, together with the carbon atom to which they are attached, form C₃₋₈ cycloalkyl, 3- to 8-membered heterocyclyl containing 1 to 3 heteroatoms selected from N, O or S, C₆₋₁₀ aryl or 5- to 8-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, R₅ and R₆, together with the carbon atom to which they are attached, form cyclopropyl, cyclobutyl, cyclopentyl or cyclohexyl.

In the above general formulas described in the present invention,
each R₇, R₈, R₉ or R₁₀ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, deuterated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl, 5- to 10-membered heteroaryl containing 1 to 3 heteroatoms selected from N, O or S, -O(CRₐₐR_{bb})ₙ₁R₃₁, -N(CRₐₐR_{bb})ₙ₁R₃₁, -S(CRₐₐR_{bb})ₙ₁R₃₁, - (CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, - (CH₂)ₙ₁P(O)₂R₃₁, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, deuterated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
each R₃₁, R₃₂ or R₃₃ is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₆ cycloalkyl, 3- to 6-membered heterocyclyl containing 1 to 3 heteroatoms selected from N, O or S, C₆₋₁₀ aryl or 5- to 8-membered heteroaryl containing 1 to 3 heteroatoms selected from N, O or S, wherein the amino, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₆ cycloalkyl, 3- to 6-membered heterocyclyl containing 1 to 3 heteroatoms selected from N, O or S, C₆₋₁₀ aryl or 5- to 8-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each R₃₁, R₃₂ or R₃₃ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, cyclopropyl, cyclobutyl, cyclopentyl, aminomethyl, methoxy, ethoxy or propoxy, wherein the amino, methylamino, dimethylamino, methyl, ethyl, isopropyl, fluoromethyl, fluoroethyl, difluoromethyl, difluoroethyl, trifluoroethyl, cyclopropyl, cyclobutyl, cyclopentyl, aminomethyl, methoxy, ethoxy or propoxy is optionally substituted with one or more substituents selected from deuterium, hydroxyl, fluorine, chlorine, bromine, cyano, amino, C₁₋₃ alkyl, C₁₋₃ haloalkyl or C₁₋₃ deuteroalkyl;
each Rₐₐ, R_{bb} or R_{cc} is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl, wherein the amino, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each Rₐₐ, R_{bb} or R_{cc} is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy.

In a preferred embodiment of the present invention,
R₈ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, -(CRₐₐR_{bb})ₙ₁C(O)R₃₁, N=S=OR₃₁R₃₂, - (CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S;
each R₃₁, R₃₂ or R₃₃ is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₆ cycloalkyl, 3- to 6-membered heterocyclyl containing 1 to 3 heteroatoms selected from N, O or S, C₆₋₁₀ aryl or 5- to 8-membered heteroaryl containing 1 to 3 heteroatoms selected from N, O or S, which is optionally substituted with one or more substituents selected from hydrogen, deuterium, hydroxyl, halogen, cyano, amino, C₁₋₃ alkyl, C₁₋₃ haloalkyl or C₁₋₃ deuteroalkyl;
preferably, each R₃₁, R₃₂ or R₃₃ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, cyclopropyl, cyclobutyl, cyclopentyl, aminomethyl, methoxy, ethoxy or propoxy;
each Rₐₐ, R_{bb} or R_{cc} is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl, which is optionally substituted with one or more substituents selected from hydrogen, deuterium, hydroxyl, halogen, cyano, amino, C₁₋₃ alkyl, C₁₋₃ haloalkyl or C₁₋₃ deuteroalkyl;
preferably, each Rₐₐ, R_{bb} or R_{cc} is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy.

In the above general formulas described in the present invention,
each R₇, R₉ or R₁₀ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
preferably, each R₇, R₉ or R₁₀ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 8-membered heterocyclyl containing 1 to 3 heteroatoms selected from N, O or S, C₆₋₁₀ aryl or 5-to 8-membered heteroaryl containing 1 to 3 heteroatoms selected from N, O or S, which is optionally substituted with one or more substituents selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, or cyano-substituted C₁₋₃ alkyl;
more preferably, each R₇, R₉ or R₁₀ is independently selected from hydrogen, deuterium, fluorine, chlorine, bromine, amino, hydroxyl, cyano, nitro, methyl, ethyl, propyl, vinyl, allyl, propenyl, ethynyl, propynyl, propargyl, deuterated methyl, deuterated ethyl, deuterated propyl, fluoromethyl, fluoroethyl, trifluoromethyl, difluoromethyl, methoxy, ethoxy, propoxy, fluoro-substituted methoxy, fluoro-substituted ethoxy, trifluoromethoxy, hydroxymethyl, hydroxyethyl, hydroxypropyl, 2-hydroxypropyl, cyano-substituted methyl, cyano-substituted ethyl, cyano-substituted propyl, cyclopropyl, cyclobutyl, cyclopentyl or cyclohexyl.

In the above general formulas described in the present invention,
R₁₁ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
preferably, R₁₁ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 8-membered heterocyclyl containing 1 to 3 heteroatoms selected from N, O or S, C₆₋₁₀ aryl or 5- to 8-membered heteroaryl containing 1 to 3 heteroatoms selected from N, O or S, which is optionally substituted with one or more substituents selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, or cyano-substituted C₁₋₃ alkyl;
more preferably, R₁₁ is selected from hydrogen, deuterium, fluorine, chlorine, bromine, amino, hydroxyl, cyano, nitro, methyl, ethyl, propyl, vinyl, allyl, propenyl, ethynyl, propynyl, propargyl, deuterated methyl, deuterated ethyl, deuterated propyl, fluoromethyl, fluoroethyl, trifluoromethyl, difluoromethyl, methoxy, ethoxy, propoxy, fluoro-substituted methoxy, fluoro-substituted ethoxy, trifluoromethoxy, hydroxymethyl, hydroxyethyl, hydroxypropyl, 2-hydroxypropyl, cyano-substituted methyl, cyano-substituted ethyl, cyano-substituted propyl, cyclopropyl, cyclobutyl, cyclopentyl or cyclohexyl.

In the above general formulas described in the present invention,
R₁₂ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
preferably, R₁₂ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 8-membered heterocyclyl containing 1 to 3 heteroatoms selected from N, O or S, C₆₋₁₀ aryl or 5- to 8-membered heteroaryl containing 1 to 3 heteroatoms selected from N, O or S, which is optionally substituted with one or more substituents selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, or cyano-substituted C₁₋₃ alkyl;
more preferably, R₁₂ is selected from hydrogen, deuterium, fluorine, chlorine, bromine, amino, hydroxyl, cyano, nitro, methyl, ethyl, propyl, vinyl, allyl, propenyl, ethynyl, propynyl, propargyl, deuterated methyl, deuterated ethyl, deuterated propyl, fluoromethyl, fluoroethyl, trifluoromethyl, difluoromethyl, methoxy, ethoxy, propoxy, fluoro-substituted methoxy, fluoro-substituted ethoxy, trifluoromethoxy, hydroxymethyl, hydroxyethyl, hydroxypropyl, 2-hydroxypropyl, cyano-substituted methyl, cyano-substituted ethyl, cyano-substituted propyl, cyclopropyl, cyclobutyl, cyclopentyl or cyclohexyl.

In the above general formulas described in the present invention,
ring A is selected from C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
preferably, ring A is selected from C₃₋₆ cycloalkyl, 5- to 6-membered heterocyclyl containing 1 to 3 heteroatoms selected from N, O or S, C₆₋₁₀ aryl or 5-to 6-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, ring A is selected from cyclopropyl, cyclobutyl, cyclopentyl, cyclohexyl, 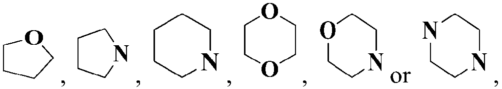 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl.

In the above general formulas described in the present invention,
ring B is selected from C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
preferably, ring B is selected from C₃₋₆ cycloalkyl, 5- to 6-membered heterocyclyl containing 1 to 3 heteroatoms selected from N, O or S, C₆₋₁₀ aryl or 5-to 6-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, ring B is selected from cyclopropyl, cyclobutyl, cyclopentyl, cyclohexyl, 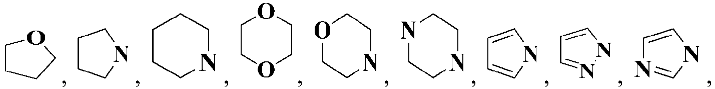 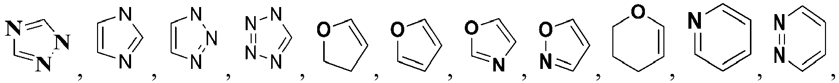 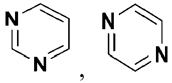 or phenyl, which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl;
further preferably, ring B is selected from 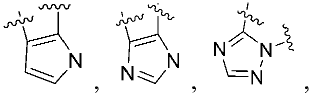 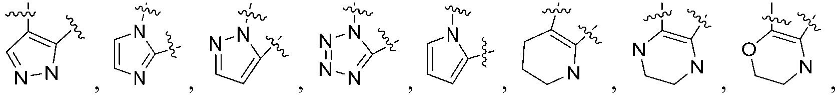 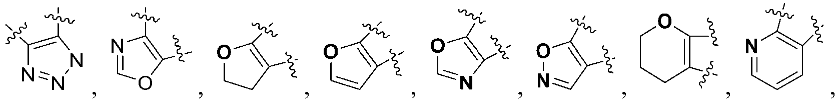 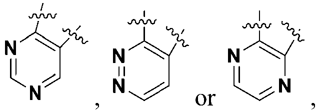 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl;
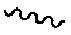 indicates the point of attachment to a fused ring.

In certain embodiments of the present invention, ring B is selected from cyclopropyl, cyclobutyl, cyclopentyl, cyclohexyl, 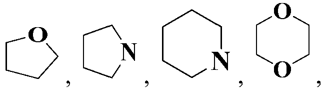 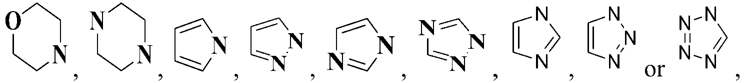 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl;

in certain embodiments of the present invention, ring B is selected from cyclopropyl, cyclobutyl, cyclopentyl, cyclohexyl, 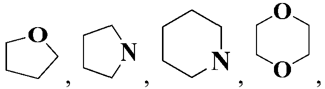 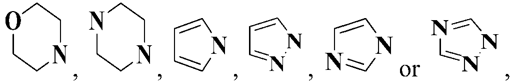 which may optionally be further substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl.

In the above general formulas described in the present invention,
ring C is selected from C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
preferably, ring C is selected from C₃₋₆ cycloalkyl, 5- to 6-membered heterocyclyl containing 1 to 3 heteroatoms selected from N, O or S, C₆₋₁₀ aryl or 5-to 6-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, ring C is selected from cyclopropyl, cyclobutyl, cyclopentyl, cyclohexyl, 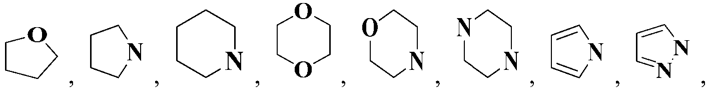 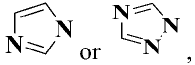 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl.

In the above general formulas described in the present invention,
ring D is selected from C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
preferably, ring D is selected from C₃₋₆ cycloalkyl, 5- to 6-membered heterocyclyl containing 1 to 3 heteroatoms selected from N, O or S, C₆₋₁₀ aryl or 5-to 6-membered heteroaryl containing 1 to 3 heteroatoms selected from N, O or S, which is optionally substituted with one or more substituents selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, ring D is selected from cyclopropyl, cyclobutyl, cyclopentyl, cyclohexyl, 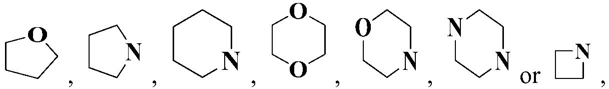 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl.

In the above general formulas described in the present invention,
ring E is selected from C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S;
preferably, ring E is selected from C₃₋₆ cycloalkyl, 5- to 6-membered heterocyclyl containing 1 to 3 heteroatoms selected from N, O or S, C₆₋₁₀ aryl or 5-to 6-membered heteroaryl containing 1 to 3 heteroatoms selected from N, O or S;
more preferably, ring E is selected from cyclopropyl, cyclobutyl, cyclopentyl, cyclohexyl, phenyl, naphthyl, pyridyl,

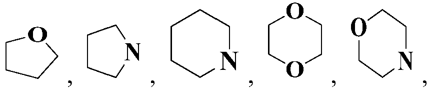

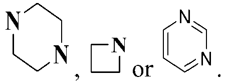

In the above general formulas described in the present invention,
each R₀ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
preferably, each R₀ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, each R₀ is independently selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl.

In the above general formulas described in the present invention,
R is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, -(CRₐₐR_{bb})ₙ₁C(O)R₃₁, N=S=OR₃₁R₃₂,-(CH₂)ₙ₁C(O)NR₃₁R₃₂, -(CH₂)ₙ₁P(O)R₃₁R₃₂, -(CH₂)ₙ₁P(O)₂R₃₁R₃₂,-(CRₐₐR_{bb})ₙ₁(NR₃₁)C(O)R₃₂, -(CRₐₐR_{bb})ₙ₁C(O)NR₃₁R₃₂,-(CRₐₐR_{bb})ₙ₁(NR₃₁)S(O)ₘ₁R₃₂, -(CRₐₐR_{bb})ₙ₁S(O)ₘ₁NR₃₁R₃₂,-(CRₐₐR_{bb})ₙ₁S(O)(=NR_{cc})R₃₂ or -(CRₐₐR_{bb})ₙ₁S(O)ₘ₁R₃₂, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S;
each R₃₁, R₃₂ or R₃₃ is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₆ cycloalkyl, 3- to 6-membered heterocyclyl containing 1 to 3 heteroatoms selected from N, O or S, C₆₋₁₀ aryl or 5- to 8-membered heteroaryl containing 1 to 3 heteroatoms selected from N, O or S, wherein the amino, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₆ cycloalkyl, 3- to 6-membered heterocyclyl containing 1 to 3 heteroatoms selected from N, O or S, C₆₋₁₀ aryl or 5- to 8-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each R₃₁, R₃₂ or R₃₃ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, cyclopropyl, cyclobutyl, cyclopentyl, aminomethyl, methoxy, ethoxy, propoxy, vinyl, propenyl, allyl, ethynyl, propynyl or propargyl;
in certain embodiments of the present invention, each R₃₁, R₃₂ or R₃₃ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, cyclopropyl, cyclobutyl, cyclopentyl, aminomethyl, methoxy, ethoxy or propoxy;
each Rₐₐ, R_{bb} or R_{cc} is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl, wherein the amino, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each Rₐₐ, R_{bb} or R_{cc} is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy.

In the above general formulas described in the present invention,
each R₆₆, R₇₇ or R₈₈ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, which is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each R₆₆, R₇₇ or R₈₈ is independently selected from hydrogen, deuterium, fluorine, chlorine, bromine, amino, hydroxyl, cyano, nitro, methyl, ethyl, propyl, isopropyl, fluoromethyl, difluoromethyl, trifluoromethyl, cyclopropyl, cyclobutyl, epoxyethyl, epoxypropyl or epoxybutyl, which is optionally substituted with one or more substituents selected from deuterium, hydroxyl, halogen, cyano, amino, C₁₋₃ alkyl, C₁₋₃ haloalkyl or C₁₋₃ deuteroalkyl.

In the above general formulas described in the present invention,
L₂ is selected from C₂₋₄ alkenylene, C₂₋₄ alkynylene, C₃₋₁₀ cycloalkylene, 3- to 10-membered heterocyclylene containing 1 to 3 heteroatoms selected from N, O or S, C₆₋₁₀ arylene or 5- to 10-membered heteroarylene containing 1 to 3 heteroatoms selected from N, O or S;
preferably, L₂ is selected from C₂₋₄ alkenylene, C₂₋₄ alkynylene, C₃₋₈ cycloalkylene, 3- to 8-membered heterocyclylene containing 1 to 3 heteroatoms selected from N, O or S, C₆₋₁₀ arylene or 5- to 8-membered heteroarylene containing 1 to 3 heteroatoms selected from N, O or S;
more preferably, L₂ is selected from C₂₋₄ alkenylene, C₂₋₄ alkynylene, C₃₋₆ monocyclic cycloalkylene, C₃₋₈ bridged cycloalkylene, C₃₋₈ spiro cycloalkylene, C₃₋₆ fused cycloalkylene, 3- to 6-membered monocyclic heterocyclylene containing 1 to 3 heteroatoms selected from N, O or S, 3- to 8-membered bridged heterocyclylene containing 1 to 3 heteroatoms selected from N, O or S, 3- to 8-membered spiro heterocyclylene containing 1 to 3 heteroatoms selected from N, O or S, 3- to 8-membered fused heterocyclylene containing 1 to 3 heteroatoms selected from N, O or S, C₆₋₁₀ aryl, 5- to 8-membered monocyclic heteroarylene containing 1 to 3 heteroatoms selected from N, O or S or 5- to 8-membered fused heteroarylene containing 1 to 3 heteroatoms selected from N, O or S;
further preferably, L₂ is selected from vinylidene, ethynylidene,

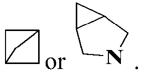

In the above general formulas described in the present invention,
L₃ is selected from a bond, (CH₂)ₙ₂, C₂₋₄ alkenylene or C₂₋₄ alkynylene;
preferably, L₂ is selected from a bond, methylene, vinylidene or ethynylidene.

In the above general formulas described in the present invention, ring F is selected from C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
preferably, ring F is selected from C₃₋₆ cycloalkyl, 5- to 6-membered heterocyclyl containing 1 to 3 heteroatoms selected from N, O or S, C₆₋₁₀ aryl or 5-to 6-membered heteroaryl containing 1 to 3 heteroatoms selected from N, O or S, wherein the C₃₋₆ cycloalkyl, 5- to 6-membered heterocyclyl containing 1 to 3 heteroatoms selected from N, O or S, C₆₋₁₀ aryl or 5- to 6-membered heteroaryl containing 1 to 3 heteroatoms selected from N, O or S is optionally substituted with one or more substituents selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, ring F is selected from cyclopropyl, cyclobutyl, cyclopentyl, cyclohexyl, 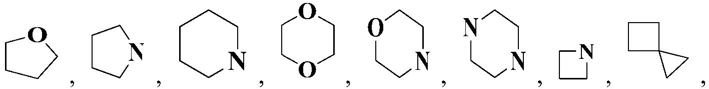 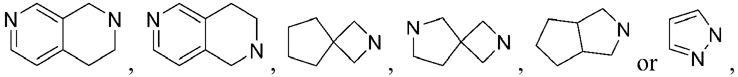 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl;
in certain embodiments of the present invention, ring F is selected from cyclopropyl, cyclobutyl, cyclopentyl, cyclohexyl, 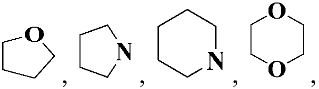 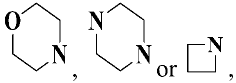 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl;
further preferably, ring F is selected from 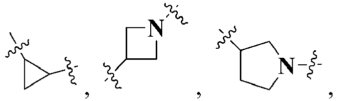 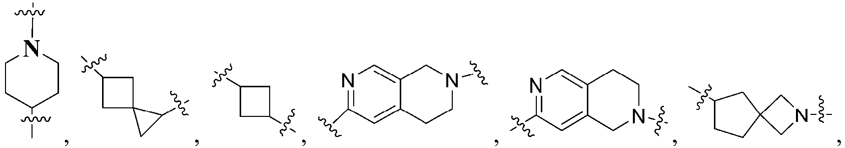 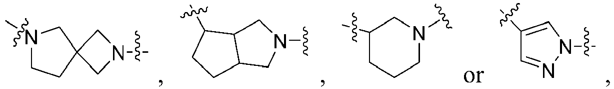 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl;
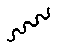 indicates the point of attachment to L₄ or L₅; when L₄ or L₅ is a bond, it indicates the point of attachment to carbonyl or ring G.

In certain embodiments of the present invention, ring F is selected from 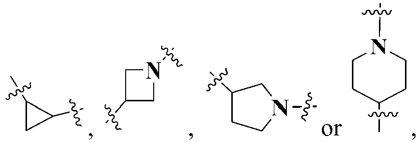 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl.

In the above general formulas described in the present invention, ring G is selected from C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from R₅₅, R₇, R₈, R₉ or R₁₀;
preferably, ring G is selected from C₃₋₈ cycloalkyl, 5- to 6-membered monocyclic heterocyclyl containing 1 to 3 heteroatoms selected from N, O or S, 6-to 10-membered fused heterocyclyl containing 1 to 3 heteroatoms selected from N, O or S, 5- to 10-membered spiro heterocyclyl containing 1 to 3 heteroatoms selected from N, O or S, C₆₋₁₀ aryl, 5- to 6-membered monocyclic heteroaryl containing 1 to 3 heteroatoms selected from N, O or S, or 8- to 10-membered fused heteroaryl containing 1 to 3 heteroatoms selected from N, O or S, which is optionally substituted with one or more substituents selected from R₅₅, R₇, R₈, R₉ or R₁₀;
more preferably, ring G is selected from a benzene ring, a naphthalene ring, pyridine, 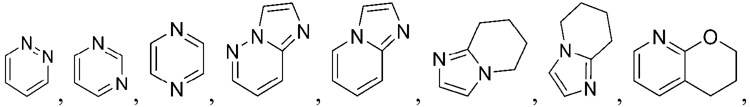 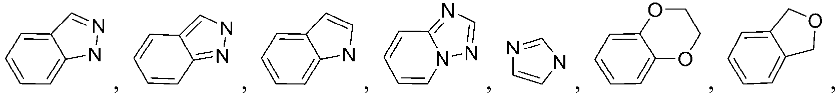 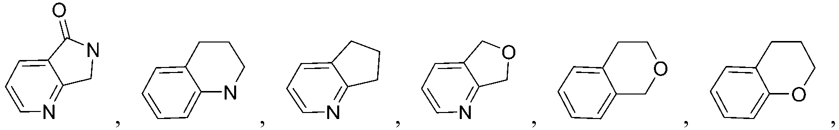 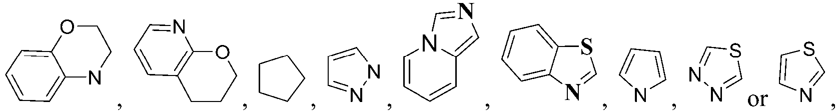 which is optionally substituted with one or more substituents selected from R₅₅, R₇, R₈, R₉ or R₁₀;
further preferably, ring G is selected from 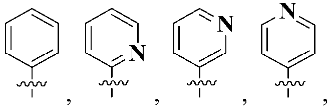 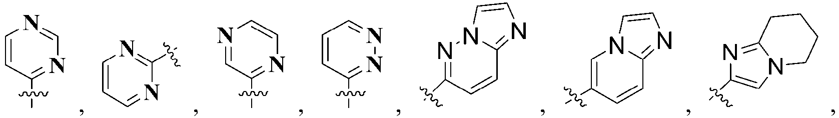 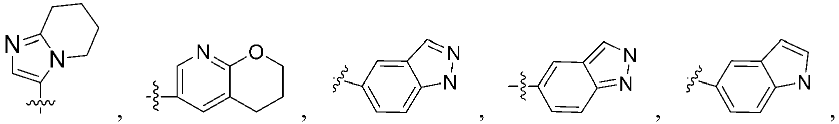 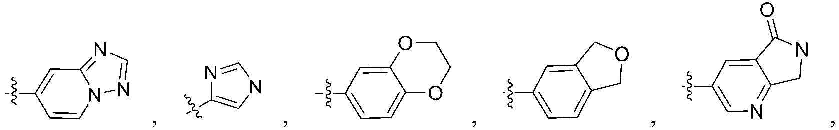 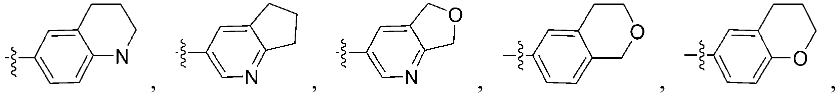 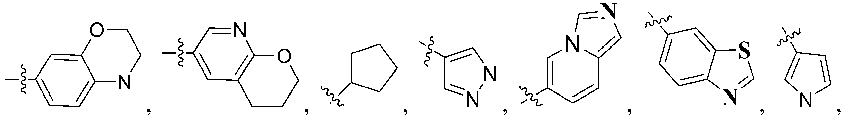 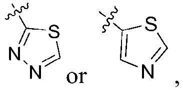 which is optionally substituted with one or more substituents selected from R₅₅, R₇, R₈, R₉ or R₁₀;
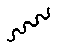 indicates the point of attachment to L₅; when L₅ is a bond, it indicates the point of attachment to ring F; when ring F is absent, it indicates the point of attachment to L₄; when L₄ is a bond, it indicates the point of attachment to C(O). In certain embodiments of the present invention, ring G is selected from a benzene ring, a naphthalene ring, pyridine, 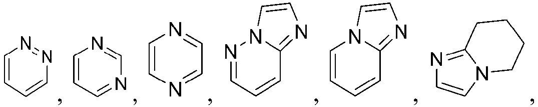 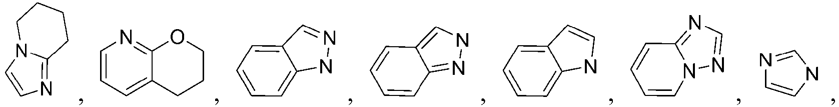 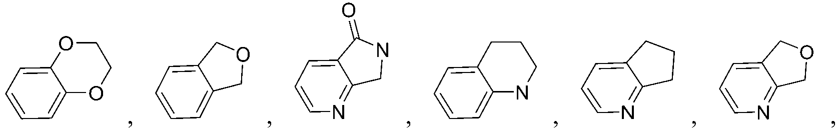 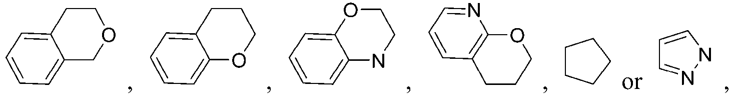 which is optionally substituted with one or more substituents selected from R₅₅, R₇, R₈, R₉ or R₁₀;
in certain embodiments of the present invention, ring G is selected from 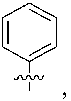 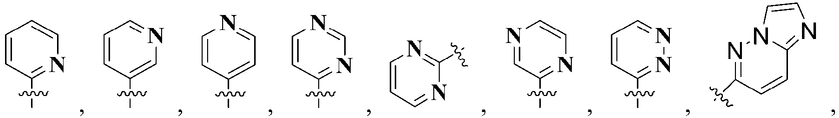 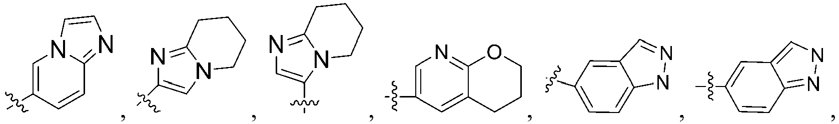 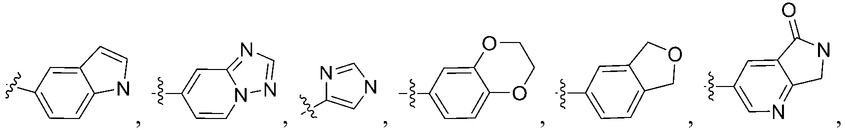 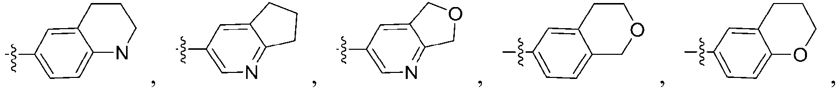 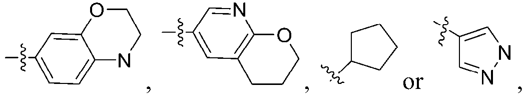 which is optionally substituted with one or more substituents selected from R₅₅, R₇, R₈, R₉ or R₁₀;
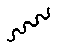 indicates the point of attachment to L₅; when L₅ is a bond, it indicates the point of attachment to ring F; when ring F is absent, it indicates the point of attachment to L₄; when L₄ is a bond, it indicates the point of attachment to C(O).

In certain embodiments of the present invention, ring G is selected from a benzene ring, a naphthalene ring, pyridine, 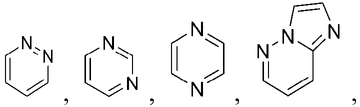 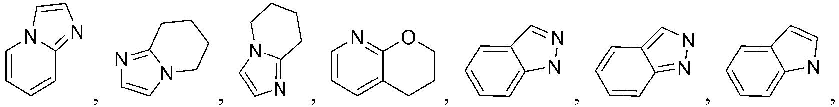 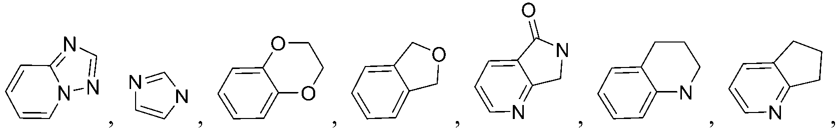 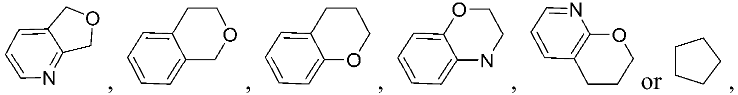 which is optionally substituted with one or more substituents selected from R₅₅, R₇, R₈, R₉ or R₁₀;
in certain embodiments of the present invention, ring G is selected from a benzene ring, a naphthalene ring, pyridine, 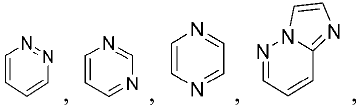 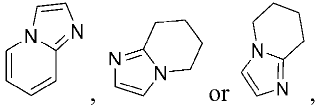 which is optionally substituted with one or more substituents selected from R₅₅, R₇, R₈, R₉ or R₁₀;
in certain embodiments of the present invention, ring G is selected from 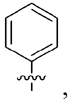 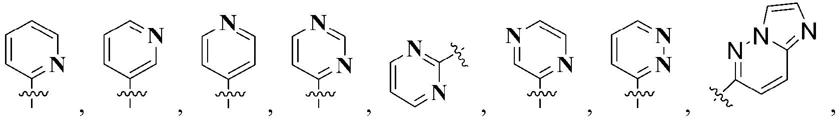 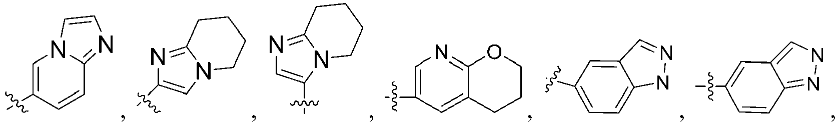 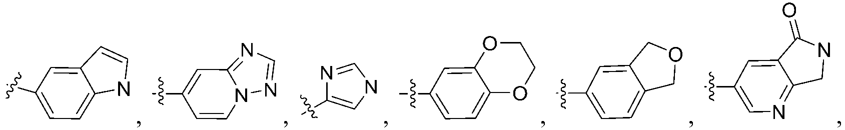 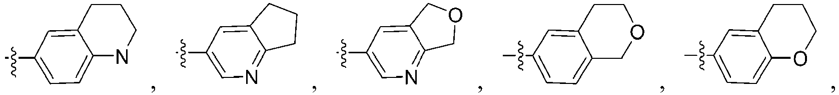 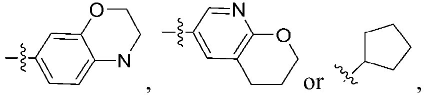 which is optionally substituted with one or more substituents selected from R₅₅, R₇, R₈, R₉ or R₁₀;
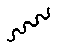 indicates the point of attachment to L₅; when L₅ is a bond, it indicates the point of attachment to ring F.

In certain embodiments of the present invention, ring G is selected from a benzene ring, a naphthalene ring, pyridine, 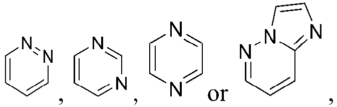 which may optionally be further substituted with one or more substituents selected from R₅₅, R₇, R₈, R₉ or R₁₀;
in certain embodiments of the present invention, ring G is selected from 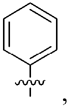 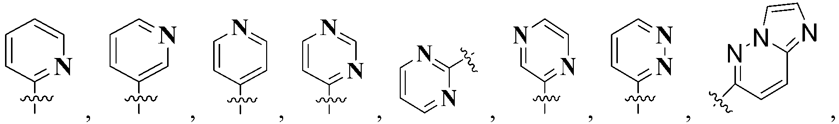 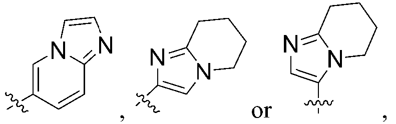 which is optionally substituted with one or more substituents selected from R₅₅, R₇, R₈, R₉ or R₁₀.

The present invention further provides a method for preparing a compound represented by general formula (II-A-a4), or a stereoisomer or pharmaceutically acceptable salt thereof, comprising the following step: 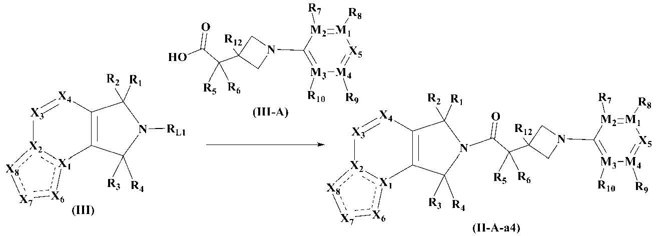
reacting a compound represented by general formula (III), or a stereoisomer or pharmaceutically acceptable salt thereof, with a compound represented by general formula (III-A), to afford general formula (II-A-a4);
wherein preferably, the reaction is carried out in a condensing reagent, wherein the condensing reagent is preferably 2-(7-azabenzotriazol-1-yl)-N,N,N',N'-tetramethyluronium hexafluorophosphate, 1-(3-dimethylaminopropyl)-3-ethylcarbodiimide hydrochloride, benzotriazol-1-yl-N,N,N',N'-tetramethyluronium hexafluorophosphate, 1-propylphosphonic anhydride, O-(benzotriazol-1-yl)-N,N,N',N'-tetramethyluronium tetrafluoroborate, N,N'-carbonyldiimidazole, N,N'-dicyclohexylcarbodiimide, N,N'-diisopropylcarbodiimide, 1H-benzotriazol-1-yloxytripyrrolidinophosphonium hexafluorophosphate, benzotriazol-1-yloxytris(dimethylamino)phosphonium hexafluorophosphate, (ethyl 2-cyano-2-(hydroxyimino)acetate)-N,N-dimethyl-morpholinourea hexafluorophosphate, N,N,N',N'-tetramethylchloroformamidinium hexafluorophosphate, 2-hydroxypyridine-N-oxide, tripyrrolidinophosphonium bromide hexafluorophosphate, bis(2-oxo-3-oxazolidinyl)phosphinic chloride, 2-chloro-1-methylpyridinium iodide, diethyl cyanophosphonate, 4-(4,6-dimethoxy-1,3,5-triazin-2-yl)-4-methylmorpholinium chloride, 2-succinimido-1,1,3,3-tetramethyluronium tetrafluoroborate, 2-ethoxy-1-ethoxycarbonyl-1,2-dihydroquinoline, 2-chloro-4,6-dimethoxy-1,3,5-triazine, ethyl 2-cyano-2-(hydroxyimino)acetate, diphenyl phosphorazidate, bis(pentafluorophenyl)carbonate, or 3-(diethoxyphosphoryloxy)-1,2,3-benzotriazin-4-one;
more preferably, the reaction is carried out in a base, wherein the base is preferably diisopropylethylamine, N-methylmorpholine, N-methylimidazole, 4-dimethylaminopyridine, 2,6-dimethylpyridine, potassium carbonate, potassium tert-butoxide, sodium tert-butoxide, 1,8-diazabicyclo[5.4.0]undec-7-ene, trimethylamine, triethylamine, pyridine, piperidine, morpholine, lithium diisopropylamide, lithium diethylamide, lithium bis(trimethylsilyl)amide, lithium isopropylcyclohexylamide, LiHMDS, potassium phosphate, tripotassium phosphate trihydrate, tripotassium phosphate dihydrate, tripotassium phosphate monohydrate, potassium acetate, sodium acetate, sodium bicarbonate, potassium bicarbonate, sodium carbonate, caesium carbonate, potassium hydroxide, sodium hydroxide, potassium hydride, sodium hydride, lithium hydroxide or 2-tert-butyl-1,1,3,3-tetramethylguanidine and mixtures thereof.

The present invention further provides a compound represented by general formula (III), or a stereoisomer or pharmaceutically acceptable salt thereof: 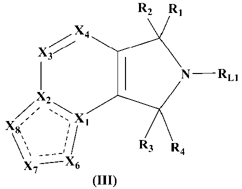
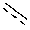 is a single bond or a double bond;
each X₁ is independently selected from a bond, C, CR₁₁, CR₁₁R₂₂ or N;
each X₂ is independently selected from a bond, C, CR₂₂, CR₁₁R₂₂ or N;
each X₃ is independently selected from a bond, C, CR₃₃ or N;
each X₄ is independently selected from a bond, C, CR₄₄ or N;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, (CR₆₆)ₙ₂, N or O;
R₁₁ is selected from hydrogen, deuterium or an amino protecting group;
each R₁₁, R₂₂, R₃₃, R₄₄, R₆₆, R₇₇, R₈₈, R₁, R₂, R₃ or R₄ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
n2 is 1 or 2.

In a preferred embodiment of the present invention, the compound represented by general formula (III) is further represented by general formula (III-1), (III-2), (III-3), (III-4) or (III-5): 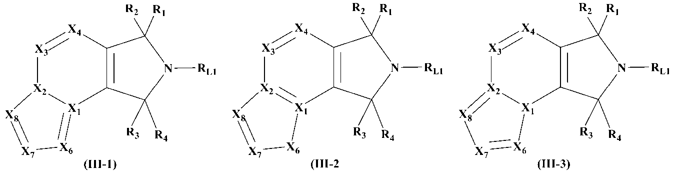 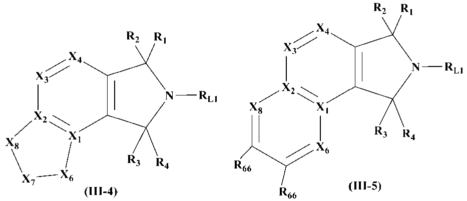
each X₁ is independently selected from a bond, C, CR₁₁ or N;
each X₂ is independently selected from a bond, C, CR₂₂ or N;
each X₃ is independently selected from a bond, CR₃₃ or N;
each X₄ is independently selected from a bond, CR₄₄ or N;
R_{L1} is selected from hydrogen, deuterium or an amino protecting group, wherein the amino protecting group is selected from a benzyl-type protecting group, a carboxylic ester-type protecting group, an acyl protecting group or a silyl protecting group;
preferably, the benzyl-type protecting group is selected from benzyl, 4-methoxybenzyl, 2,4-dimethoxybenzyl, 3,4-dimethoxybenzyl, 3-methoxybenzyl, 3,5-dimethoxybenzyl or 2,4,6-trimethoxybenzyl; the carboxylic ester-type protecting group is selected from allyloxycarbonyl, tert-butoxycarbonyl, benzyloxycarbonyl, trimethylsilylethoxycarbonyl, trichloroethoxycarbonyl, 9-fluorenylmethoxycarbonyl, methoxycarbonyl or ethoxycarbonyl; the acyl protecting group is selected from acetyl, benzoyl, p-methoxybenzylcarbonyl, acetyl, tosyl, p-nitrobenzenesulphonyl, phthaloyl, p-toluenesulphonyl or trifluoroacetyl; the silyl protecting group is selected from tert-butyldimethylsilyl or (trimethylsilyl)ethoxymethyl;
each R₁₁, R₂₂, R₃₃, R₄₄, R₆₆, R₇₇, R₈₈, R₁, R₂, R₃ or R₄ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, hydroxyl, halogen, cyano, amino, C₁₋₆ alkyl, C₁₋₆ haloalkyl or C₁₋₆ deuteroalkyl;
preferably, each R₁₁, R₂₂, R₃₃, R₄₄, R₆₆, R₇₇, R₈₈, R₁, R₂, R₃ or R₄ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl;
more preferably, each R₁₁, R₂₂, R₃₃, R₄₄, R₆₆, R₇₇ or R₈₈ is independently selected from hydrogen, deuterium, fluorine, chlorine, bromine, amino, hydroxyl, cyano, nitro, methyl, ethyl, propyl, isopropyl, fluoromethyl, difluoromethyl, trifluoromethyl, vinyl, ethynyl, cyclopropyl, cyclobutyl, epoxyethyl, epoxypropyl or epoxybutyl, wherein the amino, methyl, ethyl, propyl, isopropyl, fluoromethyl, difluoromethyl, trifluoromethyl, vinyl, ethynyl, cyclopropyl, cyclobutyl, epoxyethyl, epoxypropyl or epoxybutyl is optionally substituted with one or more substituents selected from deuterium, hydroxyl, halogen, cyano, amino, C₁₋₃ alkyl, C₁₋₃ haloalkyl or C₁₋₃ deuteroalkyl;
each R₁, R₂, R₃ or R₄ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy.

The present invention further provides a method for preparing the compound represented by general formula (III-1), or the stereoisomer or pharmaceutically acceptable salt thereof, comprising the following step: 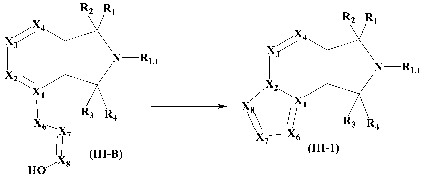
cyclising a compound represented by general formula (III-B), or a stereoisomer or pharmaceutically acceptable salt thereof to afford general formula (III-1);
preferably, the reaction is carried out in a cyclising reagent, wherein the cyclising reagent is preferably trifluoroacetic anhydride, p-toluenesulphonyl chloride, methanesulphonyl chloride, p-trifluoromethylbenzenesulphonyl chloride, 1-propylphosphonic anhydride, acetic anhydride, polyphosphoric acid, Eaton's reagent or boron trifluoride diethyl etherate;
alternatively, a method for preparing the compound represented by general formula (III-3), or the stereoisomer or pharmaceutically acceptable salt thereof, comprising the following step: 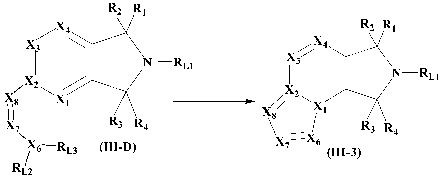
reacting a compound represented by general formula (III-D), or a stereoisomer or pharmaceutically acceptable salt thereof to afford general formula (III-3);
optionally, a method for preparing the compound represented by general formula (III-B), or the stereoisomer or pharmaceutically acceptable salt thereof, comprising the following step: 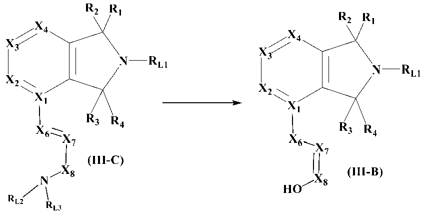
reacting a compound represented by general formula (III-C), or a stereoisomer or pharmaceutically acceptable salt thereof to afford general formula (III-B);
each R_{L2} or R_{L3} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, hydroxyl, halogen, cyano, amino, C₁₋₆ alkyl, C₁₋₆ haloalkyl or C₁₋₆ deuteroalkyl;
preferably, each R_{L2} or R_{L3} is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl.

The present invention further relates to a pharmaceutical composition comprising a therapeutically effective dose of any of the compounds represented by general formula, or a stereoisomer or pharmaceutically acceptable salt thereof, and one or more pharmaceutically acceptable carriers, diluents, or excipients.

In certain embodiments of the present invention, the weight percentage of the compound, or the stereoisomer or pharmaceutically acceptable salt thereof in the pharmaceutical composition is 0.1%-95%, preferably 5%-70%, e.g., 70%, 65%, 60%, 55%, 50%, 45%, 40%, 35%, 30%, 25%, 20%, 15%, 10%, or 5%, on a free base basis.

In certain embodiments of the present invention, the pharmaceutical composition is selected from a tablet, a capsule, a liquid preparation or an injection, preferably further comprises a filler, optionally further comprises a disintegrant, or further comprises one or more of a glidant or a lubricant.

In certain embodiments of the present invention, the pharmaceutical composition is an immediate-release preparation or a sustained-release preparation.

In certain embodiments of the present invention, the unit dose of the compound, or the stereoisomer or pharmaceutically acceptable salt thereof in the pharmaceutical composition is 1-1000 mg, preferably 1-500 mg, or preferably 1 mg, 2 mg, 3 mg, 5 mg, 10 mg, 20 mg, 40 mg, 50 mg, 60 mg, 80 mg, 100 mg, 200 mg, 300 mg, 400 mg, or 500 mg, on a free base basis.

In certain embodiments of the present invention, the compound, or the stereoisomer or pharmaceutically acceptable salt thereof can be administered by any convenient method, for example, by oral, parenteral, buccal, sublingual, nasal, rectal, intrathecal or transdermal administration, and the pharmaceutical composition can be adjusted accordingly.

In certain embodiments of the present invention, the compound, or the stereoisomer or pharmaceutically acceptable salt thereof can be formulated into a liquid or solid preparation, such as a syrup, a suspension, an emulsion, tablets, capsules, powder, granules, or a lozenge.

The present invention further relates to the use of any of the compounds represented by general formula, or the stereoisomer or pharmaceutically acceptable salt thereof, or the pharmaceutical composition in the preparation of a medicament for treating a disease associated with M4.

The present invention further relates to the use of the compound represented by general formula, or the stereoisomer or pharmaceutically acceptable salt thereof, or the pharmaceutical composition thereof in the preparation of a medicament for treating Alzheimer's disease, schizophrenia or psychosis, pain, addiction, sleep disorder, cognitive impairment, Parkinson's disease or condition, levodopa-induced dyskinesia in Parkinson's disease, Huntington's disease, xerostomia, pulmonary arterial hypertension, chronic obstructive pulmonary disease, asthma, urinary incontinence, glaucoma, Down syndrome, cerebral amyloid angiopathy, dementia, hereditary cerebral hemorrhage with amyloidosis-Dutch type, Creutzfeldt-Jakob disease, prion disease, amyotrophic lateral sclerosis, progressive supranuclear palsy, head trauma, stroke, pancreatitis, inclusion body myositis, other peripheral amyloidosis, diabetes mellitus, autism, and atherosclerosis, more preferably in the preparation of a medicament for treating Alzheimer's disease, schizophrenia, pain, addiction, and sleep disorder.

The present invention further relates to a method of using the compound represented by general formula, or the stereoisomer or pharmaceutically acceptable salt thereof, or the pharmaceutical composition thereof in the preparation of a medicament for treating a disease associated with M4.

The present invention further relates to a method for preventing and/or treating a disease associated with M4, comprising administering to a patient a therapeutically effective dose of the compound represented by general formula, or the stereoisomer or pharmaceutically acceptable salt thereof, or the pharmaceutical composition thereof.

The present invention further provides a method for treating a disease condition with the compound or the pharmaceutical composition of the present invention, wherein the disease condition includes, but is not limited to, conditions associated with M4.

The present invention further relates to a method for treating a disease associated with M4 in a mammal, comprising administering to the mammal a therapeutically effective amount of the compound of the present invention, or the pharmaceutically acceptable salt, ester, prodrug, solvate, hydrate or derivative thereof.

The compounds prepared by the present invention exhibit high M4 agonist activity and selectivity, high solubility (Ks ≥ 200 µM in PBS solution at pH 7.4), as well as higher plasma free fraction and brain exposure.

### Detailed Description of the Invention

Unless stated to the contrary, the terms used in the description and claims have the following meanings.

The term "alkyl" refers to a saturated aliphatic hydrocarbon group, which is a linear or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferably, the alkyl is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl can be substituted or unsubstituted. When the alkyl is substituted, the substituent can be substituted at any available connection point, and is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group. In the present invention, the alkyl is preferably methyl, ethyl, isopropyl, tert-butyl, haloalkyl, deuteroalkyl, alkoxy-substituted alkyl, and hydroxyl-substituted alkyl.

The term "alkylene" refers to an alkyl group in which one hydrogen atom is further substituted, for example, "methylene" refers to -CH₂-, "ethylene" refers to-(CH₂)₂-, "propylene" refers to -(CH₂)₃-, and "butylene" refers to -(CH₂)₄-, etc. The term "alkenyl" refers to an alkyl group as defined above consisting of at least two carbon atoms and at least one carbon-carbon double bond, such as ethenyl, 1-propenyl, 2-propenyl, 1-butenyl, 2-butenyl or 3-butenyl. Alkenyl can be substituted or unsubstituted. When the alkenyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and polycyclic cycloalkyl includes spiro, fused and bridged cycloalkyl, preferably cyclopropyl, cyclobutyl, cyclohexyl, cyclopentyl, and cycloheptyl.

The term "spirocycloalkyl" refers to a 5- to 20-membered polycyclic group with monocyclic rings sharing one carbon atom (called a spiro atom). It may contain one or more double bonds, but no ring has a fully conjugated π electron system. Preferably, the group is 6- to 14-membered, and more preferably 7- to 10-membered. According to the number of shared spiro atoms between the rings, the spirocycloalkyl is divided into monospirocycloalkyl, bispirocycloalkyl or polyspirocycloalkyl, preferably monospirocycloalkyl and bispirocycloalkyl. More preferably, the group is 3-membered/6-membered, 3-membered/5-membered, 4-membered/4-membered, 4-membered/5-membered, 4-membered/6-membered, 5-membered/5-membered or 5-membered/6-membered monospirocycloalkyl. Non-limiting examples of spirocycloalkyl include: 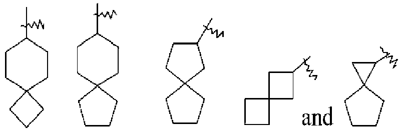 etc.;
and also include spirocycloalkyl in which monospirocycloalkyl and heterocycloalkyl share a spiro atom. Non-limiting examples include: 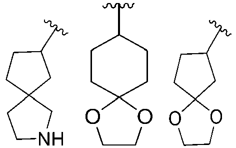 etc.

The term "fused cycloalkyl" refers to a 5- to 20-membered all-carbon polycyclic group in which each ring in the system shares an adjacent pair of carbon atoms with other rings in the system, one or more rings of which may contain one or more double bonds, but no ring has a fully conjugated π electron system. Preferably, the group is 6- to 14-membered, and more preferably 7- to 10-membered. According to the number of constituent rings, it can be divided into bicyclic, tricyclic, tetracyclic or polycyclic fused cycloalkyl, preferably bicyclic or tricyclic, and more preferably 5-membered/5-membered or 5-membered/6-membered bicyclic cycloalkyl. Non-limiting examples of fused cycloalkyl include: 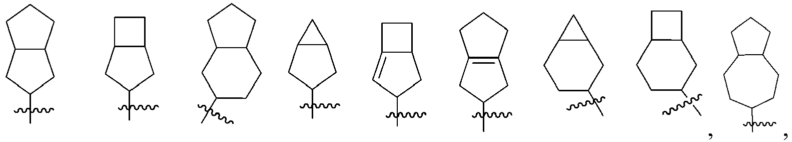 etc.

The term "bridged cycloalkyl" refers to a 5- to 20-membered all-carbon polycyclic group with any two rings sharing two carbon atoms that are not directly connected. It may contain one or more double bonds, but no ring has a fully conjugated π electron system. Preferably, the group is 6- to 14-membered, and more preferably 7- to 10-membered. According to the number of constituent rings, it can be divided into bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

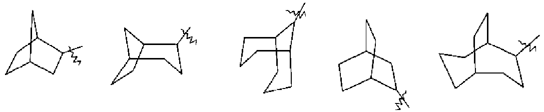

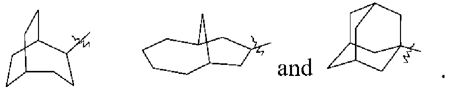

The cycloalkyl ring can be fused to an aryl, heteroaryl or heterocycloalkyl ring, wherein the ring connected to the parent structure is cycloalkyl, and non-limiting examples include indanyl, tetrahydronaphthyl, benzocycloheptanyl, etc. Cycloalkyl can be optionally substituted or unsubstituted. When the cycloalkyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group.

The term "heterocyclyl" refers to a saturated or partially unsaturated monocyclic or polycyclic hydrocarbon substituent, which comprises 3 to 20 ring atoms, wherein one or more ring atoms are heteroatoms selected from nitrogen, oxygen or S(O)ₘ (wherein m is an integer of 0 to 2), but excluding ring moieties of -O-O-, -O-S- or -S-S-, and the remaining ring atoms are carbon. Preferably, the heterocyclyl comprises 3 to 12 ring atoms, among which 1-4 are heteroatoms; more preferably, the heterocyclyl comprises 3 to 8 ring atoms; most preferably, the heterocyclyl comprises 3 to 8 ring atoms; further preferably, the heterocyclyl is a 3-to -8-membered heterocyclyl group comprising 1 to 3 nitrogen atoms, which is optionally substituted with 1 to 2 oxygen atoms, sulphur atoms, or oxo, including nitrogen-containing monocyclic heterocyclyl, nitrogen-containing spiroheterocyclyl, or nitrogen-containing fused heterocyclyl.

Non-limiting examples of monocyclic heterocyclyl include pyrrolidinyl, imidazolidinyl, tetrahydrofuryl, tetrahydrothienyl, dihydroimidazolyl, dihydrofuryl, dihydropyrazolyl, dihydropyrrolyl, piperidinyl, piperazinyl, morpholinyl, thiomorpholinyl, homopiperazinyl, azepinyl, 1,4-diazacycloheptyl, pyranyl, etc., preferably pyrrolidinyl, morpholinyl, piperidinyl, azepinyl, 1,4-diazacycloheptyl and piperazinyl. Polycyclic heterocyclyl includes spiro, fused and bridged heterocyclyl, wherein the spiro, fused and bridged heterocyclyl groups are optionally connected to other groups via a single bond, or further fused to other cycloalkyl, heterocyclyl, aryl and heteroaryl via any two or more atoms on the ring.

The term "spiroheterocyclyl" refers to 5- to 20-membered polycyclic heterocyclyl with monocyclic rings sharing one atom (called a spiro atom), wherein one or more ring atoms are heteroatoms selected from nitrogen, oxygen, or S(O)ₘ (wherein m is an integer of 0 to 2), and the remaining ring atoms are carbon. It may contain one or more double bonds, but no ring has a fully conjugated π electron system. Preferably, the group is 6- to 14-membered, and more preferably 7- to 10-membered. According to the number of shared spiro atoms between the rings, the spiroheterocyclyl is divided into monospiroheterocyclyl, bispiroheterocyclyl, or polyspiroheterocyclyl, preferably monospiroheterocyclyl and bispiroheterocyclyl. More preferably, the group is 3-membered/5-membered, 3-membered/6-membered, 4-membered/4-membered, 4-membered/5-membered, 4-membered/6-membered, 5-membered/5-membered, or 5-membered/6-membered monospiroheterocyclyl. Non-limiting examples of spiroheterocyclyl include: 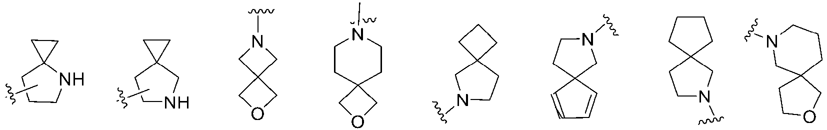 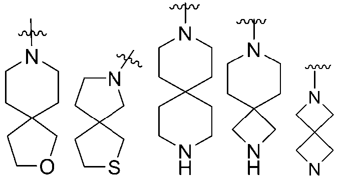 etc.

The term "fused heterocyclyl" refers to 5- to 20-membered polycyclic heterocyclyl with each ring in the system sharing a pair of atoms neighbouring other rings in the system, and one or more rings may contain one or more double bonds, but no ring has a fully conjugated π electron system, wherein one or more ring atoms are heteroatoms selected from nitrogen, oxygen, or S(O)ₘ (wherein m is an integer of 0 to 2), and the remaining ring atoms are carbon. Preferably, the group is 6- to 14-membered, and more preferably 7- to 10-membered. According to the number of constituent rings, it can be divided into bicyclic, tricyclic, tetracyclic or polycyclic fused heterocyclyl, preferably bicyclic or tricyclic, and more preferably 5-membered/5-membered or 5-membered/6-membered bicyclic fused heterocyclyl. Non-limiting examples of fused heterocyclyl include: 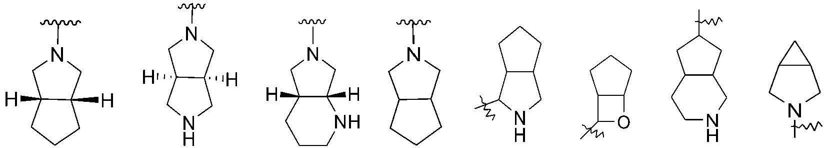 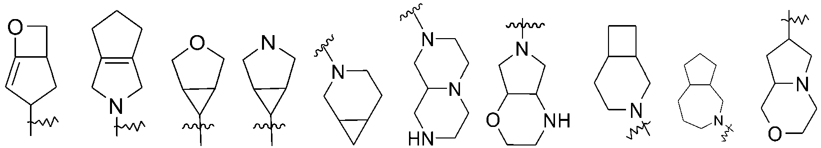 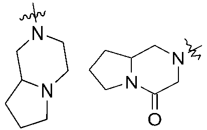 etc.

The term "bridged heterocyclyl" refers to a 5- to 14-membered polycyclic heterocyclyl with any two rings sharing two atoms that are not directly connected. It may contain one or more double bonds, but no ring has a fully conjugated π electron system, wherein one or more ring atoms are heteroatoms selected from nitrogen, oxygen, or S(O)ₘ (wherein m is an integer of 0 to 2), and the remaining ring atoms are carbon. Preferably, the group is 6- to 14-membered, and more preferably 7- to 10-membered. According to the number of constituent rings, it can be divided into bicyclic, tricyclic, tetracyclic or polycyclic bridged heterocyclyl, preferably bicyclic, tricyclic or tetracyclic, and more preferably bicyclic or tricyclic. Non-limiting examples of bridged heterocyclyl include: 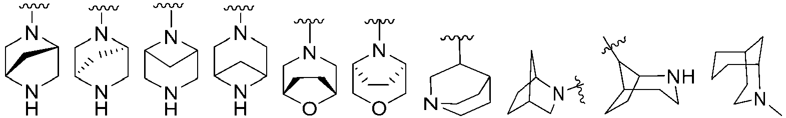 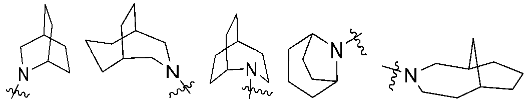 etc.

The heterocyclyl ring can be fused to an aryl, heteroaryl or cycloalkyl ring, wherein the ring connected to the parent structure is heterocyclyl, and non-limiting examples thereof include: 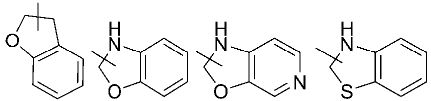 etc.

Heterocyclyl can be optionally substituted or unsubstituted. When the heterocyclyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group.

The term "aryl" refers to a 6- to 14-membered all-carbon monocyclic or fused polycyclic (i.e., rings sharing an adjacent pair of carbon atoms) group having a conjugated π electron system, preferably 6- to 12-membered aryl, such as phenyl and naphthyl, and more preferably phenyl. The aryl ring can be fused to a heteroaryl, heterocyclyl or cycloalkyl ring, including benzo 5- to 10-membered heteroaryl, benzo 3- to 8-membered cycloalkyl and benzo 3- to 8-membered heteroalkyl, preferably benzo 5- to 6-membered heteroaryl, benzo 3- to 6-membered cycloalkyl, and benzo 3- to 6-membered heteroalkyl, wherein the heterocyclyl is heterocyclyl containing 1-3 nitrogen atoms, oxygen atoms, or sulphur atoms; or further including a three-membered nitrogen-containing fused ring containing a benzene ring,

wherein the ring connected to the parent structure is an aryl ring, and non-limiting examples thereof include: 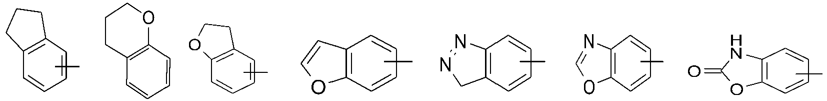 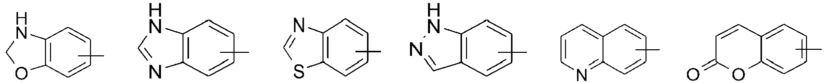 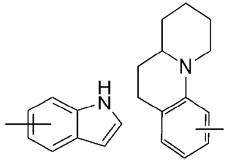 etc.

Aryl can be substituted or unsubstituted. When the aryl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "heteroaryl" refers to a heteroaromatic system containing 1 to 4 heteroatoms and 5 to 14 ring atoms, wherein the heteroatoms are selected from oxygen, sulphur, and nitrogen. The heteroaryl is preferably 5- to 12-membered, and more preferably 5-membered or 6-membered, such as imidazolyl, furyl, thienyl, thiazolyl, pyrazolyl, oxazolyl, pyrrolyl, triazolyl, tetrazolyl, pyridyl, pyrimidinyl, thiadiazole, pyrazinyl, etc., preferably triazolyl, thienyl, imidazolyl, pyrazolyl, oxazolyl, pyrimidinyl, or thiazolyl; more preferably pyrazolyl, pyrrolyl and oxazolyl. The heteroaryl ring can be fused to an aryl, heterocyclyl or cycloalkyl ring, wherein the ring connected to the parent structure is a heteroaryl ring, and non-limiting examples thereof include: 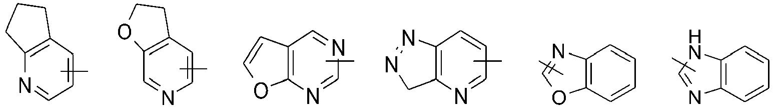 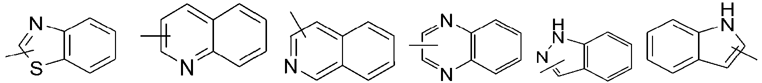 etc.

Heteroaryl can be optionally substituted or unsubstituted. When the heteroaryl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropoxy, cyclobutoxy, cyclopentoxy, and cyclohexoxy. Alkoxy can be optionally substituted or unsubstituted. When the alkoxy is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

"Haloalkyl" refers to alkyl substituted with one or more halogen, wherein the alkyl is as defined above.

"Haloalkoxy" refers to alkoxy substituted with one or more halogen, wherein the alkoxy is as defined above.

"Hydroxyalkyl" refers to alkyl substituted with hydroxyl, wherein the alkyl is as defined above.

"Alkenyl" refers to an alkene group, also known as an olefinic group, which is a linear or branched unsaturated aliphatic hydrocarbon group containing at least one carbon-carbon double bond that can be located at any position within the alkenyl group. The alkenyl is a linear or branched unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), 2 to 4 (C₂₋₄), or 2 to 3 (C₂₋₃) carbon atoms. Non-limiting examples of alkenyl include: 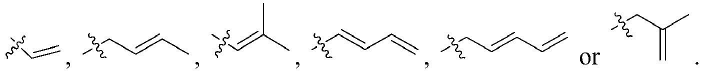 The alkenyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

"Alkynyl" refers to (CH=C-), which contains at least one carbon-carbon triple bond that can be located at any position within the alkynyl group and contains at least one carbon-carbon double bond that can be located at any position within the alkenyl group. The alkynyl is a linear or branched unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), 2 to 4 (C₂₋₄), or 2 to 3 (C₂₋₃) carbon atoms. Non-limiting examples of alkynyl include: 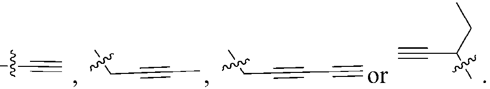 The alkynyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "alkenylcarbonyl" refers to -C(O)-(alkenyl), wherein the alkenyl is as defined above. Non-limiting examples of alkenylcarbonyl include: ethenylcarbonyl, propenylcarbonyl, and butenylcarbonyl. Alkenylcarbonyl can be optionally substituted or unsubstituted. When the alkenylcarbonyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

"Hydroxyl" refers to an -OH group.

"Halogen" refers to fluorine, chlorine, bromine, or iodine.

"Amino" refers to -NH₂.

"Cyano" refers to -CN.

"Nitro" refers to -NO₂.

"Carbonyl" refers to -C(O)-.

"Carboxyl" refers to -C(O)OH.

"THF" refers to tetrahydrofuran.

"DMF" refers to N,N-dimethylformamide.

Different expressions such as "X is selected from A, B, or C", "X is selected from A, B and C", "X is A, B or C", and "X is A, B and C" all express the same meaning, that is, X can be any one or more of A, B, and C.

The hydrogen atoms according to the present invention can be replaced with the isotope deuterium thereof, and any hydrogen atom in the example compounds involved in the present invention can also be replaced with a deuterium atom.

The compounds of the present invention include all pharmaceutically acceptable isotopically labelled compounds, wherein one or more atoms of the compounds disclosed in the present invention are replaced by atoms having the same atomic number but an atomic mass or mass number different from that normally found. Examples of isotopes that can be incorporated into the disclosed compounds include isotopes of hydrogen, carbon, nitrogen, oxygen, phosphorus, fluorine, chlorine, and iodine. Examples of such isotopes include deuterium, tritium, etc.

"Optional" or "optionally" means that the event or circumstance subsequently described may but not necessarily occur, and the description includes the occasion where the event or circumstance occurs or does not occur. For example, "heterocyclic group optionally substituted with alkyl" means the alkyl may but need not be present, the description includes the case where the heterocyclic group is substituted with alkyl and the case where the heterocyclic group is not substituted with alkyl.

"Substituted" means that one or more hydrogen atoms, preferably at most 5, more preferably 1-3 hydrogen atoms in the group are each independently substituted with a corresponding number of substituents. It goes without saying, the substituents may be only in possible chemical positions thereof, and those skilled in the art can determine the possible or impossible substitutions (by experiment or theory) without paying too much effort. For example, an amino or hydroxyl group with free hydrogen may be unstable when attached to a carbon atom with an unsaturated (e.g., olefinic) bond.

"Pharmaceutical composition" denotes a mixture containing one or more compounds described herein or physiologically/pharmaceutically acceptable salts or prodrugs thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

"Pharmaceutically acceptable salts" and "pharmaceutical salts" refer to salts of the compounds of the present invention, which are safe and effective when used in mammals, and have appropriate biological activity.

The compounds of the present invention, such as those of formulas (II-A1) to (II-19b) or specific compounds, are intended to include one or more of the following: the free base of the compound, or the pharmaceutically acceptable salt, stereoisomer, or a mixture of two or more stereoisomers thereof. Stereoisomers refer to compounds that differ only in their spatial arrangement. Stereoisomers include all diastereomeric and enantiomeric forms of the compound. Enantiomers are stereoisomers that are mirror images of each other. Diastereomers are stereoisomers that have two or more chiral centres which are not identical and are not mirror images of each other.

### Detailed Description of Embodiments

### Examples

The structures of compounds are determined by nuclear magnetic resonance (NMR) or/and mass spectrometry (MS). The NMR shift (δ) is given in 10⁻⁶ (ppm). NMR is determined with Bruker AVANCE-400 nuclear magnetic resonance spectrometer. The solvents for determination are deuterated dimethyl sulphoxide (DMSO-*d₆*), deuterated chloroform (CDCl₃), and deuterated methanol (CD₃OD), and the internal standard is tetramethylsilane (TMS).

MS is determined with a FINNIGAN LCQAd (ESI) mass spectrometer (manufacturer: Thermo, model: Finnigan LCQ advantage MAX).

HPLC is determined with Agilent 1200DAD high-pressure liquid chromatograph instrument (Sunfire C18 150 × 4.6 mm chromatographic column) and Waters 2695-2996 high-pressure liquid chromatograph instrument (Gimini C18 150 × 4.6 mm chromatographic column).

The average kinase inhibition rate and IC₅₀ value are determined with NovoStar microplate reader (BMG Company, Germany).

For thin layer chromatography (TLC), silica gel plates from Yantai Huanghai HSGF254 or Qingdao GF254 are used. Silica gel plates with a thickness of 0.15 mm-0.2 mm are employed for TLC, while plates with a thickness of 0.4 mm-0.5 mm are used for TLC separation and purification.

For column chromatography, Yantai Huanghai silica gel of 200-300 mesh is typically used as a carrier.

The known starting materials of the present invention can be synthesised by methods known in the art, or can be purchased from ABCR GmbH & Co. KG, Acros Organics, Aldrich Chemical Company, Accela ChemBio Inc., Chembee Chemicals Inc. and other companies.

Unless otherwise specially specified in examples, reactions can all be carried out under an argon atmosphere or a nitrogen atmosphere.

Argon atmosphere or nitrogen atmosphere means that a reaction flask is connected to an argon or nitrogen balloon with a volume of about 1 L.

Hydrogen atmosphere means that a reaction flask is connected to a hydrogen balloon with a volume of about 1 L.

Pressurised hydrogenation reactions are performed using either Parr 3916EKX hydrogenation apparatus with Qinglan QL-500 hydrogen generator or HC2-SS hydrogenation apparatus.

Hydrogenation reactions are typically performed by evacuating the system and refilling it with hydrogen gas, repeating this operation three times.

Microwave reactions are performed using CEM Discover-S 908860 microwave reactor.

Unless otherwise specially specified in examples, a solution refers to an aqueous solution.

Unless otherwise specially specified in examples, the reaction temperature is room temperature, which is 20°C-30°C.

The progress of the reactions in examples is monitored by thin layer chromatography (TLC). Developing agent systems used in the reactions include: A: dichloromethane and methanol system, B: n-hexane and ethyl acetate system, C: petroleum ether and ethyl acetate system, and D: acetone, wherein the volume ratio between the solvents can be adjusted depending on the polarity of the compounds.

Eluent systems for column chromatography and developing agent systems for thin layer chromatography, which are used to purify compounds, include: A: dichloromethane and methanol system, B: n-hexane and ethyl acetate system, and C: dichloromethane and acetone system, wherein the volume ratio between the solvents can be adjusted depending on the polarity of the compounds, or can also be adjusted by adding a small amount of basic or acidic reagents such as triethylamine and acetic acid.

### Reference Example 1

### 1-(2-Chloro-4-methyl-5,7-dihydro-6H-pyrrolo[3,4-d]pyrimidin-6-yl)-2-(1-(2-(trifluoromethyl)pyridin-4-yl)azetidin-3-yl)ethan-1-one

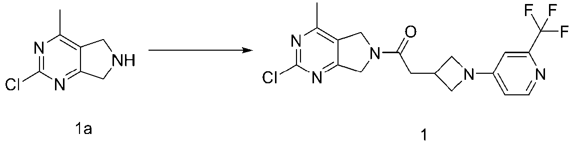

### 1-(2-Chloro-4-methyl-5,7-dihydro-6H-pyrrolo[3,4-d]pyrimidin-6-yl)-2-(1-(2-(trifluoromethyl)pyridin-4-yl)azetidin-3-yl)ethan-1-one

2-Chloro-4-methyl-6,7-dihydro-5*H*-pyrrolo[3,4-d]pyrimidine **1a** (50 mg, 0.29 mmol) was dissolved in *N,N*-dimethylformamide (2.0 mL). 2-(1-(2-(Trifluoromethyl)pyridin-4-yl)azetidin-3-yl)acetic acid (77 mg, 0.29 mmol, prepared by a known method as described in patent WO 2018002760), 2-(7-azabenzotriazol-1-yl)-*N*,*N*,*N'*,*N*'-tetramethyluronium hexafluorophosphate (168 mg, 0.44 mmol) and diisopropylethylamine (114 mg, 0.88 mmol) were added sequentially to the reaction system. The mixture was reacted at 25°C for 2 hours. Water (10 mL) was added to the reaction solution, and the mixture was extracted with ethyl acetate (10 mL×2). The organic phases were combined, washed with a saturated sodium chloride solution (10 mL×2), dried over anhydrous sodium sulphate, and filtered. The filtrate was concentrated under reduced pressure, and the resulting residue was purified by preparative HPLC, to afford title product **1** (35 mg, yield: 28.9%). MS m/z (ESI): 412.2 [M+1]

### Reference Example 2

### (3-Chloro-2,4-dimethyl-5,7-dihydro-6H-pyrrolo[3,4-b]pyridin-6-yl)(3-chloro-4-fluorophenyl)methanone

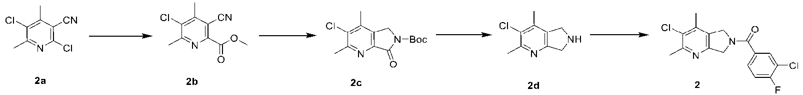

### Methyl 5-chloro-3-cyano-4,6-dimethylpicolinate

**2a** (50 g, 0.2487 mol) was dissolved in methanol (1000 mL). DPPF (13.78 g, 0.02486 mol), Et₃N (49.32 g, 0.0.4874 mol), and Pd(OAc)₂ (2.8 g, 0.01247 mol) were added. The mixture was purged three times with CO, then maintained at a pressure of 0.25 MPa, and heated to 70°C and stirred for 16 h. Upon completion of the reaction as monitored by LC/MS, the reaction solution was subjected to rotary evaporation to dryness, and the residue was subjected to column chromatography (EA:PE=1:5), to afford product **2b** (40 g, yield: 71.6%). MS m/z (ESI): 225.6.

**2b** (15 g, 0.07 mol) was dissolved in methanol (150 mL), NiCl₂·6H₂O (25.45 g, 0.7 mol) was added, and the mixture was stirred for 5 min and purged with a N₂ balloon. NaBH₄ (31.8 g, 0.1 mol) was added portionwise in an ice bath, and the mixture was stirred at room temperature for 1 h. The reaction solution was subjected to rotary evaporation to dryness, quenched with 2M HCl solution in an ice bath, and then adjusted to pH 9-10 by adding 2N NaOH solution dropwise. Boc anhydride (21.92 g, 0.1004 mol) was added, and the mixture was stirred at room temperature for 2 h, and extracted three times with EA (200 ml). The organic phases were combined, and dried over anhydrous sodium sulphate, to afford crude **2c** (20 g), which was used directly in the next step. MS m/z (ESI): 297.8.

**2c** (20 g, 0.07 mol) was dissolved in THF (200 mL). 1M BH₃-THF (169 mL, 0.1 mol) was added dropwise in an ice-water bath. The mixture was stirred at room temperature for 10 min, and then heated to reflux for 16 h. After the reaction solution was cooled to room temperature, methanol (50 mL) was added to quench BH3-THF. The methanol was added slowly at the beginning to prevent bumping. After the addition was completed, the mixture was heated to reflux for 3-4 h and then cooled to room temperature. The reaction solution was subjected to rotary evaporation to dryness, and the residue was subjected to column chromatography (EA:PE=1:5), to afford product (3 g). The Boc-containing product was dissolved in EA (15 mL), 4M EA/HCL solution (15 ml) was added, and the mixture was stirred for 16 h. Upon completion of the reaction as monitored by LCMS, the reaction solution was subjected to rotary evaporation to dryness, to afford crude **2d** (2.6 g, yield: 21.1%). MS m/z (ESI): 183.7.

Referring to the synthesis method of Reference Example 1, Reference Example 2 was obtained. MS m/z (ESI): 340.2.

### Example 20

### 1-(5,6-Dimethyl-7,9-dihydro-8H-pyrrolo[3,4-c][1,2,4]triazolo[1,5-a]pyridin-8-yl)-2-(1-(2-(trifluoromethyl)pyridin-4-yl)azetidin-3-yl)ethan-1-one

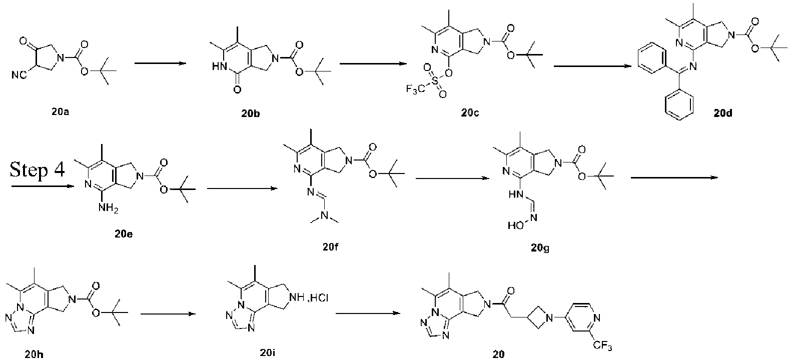

### tert-Butyl 6,7-dimethyl-4-oxo-1,3,4,5-tetrahydro-2H-pyrrolo[3,4-c]pyridine-2-carboxylate

Polyphosphoric acid (100 g) was added to a mixture of **20a** (5 g, 23 .8 mmol) and 2-butanone (20 mL) at 100°C, and then the resulting mixture was heated to 120°C and stirred for 4 hours. The reaction mixture was cooled to 15°C, and then quenched with water (1.5 L), and adjusted to pH 10-11 with solid sodium hydroxide. Di-tert-butyl dicarbonate (10 g, 45.8 mmol) was added, and then the reaction mixture was stirred overnight at 15°C. Upon completion of the reaction, the system was filtered. The collected solids were washed sequentially with water (7 x 50 mL) and a mixture of tert-butyl methyl ether and petroleum ether (1:1, 7 x 40 mL), to afford crude Example 20b. MS m/z (ESI): 265.3 [M+1].

### tert-Butyl 6,7-dimethyl-4-((trifluoromethyl)sulphonyl)oxy-1,3-dihydro-2H-pyrrolo[3,4-c]pyridine-2-carboxylate

At 0°C, triethylamine (25 g, 25 mmol) and trifluoromethanesulphonic anhydride (5.9 g, 21 mmol) were added separately to a solution of **20b** (5 g, 19 mmol) in dichloromethane (200 mL). The reaction mixture was stirred at 0°C for 2 hours, and then quenched with water (5 mL), and extracted with dichloromethane (3 x 40 mL). The combined organic layers were washed with a saturated sodium chloride aqueous solution (100 mL), dried over anhydrous sodium sulphate, filtered, and concentrated in vacuo. The residue was subjected to silica gel chromatography (eluents: petroleum ether/ethyl acetate = 5: 1), to afford product **20c** (4 g, yield: 58%). MS m/z (ESI): 397.4.

### tert-Butyl 4-(diphenylmethylene)amino)-6,7-dimethyl-1,3-dihydro-2H-pyrrolo[3,4-c]pyridine-2-carboxylate

To a solution of **20c** (9.2 g, 23.2 mmol) in toluene (100 mL) were added 1,1-diphenylmethanimine (6.31 g, 34.8 mmol), palladium acetate (521 mg, 2.32 mmol), 2,2'-bis(diphenylphosphino)-1,1'-binaphthyl (BINAP; 1.45 g, 2.33 mmol) and caesium carbonate (11.3 g, 34.7 mmol). The mixture was heated at 100°C for 16 hours, and then filtered, and the filtrate was concentrated in vacuo to afford crude 20d, which was used directly in the next step. MS m/z (ESI): 428.6.

### tert-Butyl 4-amino-6,7-dimethyl-1,3-dihydro-2H-pyrrolo[3,4-c]pyridine-2-carboxylate

To a solution of crude **20d** in methanol (100 mL) were added sodium acetate (4.0 g, 48.8 mmol) and hydroxylamine hydrochloride (3.39 g, 48.8 mmol). The reaction mixture was stirred at room temperature for 72 hours. Subsequently, the system was filtered, and the filtrate was concentrated under reduced pressure. The residue was recrystallised from ethyl acetate to afford **20e** (5 g, yield: 82%).

MS m/z (ESI): 264.3.

### tert-Butyl (E)-4-(((dimethylamino)methylene)amino)-6,7-dimethyl-1,3-dihydro-2H-pyrrolo[3,4-c]pyridine-2-carboxylate

At room temperature, 1,1-dimethoxy-*N,N-dimethylmethanamine* (1.63 g, 13.68 mmol) was added to a solution of **20e** in toluene (10 mL). The reaction mixture was heated to reflux for 2 h, and then cooled to room temperature. The system was directly concentrated to afford crude **20f,** which was used directly in the next step. MS m/z (ESI): 319.4.

### tert-Butyl (E)-4-(N'-hydroxycarbamimidoyl)-6,7-dimethyl-1,3-dihydro-2H-pyrrolo[3,4-c]pyridine-2-carboxylate

At room temperature, hydroxylamine hydrochloride (6.6 g, 94.3 mmol) was added to a solution of **20f** in methanol (20 mL). The reaction mixture was refluxed for 1 h, and then cooled to room temperature. The reaction mixture was quenched with water (15 mL), and extracted with ethyl acetate. The organic phase was washed with a saturated sodium chloride aqueous solution, dried over anhydrous sodium sulphate, and then directly concentrated to afford crude **20g,** which was used directly in the next step. MS m/z (ESI): 307.4.

### tert-Butyl 5,6-dimethyl-7,9-dihydro-8H-pyrrolo[3,4-c][1,2,4]triazolo[1,5-a]pyridine-8-carboxylate

At 0°C, trifluoroacetic anhydride (0.83 g, 3.93 mmol) was added to a solution of **20g** in THF (20 mL). The reaction mixture was stirred overnight at room temperature, and then concentrated. The residue was purified by silica gel chromatography (gradient: 33% to 100% ethyl acetate/petroleum ether), finally yielding product **20h** (450 mg, yield: 42%). MS m/z (ESI): 289.

### 5,6-Dimethyl-8,9-dihydro-7H-pyrrolo[3,4-c][1,2,4]triazolo[1,5-a]pyridine

To a solution of 20h (450 mg, 1.56 mmol) in ethyl acetate (10 mL) was added a solution of hydrogen chloride in ethyl acetate (4M, 10 mL) at 0°C. The reaction mixture was stirred at room temperature for 5 hours. The solvent was removed under reduced pressure to afford product **20i** (300 mg, yield: 85%). MS m/z (ESI): 189.2.

### 1-(5,6-Dimethyl-7,9-dihydro-8H-pyrrolo[3,4-c][1,2,4]triazolo[1,5-a]pyridin-8-yl)-2-(1-(2-(trifluoromethyl)pyridin-4-yl)azetidin-3-yl)ethan-1-one

Referring to the synthesis method of Reference Example 1, Example 20 was obtained. MS m/z (ESI): 431.4.

### Example 32

### 1-(2-(Trifluoromethyl)pyridin-4-yl)azetidin-3-yl 5,6-dimethyl-7,9-dihydro-8H-pyrrolo[3,4-c][1,2,4]triazolo[1,5-a]pyridine-8-carboxylate

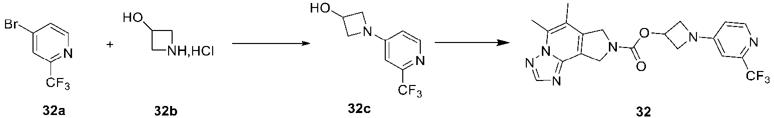

**32a** (500 mg, 2.21 mmol) was dissolved in a mixture of dioxane (5 mL) and water (1 mL). **32b** (270 mg, 2.43 mmol), 4,5-bis(diphenylphosphino)-9,9-dimethylxanthene (128 mg, 0.22 mmol), palladium acetate (49.67 mg, 0.2212 mmol) and caesium carbonate (2.16 g, 6.6372 mmol) were added. The mixture was purged three times with nitrogen, and heated to 100°C and stirred under reflux for 16 h. Upon completion of the reaction, the reaction solution was subjected to rotary evaporation to dryness, to afford product **32c** (300 mg). MS m/z (ESI): 219.2 [M+1]

32c (100 mg, 0.46 mmol) was dissolved in dry dichloromethane (1 mL). Dimethylaminopyridine (112 mg, 0.92 mmol) and BTC (112 mg, 0.38 mmol) were added, and the mixture was stirred for 2 h. **20i** (257 mg, 1.15 mmol) and triethylamine (464 mg, 4.5871 mmol) were then added, and the resulting mixture was stirred at room temperature for 16 h. The reaction solution was subjected to rotary evaporation to dryness, to afford product **32** (32.6 mg, yield: 16.3%). MS m/z (ESI): 433.4 [M+1].

### Example 39

### 1-(6-Methyl-2,3,7,9-tetrahydro-[1,4]oxazino[3,2-e]isoindol-8(1H)-yl)-2-(1-(2-(trifluoromethyl)pyridin-4-yl)azetidin-3-yl)ethan-1-one

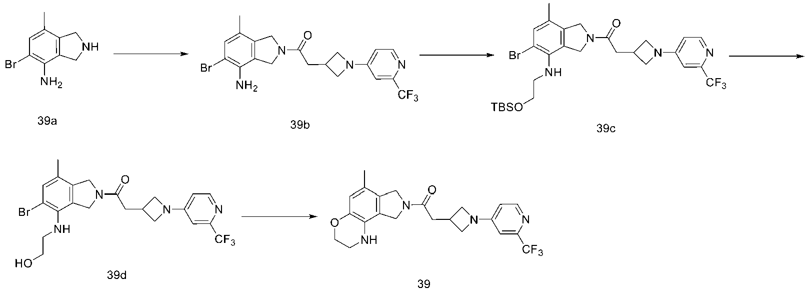

### 1-(4-Amino-5-bromo-7-methylisoindol-2-yl)-2-(1-(2-(trifluoromethyl)pyridin-4-yl)azetidin-3-yl)ethan-1-one

Referring to the synthesis method of Reference Example 1, title product **39b** was obtained. MS m/z (ESI): 471.2 [M+1]

### 1-(5-Bromo-4-((2-((tert-butyldimethylsilyl)oxy)ethyl)amino)-7-methylisoindolin-2-yl)-2-(1-(2-(trifluoromethyl)pyridin-4-yl)azetidin-3-yl)ethan-1-one

**39b** (200 mg, 0.43 mmol) was dissolved in anhydrous tetrahydrofuran (5.0 mL), and sodium hydride (34 mg, 0.85 mmol, 60%) was added to the reaction system. The mixture was stirred at 25°C for 1 hour, and then (2-bromoethoxy)(tert-butyl)dimethylsilane (154 mg, 0.65 mmol) was added to the reaction system. The resulting mixture was heated to 60°C and reacted for 15 hours. The system was cooled to room temperature. A saturated ammonium chloride aqueous solution (20 mL) was added to the reaction solution, and the mixture was extracted with ethyl acetate (10 mL×3). The organic phases were combined, washed with a saturated sodium chloride solution (10 mL×2), dried over anhydrous sodium sulphate, and filtered. The filtrate was concentrated under reduced pressure, and the resulting residue was purified by silica gel column chromatography using eluent system B, to afford title product **39c** (185 mg, yield: 69.3%). MS m/z (ESI): 629.2 [M+1]

### 1-(5-Bromo-4-((2-hydroxyethyl)amino)-7-methylisoindolin-2-yl)-2-(1-(2-(trifluoromethyl)pyridin-4-yl)azetidin-3-yl)ethan-1-one

In an ice bath, **39c** (185 mg, 0.29 mmol) was dissolved in dioxane (2 mL). A mixed solution of hydrogen chloride in 1,4-dioxane (4 M, V/V = 1:1, 2 mL) was added, and the mixture was reacted at 25°C for 2 hours. The reaction solution was adjusted to pH above 7 with a saturated sodium bicarbonate solution, and extracted with dichloromethane (10 mL×3). The organic phases were combined, washed with a saturated sodium chloride solution (10 mL×2), dried over anhydrous sodium sulphate, and filtered. The filtrate was concentrated under reduced pressure, and the resulting residue was purified by silica gel column chromatography using eluent system A, to afford title product **39d** (128 mg, yield: 84.8%). MS m/z (ESI): 513.2 [M+1]

### 1-(6-Methyl-2,3,7,9-tetrahydro-[1,4]oxazino[3,2-e]isoindol-8(1H)-yl)-2-(1-(2-(trifluoromethyl)pyridin-4-yl)azetidin-3-yl)ethan-1-one

**39d** (100 mg, 0.19 mmol) was dissolved in toluene (2.0 mL). Palladium acetate (4.4 mg, 0.02 mmol), 4,5-bis(diphenylphosphino)-9,9-dimethoxyxanthene (23 mg, 0.04 mmol) and caesium carbonate (191 mg, 0.58 mmol) were added sequentially to the reaction system. The resulting mixture was heated to 100°C and reacted for 15 hours. The system was cooled to room temperature. Water (10 mL) was added to the reaction solution, and the mixture was extracted with ethyl acetate (10 mL×3). The organic phases were combined, washed with a saturated sodium chloride solution (10 mL×2), dried over anhydrous sodium sulphate, and filtered. The filtrate was concentrated under reduced pressure, and the resulting residue was purified by preparative HPLC, to afford title product **39** (25 mg, yield: 29.8%). MS m/z (ESI): 433.2 [M+1]

### Example 42

### 1-(5-Methyl-6,8-dihydro-[1,2,3]triazolo[4,5-e]isoindol-7(1H)-yl)-2-(1-(2-(trifluoromethyl)pyridin-4-yl)azetidin-3-yl)ethan-1-one

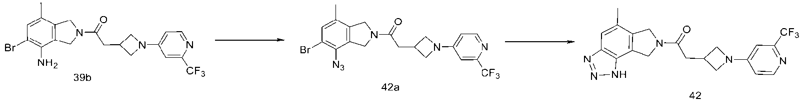

### 1-(4-Azido-5-bromo-7-methylisoindol-2-yl)-2-(1-(2-(trifluoromethyl)pyridin-4-yl)azetidin-3-yl)ethan-1-one

In an ice bath, **39b** (200 mg, 0.43 mmol) was dissolved in concentrated hydrochloric acid (6.0 M, 5.0 mL). 30% sodium nitrite aqueous solution (35 mg, 0.51 mmol) was added, and the mixture was reacted at 0°C for 0.5 hours. Sodium azide (84 mg, 1.29 mmol) was added to the reaction solution. The mixture was reacted at 25°C for 1 hour. The reaction solution was adjusted to pH above 7 with a saturated sodium bicarbonate solution, and extracted with dichloromethane (10 mL×3). The organic phases were combined, washed with a saturated sodium chloride solution (10 mL×2), dried over anhydrous sodium sulphate, and filtered. The filtrate was concentrated under reduced pressure, and the resulting residue was purified by silica gel column chromatography using eluent system A, to afford title product 1-(4-azido-5-bromo-7-methylisoindol-2-yl)-2-(1-(2-(trifluoromethyl)pyridin-4-yl)azetidin-3-yl)ethan-1-one **42a** (174 mg, yield: 82.5%). MS m/z (ESI): 497.2 [M+1]

### 1-(5-Methyl-6,8-dihydro-[1,2,3]triazolo[4,5-e]isoindol-7(1H)-yl)-2-(1-(2-(trifluoromethyl)pyridin-4-yl)azetidin-3-yl)ethan-1-one

At -78°C, **42a** (100 mg, 0.20 mmol) was dissolved in anhydrous tetrahydrofuran (2.0 mL). n-Butyllithium (0.12 mL, 0.30 mmol, 2.5 M) was added to the reaction system. The mixture was reacted at 25°C for 2 hours. A saturated ammonium chloride aqueous solution (10 mL) was added to the reaction solution, and the mixture was extracted with ethyl acetate (10 mL×3). The organic phases were combined, washed with a saturated sodium chloride solution (10 mL×2), dried over anhydrous sodium sulphate, and filtered. The filtrate was concentrated under reduced pressure, and the resulting residue was purified by preparative HPLC, to afford title product **42** (33 mg, yield: 39.3%). MS m/z (ESI): 417.2 [M+1]

### Example 43

### 1-(5-Methyl-6,8-dihydroimidazo[4,5-e]isoindol-7(1H)-yl)-2-(1-(2-(trifluoromethyl)pyridin-4-yl)azetidin-3-yl)ethan-1-one

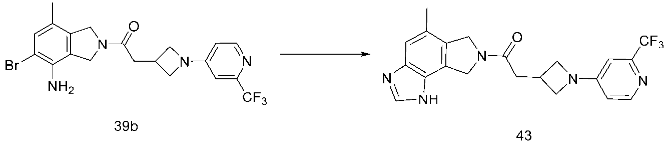

### 1-(5-Methyl-6,8-dihydroimidazo[4,5-e]isoindol-7(1H)-yl)-2-(1-(2-(trifluoromethyl)pyridin-4-yl)azetidin-3-yl)ethan-1-one

**39b** (200 mg, 0.21 mmol) was dissolved in 30% aqueous ammonia (2.0 mL). Paraformaldehyde (32 mg, 1.1 mmol), cuprous iodide (4 mg, 0.02 mmol), 1,10-phenanthroline (7.3 mg, 0.04 mmol) and sodium carbonate (67 mg, 0.63 mmol) were added sequentially to the reaction system. The resulting mixture was heated to 100°C and reacted for 10 hours. Water (10 mL) was added to the reaction solution, and the mixture was extracted with ethyl acetate (10 mL×2). The organic phases were combined, washed with a saturated sodium chloride solution (10 mL×2), dried over anhydrous sodium sulphate, and filtered. The filtrate was concentrated under reduced pressure, and the resulting residue was purified by preparative HPLC, to afford title product **43** (27 mg, yield: 30.7%). MS m/z (ESI): 416.2 [M+1].

### Example 60

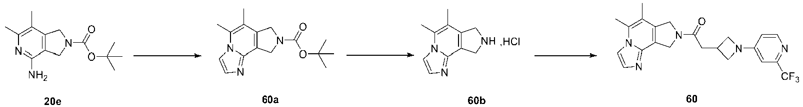

### tert-Butyl 5,6-dimethyl-7,9-dihydro-8H-imidazo[1,2-a]pyrrolo[3,4-c]pyridine-8-carboxylate

At room temperature, sodium bicarbonate (3.2 g, 38 mmol, 2 eq) and **20e** (5 g, 19 mmol, 1 eq) were added to a reaction flask, followed by ethanol (30 mL). Chloroacetaldehyde (4.5 g, 22.8 mmol, 1.2 eq) (40% aqueous solution) was added dropwise while stirring. The reaction mixture was stirred overnight at 80°C, and then cooled to room temperature. The system was extracted with dichloromethane (50 mL×3), washed with a sodium chloride aqueous solution (20 mL×3), and then dried, concentrated, and subjected to column chromatography (eluents: dichloromethane/methanol = 5%-10%), to afford **60a** (3 g, yield: 55%). MS m/z (ESI): 288.4.

### 5,6-Dimethyl-8,9-dihydro-7H-imidazo[1,2-a]pyrrolo[3,4-c]pyridine

At 0°C, a solution of hydrogen chloride in ethyl acetate (4M, 10 mL) was added to a solution of **60a** (450 mg, 1.56 mmol) in ethyl acetate (10 mL). The reaction mixture was stirred at room temperature for 5 hours. The solvent was removed under reduced pressure to afford **60b** (297 mg, yield: 85%). MS m/z (ESI): 188.2.

### 1-(5,6-Dimethyl-7,9-dihydro-8H-imidazo[1,2-a]pyrrolo[3,4-c]pyridin-8-yl)-2-(1-(2-(trifluoromethyl)pyridin-4-yl)azetidin-3-yl)ethan-1-one

Referring to the synthesis method of Reference Example 1, Example **60** (60 mg, yield: 21.5%) was obtained. MS m/z (ESI): 430.4.

### Example 76

### 1-(4,5-Dimethyl-2,3-dihydro-1H-pyrrolo[4,3-c]pyrazolo[1,5-a]pyridin-2-yl)-2-{1-[2-(trifluoromethyl)pyridin-4-yl]azetidin-3-yl}ethan-1-one

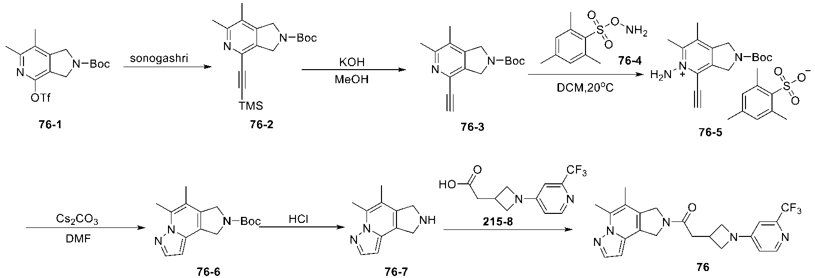

### 2-Methylpropan-2-yl 6,7-dimethyl-4-[(trimethylsilyl)ethynyl]-2,3-dihydro-1H-pyrrolo[4,3-c]pyridine-2-carboxylate

Compound **76-1** (1 g, 2.52 mmol) was dissolved in triethylamine (10 mL). Bis(triphenylphosphine)palladium(II) dichloride (89 mg, 126.14 umol), cuprous iodide (96 mg, 504.56 umol) and trimethylsilylacetylene (496 mg, 5.05 mmol, 713 uL) were added sequentially. The mixture was purged three times with nitrogen, and then stirred and reacted at 50°C under a nitrogen atmosphere for 12 hours. The reaction solution was cooled to room temperature, and directly concentrated under reduced pressure. The residue was dissolved in ethyl acetate (2 mL). The organic phase was washed sequentially with a saturated ammonium chloride aqueous solution (10 mL×2) and a saturated sodium chloride aqueous solution (10 mL), dried over anhydrous sodium sulphate, and filtered. The filtrate was concentrated under reduced pressure to afford title product **76-2** (1 g, crude).

MS m/z (ESI): 345.2 [M+1]

### 2-Methylpropan-2-yl 4-ethynyl-6,7-dimethyl-2,3-dihydro-1H-pyrrolo[4,3-c]pyridine-2-carboxylate

Compound **76-2** (1 g, 2.52 mmol) was dissolved in methanol (20 mL). Potassium hydroxide (89 mg, 126.14 umol) was added, and then the mixture was stirred and reacted at 20°C for 1 hour. The reaction solution was diluted with water (40 mL), and extracted with ethyl acetate (20 mL×2). The organic phases were combined, washed with a saturated sodium chloride aqueous solution (20 mL), dried over anhydrous sodium sulphate, and filtered. The filtrate was concentrated under reduced pressure, and the residue was separated and purified by column chromatography (eluent system A) to afford title product **76-3** (400 mg, yield: 50.6%). MS m/z (ESI): 273.2 [M+1]

### 5-Amino-4-ethynyl-6,7-dimethyl-2-{[(2-methylpropan-2-yl)oxy]carbonyl}-2,3-dihydro-1H-pyrrolo[4,3-c]pyridin-5-ium

Compound **76-3** (100 mg, 367.19 umol) was dissolved in dichloromethane (5 mL). Compound **76-4** (87 mg, 403.90 umol) was added, and then the mixture was stirred and reacted at 20°C for 12 hours. The reaction solution was directly concentrated under reduced pressure, to afford title product **76-5** (200 mg, crude). MS m/z (ESI): 288.2 [M]

### 2-Methylpropan-2-yl 4,5-dimethyl-2,3-dihydro-1H-pyrrolo[4,3-c]pyrazolo[1,5-a]pyridine-2-carboxylate

Compound **76-5** (200 mg) was dissolved in anhydrous *N,N*-dimethylformamide (4 mL). Caesium carbonate (678 mg, 2.08 mmol) was added, and then the mixture was stirred and reacted at 20°C for 12 hours. The reaction solution was diluted with water (20 mL), and extracted with ethyl acetate (10 mL×2). The organic phases were combined, washed with a saturated sodium chloride aqueous solution (10 mL), dried over anhydrous sodium sulphate, and filtered. The filtrate was concentrated under reduced pressure, and the residue was separated and purified by column chromatography (eluent system A) to afford title product **76-6** (20 mg, yield: 10.0%).

MS m/z (ESI): 288.2 [M+1]

### 4,5-Dimethyl-2,3-dihydro-1H-pyrrolo[4,3-c]pyrazolo[1,5-a]pyridine

Compound **76-6** (20 mg) was dissolved in dichloromethane (2 mL). A hydrogen chloride-dioxane solution (2 mL, 4 M) was added, and then the mixture was stirred and reacted at 20°C for 2 hours. The reaction solution was directly concentrated under reduced pressure to afford title product **76-7** (20 mg, crude, hydrochloride).

MS m/z (ESI): 188.1 [M+1]

### 1-(4,5-Dimethyl-2,3-dihydro-1H-pyrrolo[4,3-c]pyrazolo[1,5-a]pyridin-2-yl)-2-{1-[2-(trifluoromethyl)pyridin-4-yl]azetidin-3-yl} ethan-1-one

Compound **76-7** (20 mg) and compound **76-8** (25 mg) were dissolved in anhydrous *N,N*-dimethylformamide (2 mL). Triethylamine (67 uL) and O-(7-azabenzotriazol-1-yl)-N,N,N',N'-tetramethyluronium hexafluorophosphate (40 mg) were added sequentially, and then the mixture was stirred and reacted at 20°C for 1 hour. The reaction solution was diluted with water (20 mL), and extracted with ethyl acetate (10 mL×2). The organic phases were combined, washed with a saturated sodium chloride aqueous solution (10 mL), dried over anhydrous sodium sulphate, and filtered. The filtrate was concentrated under reduced pressure, and the residue was separated and purified by high performance liquid chromatography (0.1% formic acid/acetonitrile/water) to afford title product 1-(4,5-dimethyl-2,3-dihydro-1*H*-pyrrolo[4,3-c]pyrazolo[1,5-a]pyridin-2-yl)-2-{1-[2-(trifluoromethyl)pyridin-4-yl]azetidin-3-yl}ethan-1-one **76** (10 mg).

MS m/z (ESI): 430.2 [M+1]

### Example 77

### 1-(1,5-Dimethyl-6,8-dihydroimidazo[4,5-e]isoindol-7(1H)-yl)-2-(1-(2-(trifluoromethyl)pyridin-4-yl)azetidin-3-yl)ethan-1-one

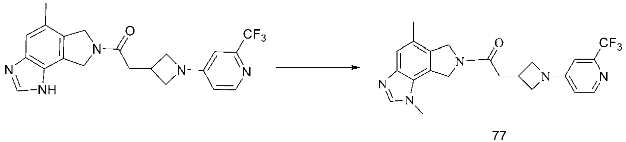

### 1-(1,5-Dimethyl-6,8-dihydroimidazo[4,5-e]isoindol-7(1H)-yl)-2-(1-(2-(trifluoromethyl)pyridin-4-yl)azetidin-3-yl)ethan-1-one

1-(5-Methyl-6,8-dihydroimidazo[4,5-*e*]isoindol-7(1*H*)-yl)-2-(1-(2-(trifluoromethyl)pyridin-4-yl)azetidin-3-yl)ethan-1-one (50 mg, 0.12 mmol) was dissolved in *N,N*-dimethylformamide (2.0 mL). Potassium carbonate (50 mg, 0.36 mmol) and iodomethane (26 mg, 0.18 mmol) were added sequentially to the reaction system. The resulting mixture was heated to 50°C and reacted for 2 hours. Water (10 mL) was added to the reaction solution, and the mixture was extracted with ethyl acetate (10 mL×2). The organic phases were combined, washed with a saturated sodium chloride solution (10 mL×2), dried over anhydrous sodium sulphate, and filtered. The filtrate was concentrated under reduced pressure, and the resulting residue was purified by preparative HPLC, to afford title product 77 (18 mg, yield: 34.8%). MS m/z (ESI): 430.2 [M+1]

### Example 78

### 1-(1,6-Dimethyl-2,3,7,9-tetrahydro-[1,4]oxazino[3,2-e]isoindol-8(1H)-yl)-2-(1-(2-(trifluoromethyl)pyridin-4-yl)azetidin-3-yl)ethan-1-one

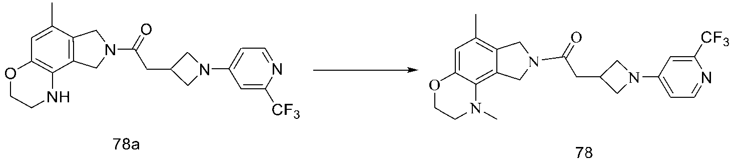

Referring to the synthesis method of Example **77,** title product **78** was obtained from starting material 78a.

MS m/z (ESI): 447.2 [M+1]

### Example 103

### 1-(5,6-Dimethyl-7,9-dihydro-8H-pyrrolo[3,4-c][1,2,4]triazolo[1,5-a]pyridin-8-yl)-2-((1S,2R)-2-(6-fluoropyridin-3-yl)cyclopropyl)ethan-1-one

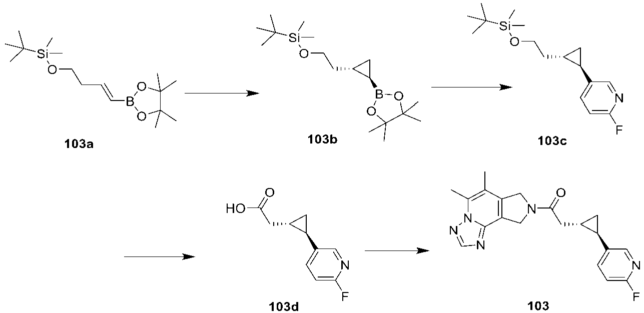

Under a nitrogen atmosphere at -40°C, diiodomethane (5.7 g, 21 mmol, 6.1 eq.) was added dropwise to a solution of diethylzinc (1 M, 10.5 mL, 3 eq.) in dichloromethane (25 mL). The mixture was stirred for two hours, and then a solution of trichloroacetic acid (1.7 g, 10.5 mmol, 3 eq.) in dichloromethane (3 mL) was added dropwise. The reaction mixture was warmed to -10°C and stirred for two hours. A solution of **103a** (1 g, 3.5 mmol, 1 eq.) in dichloromethane (3 mL) was added slowly. The reaction mixture was stirred overnight at room temperature, and then quenched with a citric acid aqueous solution (10%, 25 mL). The organic phase was washed with a saturated sodium chloride aqueous solution, and concentrated in vacuo. The residue was subjected to silica gel chromatography (eluents: petroleum ether/ethyl acetate = 0%-7%) to afford product **103b** (340 mg, yield: 65%).

Di(1-adamantyl)-butylphosphine (cataCXium A; 36 mg, 0.1 mmol, 0.1 eq.) was added to palladium acetate (22.5 mg, 0.1 mmol, 0.1 eq.). After adding degassed 2-methylbutan-2-ol (6 mL), the reactor was purged and filled with nitrogen. The catalyst and ligand were stirred under a nitrogen atmosphere for one hour. Then, degassed water (1 mL), caesium carbonate (1.63 g, 5 mmol, 5 eq.) and 5-bromo-2-fluoropyridine (176 mg, 1.1 mmol, 1.1 eq.) were added to the mixture. Finally, a solution of **103b** (340 mg, 1 mmol, 1 eq.) in 2-methylbutan-2-ol (1 mL) was added using a syringe, and the mixture was subjected to repeated evacuation and nitrogen purging. The reaction mixture was heated at 75°C for 16 hours, and then diluted with water (2 mL), and the diluted mixture was extracted with EA (3×20 mL). The combined organic phase was washed with a saturated sodium chloride aqueous solution (10 mL), dried, and concentrated. The residue was subjected to silica gel chromatography (eluents: petroleum ether/ethyl acetate = 2%-10%) to afford product **103c** (291 mg, yield: 92%). MS m/z (ESI): 296.5

At room temperature, ruthenium chloride (21 mg, 0.1 mmol, 0.1 eq.) was added to a solution of **103c** (291 mg, 0.98 mmol, 1 eq.) in acetonitrile (5 mL). Subsequently, a solution of sodium periodate (650 mg, 3 mmol, 3 eq) in water (1 mL) was added while stirring, and the reaction mixture was stirred overnight at room temperature. Hydrochloric acid (1M, 1 mL) was then added, followed by vacuum removal of the solution. The residual mixture was dissolved in acetonitrile (5 mL), filtered through Celite, and washed with acetonitrile (5 mL). The product was concentrated to afford **103d** (180 mg, yield: 92%). MS m/z (ESI): 196.2

**103d** (100 mg, 0.51 mmol), 2-(7-azabenzotriazol-1-yl)-*N*,*N*,*N*',*N*'-tetramethyluronium hexafluorophosphate (319 mg, 0.84 mmol, 1.8 eq.) and **20i** (104 mg, 0.47 mmol) were dissolved in DMF (5 mL) at room temperature, and the mixture was stirred for 5 min. Then, *N*,*N*-diisopropylethylamine (210.8 mg, 1.63 mmol) was added dropwise at 0°C. The resulting mixture was stirred at room temperature for half an hour and concentrated, and the residue was subjected to reversed-phase preparative chromatography to afford 103 (93 mg, yield: 49.7%).

MS m/z (ESI): 366.4

### Example 144

### 1-(5,6-Dimethyl-7,9-dihydro-8H-pyrrolo[3,4-c][1,2,4]triazolo[1,5-a]pyridin-8-yl)-2-(1-(5-(trifluoromethyl)pyrimidin-2-yl)azetidin-3-yl)ethan-1-one

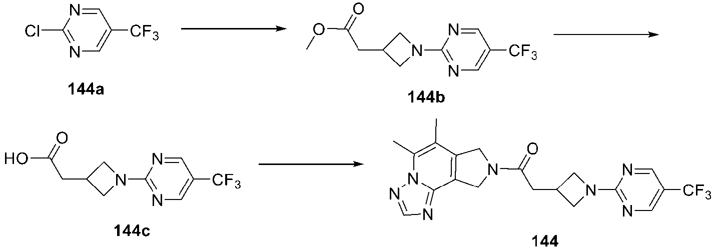

### Methyl 2-(1-(5-(trifluoromethyl)pyrimidin-2-yl)azetidin-3-yl)acetate

Compound 2-chloro-5-(trifluoromethyl)pyrimidine **144a** (2.0 g, 11.0 mmol) and methyl 2-(azetidin-3-yl)acetate (1.42 g, 11.0 mmol) were dissolved in acetonitrile (30 mL). Caesium carbonate (8.93 g, 27.4 mmol) was added. After the addition, the mixture was heated to 55°C and reacted for 1 hour. Water (50 mL) was added to the reaction solution, and the mixture was extracted with ethyl acetate (30 mL×2). The organic phases were combined, washed with a saturated sodium chloride solution (20 mL×2), dried over anhydrous sodium sulphate, and filtered. The filtrate was concentrated under reduced pressure, and the resulting residue was subjected to silica gel column chromatography using eluent system B, to afford title product **144b** (3.0 g, yield: 99.5%). MS m/z (ESI): 276.2 [M+1]

### 2-(1-(5-(Trifluoromethyl)pyrimidin-2-yl)azetidin-3-yl)acetic acid

**144b** (800 mg, 2.9 mmol) was dissolved in a mixed solvent of methanol (15 mL) and water (V/V = 4:1). Anhydrous lithium hydroxide (278 mg, 11.6 mmol) was added. After the addition, the mixture was stirred and reacted at 25°C for 1 hour. The methanol solvent was removed by rotary evaporation. 1 M hydrochloric acid (10 mL) was added, and the mixture was extracted with dichloromethane (20 mL x 3). The organic phases were combined, dried over anhydrous sodium sulphate, and concentrated under reduced pressure, to afford title product 2-(1-(5-(trifluoromethyl)pyrimidin-2-yl)azetidin-3-yl)acetic acid **144c** (759 mg), which was used directly in the next step. MS m/z (ESI): 262.2 [M+1]

### 1-(5,6-Dimethyl-7,9-dihydro-8H-pyrrolo[3,4-c][1,2,4]triazolo[1,5-a]pyridin-8-yl)-2-(1-(5-(trifluoromethyl)pyrimidin-2-yl)azetidin-3-yl)ethan-1-one

144c (759 mg, 2.9 mmol) was dissolved in *N*,*N*-dimethylformamide (15 mL). 5,6-Dimethyl-8,9-dihydro-7H-pyrrolo[3,4-c][1,2,4]triazolo[1,5-a]pyridine (750 mg, 2.9 mmol), 2-(7-azabenzotriazol-1-yl)-*N*,*N*,*N',N*'-tetramethyluronium hexafluorophosphate (1.6 g, 4.3 mmol) and diisopropylethylamine (1.1 g, 8.7 mmol) were added sequentially to the reaction system. The mixture was reacted at 25°C for 1 hour. Water (30 mL) was added to the reaction solution, and the mixture was extracted with dichloromethane (20 mL×2). The organic phases were combined, washed with a saturated sodium chloride solution (20 mL×2), dried over anhydrous sodium sulphate, and filtered. The filtrate was concentrated under reduced pressure, and the resulting residue was purified by preparative HPLC, to afford title product **144** (1.05 g, yield: 84.1%). MS m/z (ESI): 432.2 [M+1]

¹H NMR (400 MHz, DMSO) δ 8.66 (t, J = 1.4 Hz, 2H), 8.46 (d, J = 2.1 Hz, 1H), 5.12 (s, 1H), 4.98 (s, 1H), 4.87 (s, 1H), 4.77 (s, 1H), 4.37 - 4.23 (m, 2H), 3.90 - 3.80 (m, 2H), 3.11 (s, 2H), 3.01 - 2.88 (m, 1H), 2.73 (s, 3H), 2.29 (s, 3H).

### Example 284

### (5,6-Dimethyl-7,9-dihydro-8H-pyrrolo[3,4-c][1,2,4]triazolo[1,5-a]pyridin-8-yl)(3-((2-(trifluoromethyl)pyridin-4-yl)amino)cyclobutyl)methanone

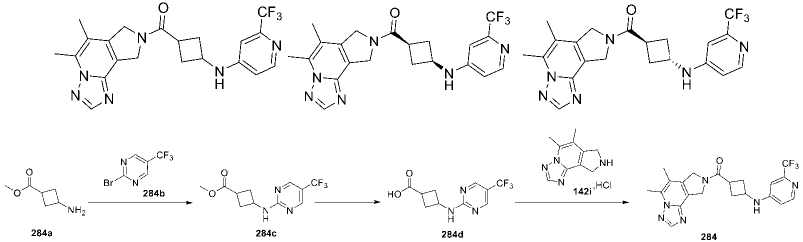

### Methyl 3-((5-(trifluoromethyl)pyrimidin-2-yl)amino)cyclobutane-1-carboxylate

Compound 284a (313 mg, 1.1 eq), compound 284b (500 mg, 1.0 eq) and *N*,*N-*diisopropylethylamine (854 mg, 3.0 eq) were added to *N*-methylpyrrolidone (10 mL), and the mixture was reacted at 120°C for 12 h. Upon completion of the reaction, the system was cooled to room temperature. Water (50 mL) was added, and the mixture was extracted twice with ethyl acetate (100 mL). The combined organic phase was washed with a saturated sodium chloride aqueous solution, dried, filtered, and concentrated. The residue was purified by silica gel chromatography (eluents: dichloromethane/methanol = 100%-5%) to afford product **284c** (420 mg, yield: 69.4%). MS m/z (ESI): 276.1.

### 3-((5-(Trifluoromethyl)pyrimidin-2-yl)amino)cyclobutane-1-carboxylic acid

Compound 284c (420 mg, 1.0 eq) and lithium hydroxide (183 mg, 5 eq) were dissolved in methanol (10 mL), and the mixture was reacted at 50°C for 4 h. Upon completion of the reaction, the system was cooled to 0°C, adjusted to pH 3-4 by slowly adding 2M dilute hydrochloric acid solution dropwise, and concentrated to dryness to afford a crude product, which was used in the next step without purification, assuming a theoretical yield. MS m/z (ESI): 262.2.

Referring to the synthesis method of Step 9 in Example 20, Example **284** (220 mg, yield: 34.6%) was obtained in Step 3.

MS m/z (ESI): 431.4.

The compound of Example **284** was resolved using a chiral column (chromatographic column: CHIRALCEL AS, 4.6*150 mm, 5 µm; detection wavelength (nm): 214; temperature (°C): 35; flow rate (mL/min): 10; mobile phase: Hexane : IPA : DEA=70 : 30 : 0.1%; run time (min): 12; elution mode: isocratic), to afford title product 284-P1 (7.8 mg, RT (retention time) = 6.667 min, yield: 7.2%); and title product 284-P2 (65.0 mg, RT (retention time) = 9.327 min, yield: 60.2%).

### Example 292

### 1-(4-Methoxy-5-methyl-7,8-dihydro-6H-[1,2,4]triazolo[5,1-f]pyrrolo[4,3-b]pyridin-7-yl)-2-{1-[2-(trifluoromethyl)pyridin-4-yl]azetidin-3-yl}ethan-1-one

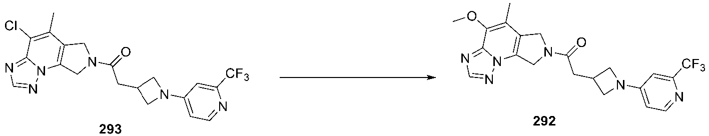

### 1-(4-Methoxy-5-methyl-7,8-dihydro-6H-[1,2,4]triazolo[5,1-f]pyrrolo[4,3-b]pyridin-7-yl)-2-{1-[2-(trifluoromethyl)pyridin-4-yl]azetidin-3-yl}ethan-1-one

Compound **293** (10 mg) was dissolved in anhydrous methanol (1 mL). A solution of sodium methoxide (10 mg) in anhydrous methanol (1 mL) was added, and then the mixture was stirred and reacted at 70°C under a nitrogen atmosphere for 12 hours. The reaction solution was cooled to room temperature, and concentrated under reduced pressure. The residue was separated and purified by high performance liquid chromatography (0.1% formic acid/acetonitrile/water) to afford title product **292** (1.3 mg, yield: 12.1%).

MS m/z (ESI): 447.2 [M+1]

### Example 293

### 1-(4-Chloro-5-methyl-7,8-dihydro-6H-[1,2,4]triazolo[5,1-f]pyrrolo[4,3-b]pyridin-7-yl)-2-{1-[2-(trifluoromethyl)pyridin-4-yl]azetidin-3-yl}ethan-1-one

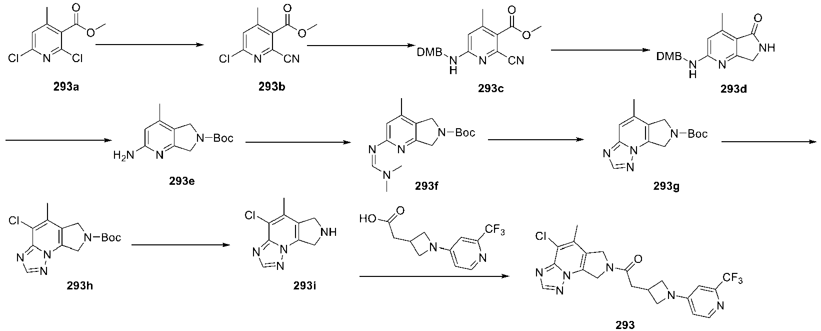

### Methyl 6-chloro-2-cyano-4-methylpyridine-3-carboxylate

Compound **293a** (2.5 g, 11.36 mmol) was transferred to a 20 mL microwave tube. N-Methylpyrrolidone (10 mL) was added, followed by cuprous cyanide (89 mg, 126.14 umol). The mixture was bubbled with nitrogen for 30 seconds, and then stirred and reacted under microwave irradiation at 180°C for 2 hours. Two parallel reactions were set up and combined for workup. The reaction solution was cooled to room temperature, and poured into 5% aqueous ammonia (100 mL). The mixture was extracted with ethyl acetate (100 mL), and filtered through Celite to remove black insoluble materials, and the phases were separated. The organic phase was washed with a saturated sodium chloride solution (20 mL×2), dried over anhydrous sodium sulphate, and filtered. The filtrate was concentrated under reduced pressure, and the resulting residue was purified by silica gel column chromatography using eluent system B, to afford title product **293b** (1.7 g, yield: 35.5%). MS m/z (ESI): 211.0 [M+1]

### Methyl 2-cyano-6-{[(2,4-dimethoxyphenyl)methyl]amino}-4-methylpyridine-3-carboxylate

Compound **293b** (1.7 g, 8.07 mmol) was dissolved in acetonitrile (34 mL). 2,4-Dimethoxybenzylamine (1.62 g, 9.69 mmol, 1.46 mL) and triethylamine (1.23 g, 12.11 mmol, 1.69 mL) were added sequentially, and then the mixture was stirred and reacted at 80°C for 16 hours. The reaction solution was cooled to room temperature, and directly concentrated under reduced pressure. The resulting residue was purified by silica gel column chromatography using eluent system B, to afford title product **293c** (1.2 g, yield: 43.6%). MS m/z (ESI): 310.2 [M+1]

### 2-{[(2,4-Dimethoxyphenyl)methyl]amino}-4-methyl-6,7-dihydro-5H-pyrrolo[4,3-b]pyridin-5-one

Compound **293c** (1.2 g, 3.52 mmol) was dissolved in methanol (24 mL). Nickel(II) chloride hexahydrate (1.67 g, 7.03 mmol) and sodium borohydride (1.33 g, 35.15 mmol) were added sequentially in portions at 0°C, and then the mixture was stirred and reacted at 20°C for 1 hour. Triethylamine (1.07 g, 10.55 mmol, 1.47 mL) was added, and the resulting mixture was heated to 50°C, and stirred and reacted for another 12 hours. The reaction solution was cooled to room temperature. A saturated ammonium chloride solution (100 mL) and ethyl acetate (40 mL) were added to the reaction solution. The mixture was filtered through Celite to remove insoluble materials, and the phases were separated. The organic phase was washed with a saturated sodium chloride solution (10 mL), dried over anhydrous sodium sulphate, and filtered. The filtrate was concentrated under reduced pressure, and the resulting residue was purified by silica gel column chromatography using eluent system B, to afford title product **293d** (600 mg, yield: 54.5%).

MS m/z (ESI): 282.2 [M+1]

### 2-Methylpropan-2-yl 2-amino-4-methyl-6,7-dihydro-5H-pyrrolo[4,3-b]pyridine-6-carboxylate

Compound **293d** (600 mg, 1.91 mmol) was dissolved in anhydrous tetrahydrofuran (6 mL). A borane in dimethyl sulphide solution (2 M, 6 mL) was added under a nitrogen atmosphere, and then the mixture was stirred and reacted at 70°C for 12 hours. The reaction solution was cooled to 0°C, and quenched by adding anhydrous methanol (3 mL) dropwise. Concentrated hydrochloric acid (12 M, 3 mL) was then added, and the resulting mixture was heated to 70°C and stirred for 12 hours. The reaction solution was cooled to 20°C again, and adjusted to pH>8 by adding a sodium hydroxide solution (2 M, 18 mL) slowly. Finally, di-tert-butyl dicarbonate (836 mg, 3.83 mmol) was added, and the resulting mixture was stirred and reacted at 20°C for 2 hours. The reaction solution was diluted with water (20 mL), and extracted with ethyl acetate (10 mL×2). The organic phases were combined, washed with a saturated sodium chloride solution (10 mL), dried over anhydrous sodium sulphate, and filtered. The filtrate was concentrated under reduced pressure, and the resulting residue was purified by silica gel column chromatography using eluent system B, to afford title product 293e (200 mg, yield: 41.9%).

MS m/z (ESI): 250.2 [M+1]

### 2-Methylpropan-2-yl 2-{[(Z)-(dimethylamino)methylidene]amino}-4-methyl-6,7-dihydro-5H-pyrrolo[4,3-b]pyridine-6-carboxylate

Compound **293e** (180 mg, 722 umol) was dissolved in anhydrous toluene (5 mL). N,N-Dimethylformamide dimethyl acetal (172 mg, 1.44 mmol) was added under a nitrogen atmosphere, and then the mixture was stirred and reacted at 110°C for 2 hours. The reaction solution was cooled to room temperature, and directly concentrated under reduced pressure to afford title product 2-methylpropan-2-yl 2-{[(Z)-(dimethylamino)methylidene]amino}-4-methyl-6,7-dihydro-5*H*-pyrrolo[4,3-b]pyridine-6-carboxylate **293f** (240 mg, crude). MS m/z (ESI): 305.2 [M+1]

### 2-Methylpropan-2-yl 5-methyl-7,8-dihydro-6H-[1,2,4]triazolo[5,1-f]pyrrolo[4,3-b]pyridine-7-carboxylate

Compound **293f** (230 mg, 756 umol) was dissolved in anhydrous methanol (10 mL). Anhydrous pyridine (179 mg, 2.27 mmol) and hydroxylamine-O-sulphonic acid (171 mg, 1.51 mmol) were added sequentially under a nitrogen atmosphere, and then the mixture was stirred and reacted at 60°C for 12 hours. The reaction solution was cooled to room temperature. A saturated ammonium chloride solution (20 mL) was added to the reaction solution, and the mixture was extracted with ethyl acetate (10 mL×2). The organic phases were combined, washed with a saturated sodium chloride solution (10 mL), dried over anhydrous sodium sulphate, and filtered. The filtrate was concentrated under reduced pressure, and the resulting residue was purified by silica gel column chromatography using eluent system B, to afford title product **293g** (100 mg, yield: 48.2%).

MS m/z (ESI): 275.1 [M+1]

### 2-Methylpropan-2-yl 4-chloro-5-methyl-7,8-dihydro-6H-[1,2,4]triazolo[5,1-f]pyrrolo[4,3-b]pyridine-7-carboxylate

Compound **293g** (40 mg, 146 umol) was dissolved in anhydrous N,N-dimethylformamide (4 mL). N-Chlorosuccinimide (39 mg, 292 umol) was added, and then the mixture was stirred and reacted at 60°C for 2 hours. The reaction solution was cooled to room temperature, and diluted with water (40 mL). The mixture was extracted with ethyl acetate (10 mL×2). The organic phases were combined, washed with a saturated sodium chloride solution (10 mL), dried over anhydrous sodium sulphate, and filtered. The filtrate was concentrated under reduced pressure, and the resulting residue was purified by silica gel column chromatography using eluent system B, to afford title product **293h** (40 mg, yield: 88.8%). MS m/z (ESI): 309.1 [M+1]

### 4-Chloro-5-methyl-7,8-dihydro-6H-[1,2,4]triazolo[5,1-f]pyrrolo[4,3-b]pyridine

Compound **293h** (40 mg, 130 umol) was dissolved in anhydrous dichloromethane (1 mL). A solution of hydrogen chloride in 1,4-dioxane (4 M, 1 mL) was added, and then the mixture was stirred and reacted at 25°C for 12 hours. The reaction solution was directly concentrated under reduced pressure to afford title product **293i** (40 mg, crude dihydrochloride). MS m/z (ESI): 209.1 [M+1]

### 1-(4-Chloro-5-methyl-7,8-dihydro-6H-[1,2,4]triazolo[5,1-f]pyrrolo[4,3-b]pyridin-7-yl)-2-{1-[2-(trifluoromethyl)pyridin-4-yl]azetidin-3-yl}ethan-1-one

Compound **293i** (100 mg) and compound {1-[2-(trifluoromethyl)pyridin-4-yl]azetidin-3-yl} acetic acid (37 mg) were dissolved in anhydrous N,N-dimethylformamide (2 mL). Triethylamine (100 uL) and O-(7-azabenzotriazol-1-yl)-N,N,N',N'-tetramethyluronium hexafluorophosphate (59 mg) were added sequentially at 0°C, and then the mixture was stirred and reacted at 20°C for 1 hour. The reaction solution was diluted with water (20 mL), and extracted with ethyl acetate (10 mL×2). The organic phases were combined, washed with a saturated sodium chloride solution (10 mL), dried over anhydrous sodium sulphate, and filtered. The filtrate was concentrated under reduced pressure, and the residue was separated and purified by high performance liquid chromatography (0.1% formic acid/acetonitrile/water) to afford title product **293** (22.1 mg, yield: 32.9%). MS m/z (ESI): 451.1 [M+1]
1H NMR (400 MHz, DMSO) δ 8.59 (s, 1H), 8.23 (d, 1H), 6.81 (s, 1H), 6.59 (d, 1H), 5.27 (s, 1H), 5.00 (d, 2H), 4.79 (s, 1H), 4.25 (t, 2H), 3.79 (d, 2H), 3.16 (s, 1H), 2.96 (d, 2H), 2.45 (d, 3H).

### Example 296

### (5,6-Dimethyl-8,9-dihydro-7H-[1,2,4]triazolo[1,5-a]pyrrolo[4,3-c]pyridin-8-yl){1-[2-(trifluoromethyl)pyridin-4-yl]spiro[2.3]hexan-5-yl}methanone

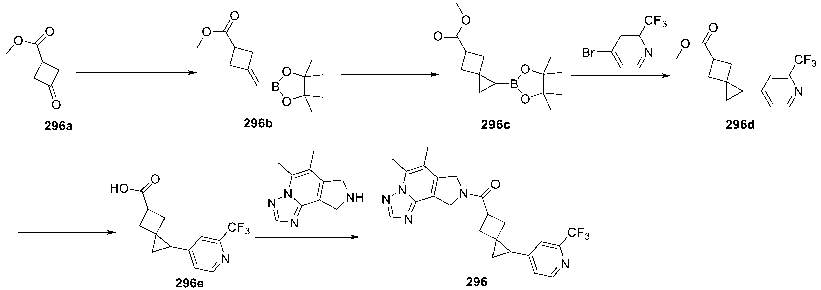

### Methyl 3-[(4,4,5,5-tetramethyl-1,3,2-dioxaborolan-2-yl)methylidene]cyclobutane-1-carboxylate

2,2,6,6-Tetramethylpiperidine (1.32 g, 9.37 mmol, 1.58 mL) was dissolved in anhydrous tetrahydrofuran (15 mL). n-Butyllithium (2.5 M, 3.75 mL) was added dropwise at -30°C under a nitrogen atmosphere, and the mixture was stirred for 0.5 hours. Subsequently, the reaction solution was cooled to -78°C. A solution of bis[(pinacolato)boryl]methane (2.09 g, 7.80 mmol) in anhydrous tetrahydrofuran (15 mL) was added dropwise, and the mixture was stirred at -78°C for 0.5 hours. Finally, a solution of methyl 3-oxocyclobutanecarboxylate **296a** (2.09 g, 7.80 mmol) in anhydrous tetrahydrofuran (15 mL) was added dropwise, and the resulting mixture was warmed naturally to 20°C, and stirred and reacted for 1 hour. The reaction solution was cooled to 0°C, and a saturated ammonium chloride solution (100 mL) was added dropwise to quench and dilute the reaction. The mixture was extracted with ethyl acetate (30 mL×2). The organic phases were combined, washed with a saturated sodium chloride solution (10 mL), dried over anhydrous sodium sulphate, and filtered. The filtrate was concentrated under reduced pressure, and the resulting residue was purified by silica gel column chromatography using eluent system B, to afford title product 296**b** (1.2 g, yield: 61.0 %), which was used directly in the next step.

### Methyl 1-(4,4,5,5-tetramethyl-1,3,2-dioxaborolan-2-yl)spiro[2.3]hexane-5-carboxylate

Under a nitrogen atmosphere at 0°C, diethylzinc (1 M, 1.19 mL) was added to anhydrous dichloromethane (1 mL), followed by a solution of diiodomethane (319 mg, 1.19 mmol) in anhydrous dichloromethane (0.5 mL), and the mixture was stirred at 0°C for 0.5 hours. Subsequently, a solution of compound **296b** (100 mg, 397 umol) in anhydrous dichloromethane (0.5 mL) was added dropwise, and the resulting mixture was warmed naturally to 20°C, and stirred and reacted for 12 hours. The reaction solution was cooled to 0°C, and a saturated ammonium chloride solution (20 mL) was added dropwise to quench and dilute the reaction. The mixture was extracted with ethyl acetate (10 mL×2). The organic phases were combined, washed with a saturated sodium chloride solution (10 mL), dried over anhydrous sodium sulphate, and filtered. The filtrate was concentrated under reduced pressure to afford title product 296**c** (115 mg, crude), which was used directly in the next step.

¹H NMR (400 MHz, CDCl3) δ 3.70 (d, 3H), 3.34 - 3.17 (m, 1H), 2.60 - 2.45 (m, 2H), 2.37 - 2.19 (m, 2H), 1.22 (dd, 12H), 0.84 - 0.71 (m, 2H), -0.02 - -0.12 (m, 1H).

### Methyl 1-[2-(trifluoromethyl)pyridin-4-yl]spiro[2.3]hexane-5-carboxylate

Compound **296c** (99 mg, 372 umol) and 4-bromo-2 trifluoromethylpyridine (70 mg, 310 umol) were dissolved in a mixed solvent of 1,4-dioxane and water (V/V = 5:1, 3.6 mL). Potassium carbonate (86 mg, 619 umol) and 1,1'-bis(diphenylphosphino)ferrocene palladium(II) dichloride (23 mg, 31 umol) were added sequentially. The mixture was bubbled with nitrogen for 30 seconds, and then stirred and reacted under microwave irradiation at 90°C for 2 hours. The reaction solution was cooled to room temperature, and diluted with water (20 mL). The mixture was extracted with ethyl acetate (10 mL×2). The organic phases were combined, washed with a saturated sodium chloride solution (10 mL), dried over anhydrous sodium sulphate, and filtered. The filtrate was concentrated under reduced pressure, and the resulting residue was purified by silica gel column chromatography using eluent system B, to afford title product 296**d** (40 mg, yield: 45.3%). MS m/z (ESI): 286.1 [M+1]

### 1-[2-(Trifluoromethyl)pyridin-4-yl]spiro[2.3]hexane-5-carboxylic acid

Compound **296d** (40 mg, 140 umol) was dissolved in anhydrous tetrahydrofuran (3 mL). Potassium trimethylsilanolate (54 mg, 421 umol) was quickly added under a nitrogen atmosphere, and then the mixture was stirred and reacted at 20°C for 1 hour. Dilute hydrochloric acid (2 M) was added dropwise to the reaction solution until weakly acidic. The mixture was concentrated under reduced pressure, and water was removed by azeotropic distillation with toluene, to afford title product **296e** (38 mg, crude), which was used directly in the next step.

MS m/z (ESI): 272.1 [M+1]

### (5,6-Dimethyl-8,9-dihydro-7H-[1,2,4]triazolo[1,5-a]pyrrolo[4,3-c]pyridin-8-yl){1-[2-(trifluoromethyl)pyridin-4-yl]spiro[2.3]hexan-5-yl}methanone

Compound **296e** (36 mg) and compound 5,6-dimethyl-8,9-dihydro-7H-[1,2,4]triazolo[1,5-a]pyrrolo[4,3-c]pyridine (30 mg) were dissolved in anhydrous N,N-dimethylformamide (2 mL). Triethylamine (48 uL) and O-(7-azabenzotriazol-1-yl)-N,N,N',N'-tetramethyluronium hexafluorophosphate (48 mg) were added sequentially at 0°C, and then the mixture was stirred and reacted at 20°C for 1 hour. The reaction solution was diluted with water (20 mL), and extracted with ethyl acetate (10 mL×2). The organic phases were combined, washed with a saturated sodium chloride solution (10 mL), dried over anhydrous sodium sulphate, and filtered. The filtrate was concentrated under reduced pressure, and the residue was separated and purified by high performance liquid chromatography (0.1% formic acid/acetonitrile/water) to afford title product **296** (28.4 mg, yield: 54.0%). MS m/z (ESI): 442.2 [M+1]
1H NMR (400 MHz, CDCl3) δ 8.57 (dd, 1H), 8.42 - 8.32 (m, 1H), 7.34 - 7.27 (m, 1H), 7.02 (td, 1H), 5.23 - 4.98 (m, 2H), 4.88 (d, 2H), 3.59 - 3.29 (m, 1H), 2.81 (s, 3H), 2.66 (dd, 1H), 2.59 - 2.42 (m, 2H), 2.35 (d, 3H), 2.20 - 1.98 (m, 2H), 1.40 (ddd, 1H), 1.15 (dt, 1H).

### Example 297

### 1-(5,6-Dimethyl-7,9-dihydro-8H-pyrrolo[3,4-c][1,2,4]triazolo[1,5-a]pyridin-8-yl)-2-(2-(2-(trifluoromethyl)pyridin-4-yl)cyclopropyl)ethan-1-one

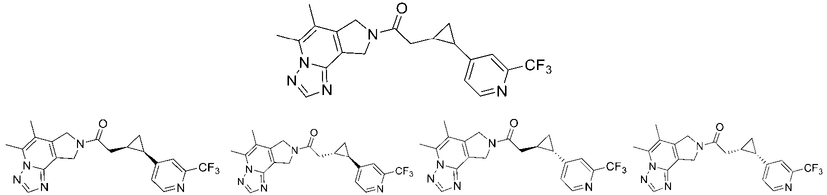

Referring to the synthesis method of Example 103, Example 297 was obtained. MS m/z (ESI): 416.4.

### Example 298

### 1-(5,6-Dimethyl-7,9-dihydro-8H-pyrrolo[3,4-c][1,2,4]triazolo[1,5-a]pyridin-8-yl)-2-(2-(5-(trifluoromethyl)pyrimidin-2-yl)cyclopropyl)ethan-1-one

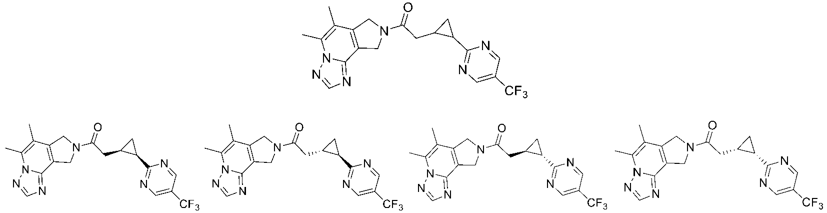

Referring to the synthesis method of Example 103, Example 298 was obtained. MS m/z (ESI): 417.4.

### Example 299

### (5,6-Dimethyl-7,9-dihydro-8H-pyrrolo[3,4-c][1,2,4]triazolo[1,5-a]pyridin-8-yl)(3-((5-(trifluoromethyl)pyrimidin-2-yl)amino)cyclobutyl)methanone

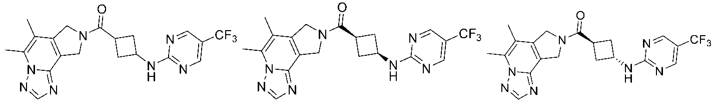

Referring to the synthesis method of Example 284, Example 299 was obtained. MS m/z (ESI): 432.4.

### Example 304

### 1-(5,6-Dimethyl-7,9-dihydro-8H-pyrrolo[3,4-c][1,2,4]triazolo[1,5-a]pyridin-8-yl)-2-(1-(6-ethoxypyridin-3-yl)azetidin-3-yl)ethan-1-one

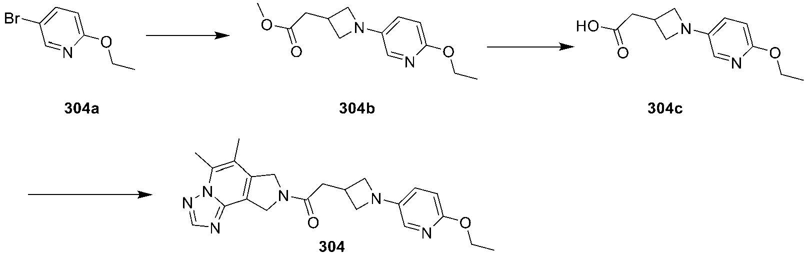

### Methyl 2-(1-(6-ethoxypyridin-3-yl)azetidin-3-yl)acetate

Under a nitrogen atmosphere, methyl 2-(azetidin-3-yl)acetate (3.84 g, 29.70 mmol), 5-bromo-2-ethoxypyridine (3 g, 14.85 mmol) **304a,** caesium carbonate (14.61 g, 44.54 mmol), and Brettsphos-G4 (714.19 mg, 742.40 µmol) were added to 1,4-dioxane (60 mL). The reaction solution was stirred at 120°C for 5 hours. Water (60 mL) was added, and the mixture was extracted with ethyl acetate (80 mL). The organic phases were combined, washed with a saturated sodium chloride solution (10 mL×2), dried over anhydrous sodium sulphate, and filtered. The filtrate was concentrated under reduced pressure, and the resulting residue was purified by silica gel column chromatography using eluent system B, to afford title product **304b** (1.6 g, yield: 43%). MS m/z (ESI): 251.2 [M+1]

### 2-(1-(6-Ethoxypyridin-3-yl)azetidin-3-yl)acetic acid

**304b** (1.6 g, 6.39 mmol) and lithium hydroxide monohydrate (1 M, 19.18 mL) were added to methanol (30 ml). The reaction solution was stirred at 25°C for 1 hour, and subjected to rotary evaporation to dryness to afford **304c** (1.5 g, yield: 99%). MS m/z (ESI): 237.2 [M+1]

### 1-(5,6-Dimethyl-7,9-dihydro-8H-pyrrolo[3,4-c][1,2,4]triazolo[1,5-a]pyridin-8-yl)-2-(1-(6-ethoxypyridin-3-yl)azetidin-3-yl)ethan-1-one

**20i** (1.55 g, 8.25 mmol), **304c** (1.5 g, 6.35 mmol), 2-(7-azabenzotriazol-1-yl)-*N*,*N*,*N*',*N*'-tetramethyluronium hexafluorophosphate (3.60 g, 9.52 mmol), and *N*,*N-*diisopropylethylamine (2.46 g, 19.05 mmol, 3.32 mL) were added to *N,N-*dimethylformamide (30 mL). The reaction solution was stirred at 25°C for 1 hour. Water (20 mL) was added, and the mixture was extracted with ethyl acetate (30 mL). The organic phase was concentrated under reduced pressure, and the resulting residue was purified by preparative HPLC, to afford title product **304** (1.25 g, yield: 48.6%). MS m/z (ESI): 407.2 [M+1]
¹H NMR (400 MHz, DMSO) δ 8.46 (s, 1H), 7.37 - 7.30 (m, 1H), 6.92 (dd, J = 8.9, 3.2 Hz, 1H), 6.63 (d, J = 8.7 Hz, 1H), 5.11 (s, 1H), 4.98 (s, 1H), 4.86 (s, 1H), 4.75 (s, 1H), 4.16 (q, J = 7.0 Hz, 2H), 3.97 (t, J = 7.5 Hz, 2H), 3.47 (d, J = 6.6 Hz, 2H), 3.06 (s, 1H), 2.87 (t, J = 6.9 Hz, 2H), 2.72 (s, 3H), 2.29 (d, J = 2.7 Hz, 3H), 1.27 (d, J = 7.0 Hz, 3H).

### Example 317

### 1-(4-Chloro-5-methyl-6,8-dihydro-7H-pyrrolo[3,4-e][1,24]triazolo[1,5-a]pyridin-7-yl)-2-(1-(5-(trifluoromethyl)pyrimidin-2-yl)azetidin-3-yl)ethan-1-one

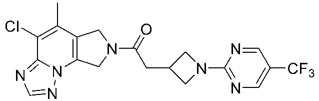

Referring to the preparation method of Step 1 to Step 8 in Example 293, 293i was obtained. Then, referring to the preparation method of Step 9 thereof, Example 317 was prepared by reacting with 144c. MS m/z (ESI): 452.8;
¹H NMR (400 MHz, DMSO) δ 8.67 (s, 2H), 8.58 (d, J = 2.0 Hz, 1H), 4.99 (d, J = 10.3 Hz, 2H), 4.29 (t, J = 9.3 Hz, 2H), 3.90 - 3.80 (m, 2H), 3.00 - 2.88 (m, 2H), 2.66 (s, 1H), 2.44 (d, J = 5.5 Hz, 3H), 2.06 - 1.93 (m, 2H).

### Example 326

### 1-(5-Methyl-1,2,3,6,7,8-hexahydrocyclopenta[1,2-d]pyrrolo[4,3-b]pyridin-2-yl)-2-{1-[2-(trifluoromethyl)pyridin-4-yl]azetidin-3-yl}ethan-1-one

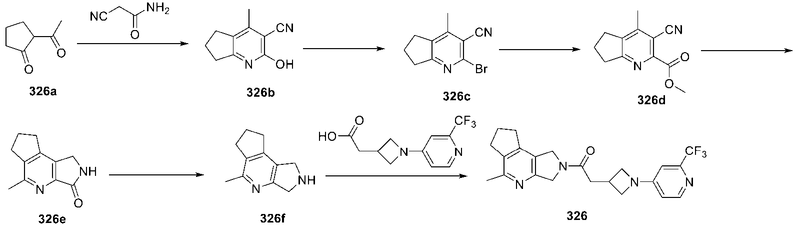

### 2-Hydroxy-4-methyl-6,7-dihydro-5H-cyclopenta[1,2-b]pyridine-3-carbonitrile

Compound **326a** (5 g, 39.63 mmol) was dissolved in ethanol (100 mL). Cyanoacetamide (3.33 g, 39.63 mmol) and piperidine (3.37 g, 39.63 mmol) were added sequentially, and then the mixture was stirred and reacted at 80°C under reflux for 12 hours. The reaction solution was cooled to room temperature, resulting in the precipitation of a large amount of solid, which was collected by filtration under reduced pressure. The filter cake was rinsed with ice-cold ethanol (10 mL×3), collected, and dried under reduced pressure to afford title product 2-hydroxy-4-methyl-6,7-dihydro-5*H*-cyclopenta[1,2-b]pyridine-3-carbonitrile **326b** (3.4 g, yield: 49.2%).

MS m/z (ESI): 175.1 [M+1]

### 2-Bromo-4-methyl-6,7-dihydro-5H-cyclopenta[1,2-b]pyridine-3-carbonitrile

Compound **326b** (1 g, 5.74 mmol) was dissolved in anhydrous 1,4-dioxane (10 mL). Phosphorus oxybromide (1.62 g, 9.69 mmol, 1.46 mL) was added under a nitrogen atmosphere, and then the mixture was warmed to 90°C, and stirred and reacted for 2 hours. The reaction solution was cooled to room temperature, diluted with a saturated sodium bicarbonate solution (100 mL), and extracted with ethyl acetate (20 mL×2). The organic phases were combined, washed with a saturated sodium chloride solution (10 mL), dried over anhydrous sodium sulphate, and filtered. The filtrate was concentrated under reduced pressure, and the resulting residue was purified by silica gel column chromatography using eluent system B, to afford title product 2-bromo-4-methyl-6,7-dihydro-5*H*-cyclopenta[1,2-b]pyridine-3-carbonitrile **326c** (700 mg, yield: 51.4%).

MS m/z (ESI): 237.0 [M+1]

### Methyl 3-cyano-4-methyl-6,7-dihydro-5H-cyclopenta[1,2-b]pyridine-2-carboxylate

Compound **326c** (700 mg, 2.95 mmol) was dissolved in a mixed solvent of methanol (7 mL) and N,N-dimethylformamide (7 mL). [1,1'-Bis(diphenylphosphino)ferrocene]palladium(II) dichloride dichloromethane complex (241 mg, 0.30 mmol) and triethylamine (596 mg, 5.90 mmol) were added sequentially. The mixture was purged three times with carbon monoxide gas, and then stirred and reacted at 100°C under a carbon monoxide balloon atmosphere (about 15 psi) for 12 hours. The reaction solution was cooled to room temperature. A saturated ammonium chloride solution (70 mL) and ethyl acetate (40 mL) were added to the reaction solution. The mixture was filtered through Celite to remove insoluble materials, and the phases were separated. The organic phase was washed with a saturated sodium chloride solution (10 mL), dried over anhydrous sodium sulphate, and filtered. The filtrate was concentrated under reduced pressure, and the resulting residue was purified by silica gel column chromatography using eluent system B, to afford title product methyl 3-cyano-4-methyl-6,7-dihydro-5H-cyclopenta[1,2-b]pyridine-2-carboxylate **326d** (400 mg, yield: 62.7%).

MS m/z (ESI): 217.1 [M+1]

### 5-Methyl-1,6,7,8-tetrahydrocyclopenta[d]pyrrolo[3,4-b]pyridin-3(2H)-one

Compound **326d** (400 mg, 2.95 mmol) was dissolved in anhydrous methanol (8 mL). Nickel(II) chloride hexahydrate (1.40 g, 5.90 mmol) and sodium borohydride (1.12 g, 29.50 mmol) were added sequentially in an ice bath, and then the mixture was stirred and reacted at 25°C for 1 hour. Triethylamine (894 mg, 8.85 mmol, 1.23 mL) was added, and the resulting mixture was warmed to 50°C, and stirred and reacted for another 12 hours. The reaction solution was cooled to room temperature. A saturated ammonium chloride solution (80 mL) and ethyl acetate (40 mL) were added to the reaction solution. The mixture was filtered through Celite to remove insoluble materials, and the phases were separated. The organic phase was washed with a saturated sodium chloride solution (10 mL), dried over anhydrous sodium sulphate, and filtered. The filtrate was concentrated under reduced pressure, and the resulting residue was purified by silica gel column chromatography using eluent system B, to afford title product **326e** (200 mg, yield: 57.5%).

MS m/z (ESI): 189.1 [M+1]

### 5-Methyl-1,2,3,6,7,8-hexahydrocyclopenta[d]pyrrolo[3,4-b]pyridine

Compound **326e** (200 mg, 1.91 mmol) was dissolved in anhydrous tetrahydrofuran (4 mL). A borane in dimethyl sulphide solution (2 M, 4 mL) was added under a nitrogen atmosphere, and then the mixture was stirred and reacted at 70°C for 12 hours. The reaction solution was cooled to 0°C, and quenched by adding anhydrous methanol (4 mL) dropwise. Concentrated hydrochloric acid (12 M, 2 mL) was then added, and the resulting mixture was heated to 70°C and stirred for 12 hours. The reaction solution was cooled to 20°C again, and adjusted to pH>8 by adding a sodium hydroxide solution (2 M, 12 mL) slowly. The reaction solution was diluted with water (40 mL), and extracted with ethyl acetate (20 mL×2). The organic phases were combined, washed with a saturated sodium chloride solution (10 mL), dried over anhydrous sodium sulphate, and filtered. The filtrate was concentrated under reduced pressure, and the resulting residue was purified by silica gel column chromatography using eluent system B, to afford title product **326f** (100 mg, yield: 54.1%).

MS m/z (ESI): 175.1 [M+1]

### 1-(5-Methyl-3,6,7,8-tetrahydrocyclopenta[d]pyrrolo[3,4-b]pyridin-2(1H)-yl)-2-(1-(2-(trifluoromethyl)pyridin-4-yl)azetidin-3-yl)ethan-1-one

Compound **326f** (100 mg) and compound {1-[2-(trifluoromethyl)pyridin-4-yl]azetidin-3-yl}acetic acid (100 mg) were dissolved in anhydrous *N,N-*dimethylformamide (2 mL). Triethylamine (100 uL) and O-(7-azabenzotriazol-1-yl)-N,N,N',N'-tetramethyluronium hexafluorophosphate (100 mg) were added sequentially at 0°C, and then the mixture was stirred and reacted at 25°C for 1 hour. The reaction solution was diluted with water (20 mL), and extracted with ethyl acetate (10 mL×2). The organic phases were combined, washed with a saturated sodium chloride solution (10 mL), dried over anhydrous sodium sulphate, and filtered. The filtrate was concentrated under reduced pressure, and the residue was separated and purified by high performance liquid chromatography (0.1% formic acid/acetonitrile/water) to afford title product **326** (20 mg).

¹H NMR (400 MHz, CDCl3) δ 8.27 (d, 1H), 6.58 (d, 1H), 6.34 (s, 1H), 4.86 (d, 2H), 4.74 (s, 2H), 4.28 (t, 2H), 3.83 - 3.73 (m, 2H), 3.31 (s, 1H), 2.99 - 2.86 (m, 4H), 2.82 (d, 2H), 2.56 (d, 3H), 2.23 (p, 2H).

MS m/z (ESI): 417.2 [M+1]

### Example 327

### 1-(5-Methyl-3,6,7,8-tetrahydrocyclopenta[d]pyrrolo[3,4-b]pyridin-2(1H)-yl)-2-(1-(5-(trifluoromethyl)pyrimidin-2-yl)azetidin-3-yl)ethan-1-one

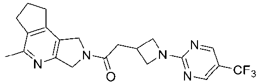

Referring to the synthesis method of Example 326, Example 327 was obtained. MS m/z (ESI): 418.4.

### Example 328

### 2-(1-(6-Ethoxypyridin-3-yl)azetidin-3-yl)-1-(5-methyl-3,6,7,8-tetrahydrocyclopenta[d]pyrrolo[3,4-b]pyridin-2(1H)-yl)ethan-1-one

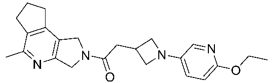

Referring to the synthesis method of Example 326, Example 328 was obtained. MS m/z (ESI): 393.5.

### Example 330

### 1-(5,6-Dimethyl-8,9-dihydro-7H-pyrrolo[4,3-f]quinoxalin-8-yl)-2-{1-[5-(trifluoromethyl)pyrimidin-2-yl]azetidin-3-yl}ethan-1-one

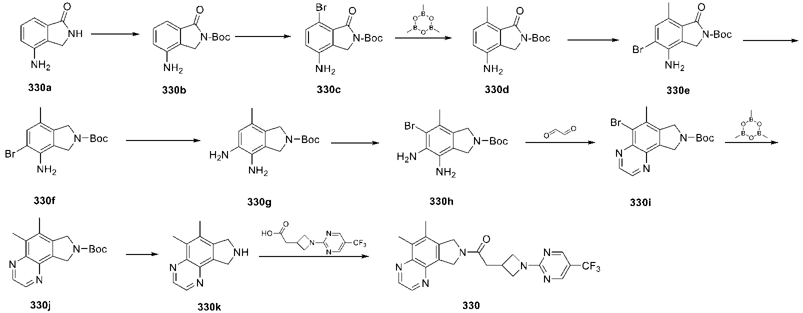

### 2-Methylpropan-2-yl 4-amino-1-oxo-2,3-dihydro-1H-isoindole-2-carboxylate

Sodium hydride (1.4 g, 34 mmol, 60% purity) was added portionwise to a solution of 4-amino-isoindolin-1-one (5 g, 34 mmol) in tetrahydrofuran (50 mL) at 0°C, and the mixture was stirred at 0°C for 30 minutes. Di-tert-butyl dicarbonate (7.4 g, 34 mmol) was added to the reaction solution, and the reaction solution was stirred at room temperature for another 1 hour. After the reaction was completed, the reaction solution was quenched by slowly adding a saturated ammonium chloride aqueous solution (30 mL) and water (20 mL) dropwise, and extracted with ethyl acetate (100 mL×2). The organic phases were combined, washed with a saturated sodium chloride solution (50 mL), dried over anhydrous sodium sulphate, and filtered. The filtrate was concentrated under reduced pressure, and the resulting residue was purified by silica gel column chromatography using eluent system C, to afford title product **330b** (4.6 g, yield: 54.9%). MS m/z (ESI): 249.1 [M+1]

### 2-Methylpropan-2-yl 4-amino-7-bromo-1-oxo-2,3-dihydro-1H-isoindole-2-carboxylate

*N*-Bromosuccinimide (3.3 g, 19 mmol) was added portionwise to a solution of **330b** (4.6 g, 19 mmol) in acetonitrile (60 mL) at 0°C, and the reaction solution was warmed naturally to room temperature and reacted for another 1 hour. After the reaction was completed, the reaction solution was quenched with water (100 mL), and extracted with ethyl acetate (100 mL×2). The organic phases were combined, washed with a saturated sodium chloride solution (50 mL), dried over anhydrous sodium sulphate, and filtered. The filtrate was concentrated under reduced pressure, and the resulting residue was purified by silica gel column chromatography using eluent system C, to afford title product 330**c** (5.2 g, yield: 85.8%). MS m/z (ESI): 327.1 [M+1]

### 2-Methylpropan-2-yl 4-amino-7-methyl-1-oxo-2,3-dihydro-1H-isoindole-2-carboxylate

[1,1'-Bis(di-tert-butylphosphino)ferrocene]palladium(II) dichloride (296 mg, 458 µmol) was added to a solution of **330c** (3 g, 9 mmol), trimethylboroxine (2.3 g, 18 mmol) and potassium carbonate (2.53 g, 18 mmol) in dioxane (30 mL) and water (3 mL). The mixture was purged three times with nitrogen, and reacted at 100°C for 2 hours. After the reaction was completed, the reaction system was quenched with water (100 mL), and extracted with ethyl acetate (100 mL×2). The organic phases were combined, dried over anhydrous sodium sulphate, and filtered. The filtrate was concentrated under reduced pressure, and the resulting residue was purified by silica gel column chromatography using eluent system C, to afford title product **330d** (2.1 g, yield: 87.3%).

MS m/z (ESI): 263.1 [M+1].

### 2-Methylpropan-2-yl 4-amino-5-bromo-7-methyl-1-oxo-2,3-dihydro-1H-isoindole-2-carboxylate

*N*-Bromosuccinimide (1.1 g, 6.1 mmol) was added portionwise to a solution of **330d** (1.6 g, 6.1 mmol) in acetonitrile (30 mL) at 0°C, and the reaction solution was warmed naturally to room temperature and reacted for another 1 hour. After the reaction was completed, the reaction solution was quenched with water (100 mL), and extracted with ethyl acetate (100 mL×2). The organic phases were combined, washed with a saturated sodium chloride solution (50 mL), dried over anhydrous sodium sulphate, and filtered. The filtrate was concentrated under reduced pressure, and the resulting residue was purified by silica gel column chromatography using eluent system C, to afford title product **330e** (1.6 g, yield: 76.9%). MS m/z (ESI): 341.0 [M+1].

### 2-Methylpropan-2-yl 4-amino-5-bromo-7-methyl-2,3-dihydro-1H-isoindole-2-carboxylate

A borane in tetrahydrofuran solution (1 M, 25 mL) was added to a solution of **330e** (1.7 g, 5 mmol) in tetrahydrofuran (2 mL). The reaction solution was heated to 70°C and reacted under a nitrogen atmosphere for 12 hours. After the reaction was completed, a methanol solution was added dropwise to the reaction solution until no more bubbles were evolved, and the mixture was heated to 70°C and stirred for 30 minutes. Subsequently, the reaction solution was filtered through Celite, and the filter cake was washed with dichloromethane (100 ml). The filtrate was subjected to rotary evaporation to dryness, and the resulting residue was purified by silica gel column chromatography using eluent system C, to afford title product 330**f** (1.1 g, yield: 67.5%). MS m/z (ESI): 326.1 [M+1].

### 2-Methylpropan-2-yl 4,5-diamino-7-methyl-2,3-dihydro-1H-isoindole-2-carboxylate

Aqueous ammonia (2.2 g, 13 mmol) was added to a mixed solution of 330**f** (1.1 g, 3 mmol), cuprous iodide (64 mg, 336 µmol), tripotassium phosphate (1.4 g, 7 mmol) and N1,N2-bis(5-methyl-[1,1'-biphenyl]-2-yl)oxalamide (283 mg, 672 µmol) in dimethyl sulphoxide (2 mL). The mixture was purged three times with nitrogen, and reacted under microwave irradiation at 100°C for 3 hours. After the reaction was completed, the reaction solution was filtered through Celite, and the filter cake was washed with dichloromethane (50 ml). The filtrate was subjected to rotary evaporation to dryness, and the resulting residue was purified by silica gel column chromatography using eluent system C, to afford title product 330**g** (750 mg, yield: 84.7%). MS m/z (ESI): 264.2 [M+1].

### 2-Methylpropan-2-yl 4,5-diamino-6-bromo-7-methyl-2,3-dihydro-1H-isoindole-2-carboxylate

*N*-Bromosuccinimide (152 mg, 854 µmol) was added portionwise to a solution of 330**g** (150 mg, 569 µmol) in acetonitrile (4 mL) at room temperature, and the reaction solution was reacted at room temperature for 10 minutes. After the reaction was completed, a saturated sodium bicarbonate solution (20 ml) was added to the reaction solution, and the mixture was extracted with ethyl acetate (20 ml×2). The organic phases were combined, dried over anhydrous sodium sulphate, and filtered. The filtrate was concentrated under reduced pressure to afford crude title product 330**h** (120 mg, yield: 61.6%). MS m/z (ESI): 342.1 [M+1].

### 2-Methylpropan-2-yl 5-bromo-6-methyl-8,9-dihydro-7H-pyrrolo[4,3-f]quinoxaline-8-carboxylate

40% aqueous glyoxal solution (229 mg, 1.6 mmol) was added to a solution of 330**h** (180 mg, 526 µmol) in ethanol (5 mL), and the reaction solution was stirred at 50°C for 3 hours. After the reaction was completed, the reaction solution was directly concentrated under reduced pressure, and the resulting residue was purified by silica gel column chromatography using eluent system C, to afford title product **330i** (45 mg, yield: 23.5%). MS m/z (ESI): 364.1 [M+1].

### 2-Methylpropan-2-yl 5,6-dimethyl-8,9-dihydro-7H-pyrrolo[4,3-f]quinoxaline-8-carboxylate

[1,1'-Bis(di-tert-butylphosphino)ferrocene]palladium(II) dichloride (2 mg, 3 µmol) was added to a solution of **330i** (20 mg, 55 µmol), trimethylboroxine (14 mg, 110 µmol) and potassium carbonate (15 mg, 110 µmol) in dioxane (0.8 mL) and water (0.1 mL). The mixture was purged three times with nitrogen, and reacted at 100°C for 2 hours. After the reaction was completed, the reaction system was quenched with water (10 mL), and extracted with ethyl acetate (10 mL×2). The organic phases were combined, dried over anhydrous sodium sulphate, and filtered. The filtrate was concentrated under reduced pressure, and the resulting residue was purified by silica gel column chromatography using eluent system C, to afford title product 330j (15 mg, yield: 91.3%).

MS m/z (ESI): 300.2 [M+1].

### 5,6-Dimethyl-8,9-dihydro-7H-pyrrolo[4,3-f]quinoxaline

A solution of hydrogen chloride in 1,4-dioxane (4 M, 1 mL) was added to a solution of **330j** (20 mg, 67 µmol) in dioxane (0.2 mL), and the reaction solution was stirred at room temperature for 1 hour. After the reaction was completed, the reaction solution was directly concentrated under reduced pressure to afford title product **330k** (15 mg, yield: 95%). MS m/z (ESI): 200.1 [M+1].

### 1-(5,6-Dimethyl-8,9-dihydro-7H-pyrrolo[4,3-f]quinoxalin-8-yl)-2-{1-[5-(trifluoromethyl)pyrimidin-2-yl]azetidin-3-yl}ethan-1-one

**330k** (15 mg, 64 µmol) was added to a solution of {1-[5-(trifluoromethyl)pyrimidin-2-yl]azetidin-3-yl}acetic acid (17 mg, 64 µmol), O-(7-azabenzotriazol-1-yl)-*N,N,N',N*'-tetramethyluronium hexafluorophosphate (29 mg, 76 µmol) and *N,N*-diisopropylethylamine (16 mg, 127 µmol) in *N,N-*dimethylformamide (0.6 mL). The reaction solution was stirred at room temperature for 1 hour. After the reaction was completed, the reaction system was quenched with water (2 mL), and extracted with ethyl acetate (3 mL×2). The organic phases were combined, dried over anhydrous sodium sulphate, and filtered. The filtrate was concentrated under reduced pressure, and the resulting residue was purified by preparative HPLC, to afford title product **330** (20 mg, yield: 70%). MS m/z (ESI): 443.2 [M+1].

### Example 331

### (4-Chloro-5-methyl-6,8-dihydro-7H-pyrrolo[3,4-e][1,2,4]triazolo[1,5-a]pyridin-7-yl)(1-(trifluoromethyl)-1H-pyrazol-4-yl)methanone

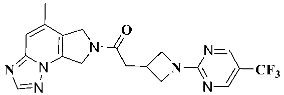

Referring to the preparation method of Step 1 to Step 6 in Example 293, 293g was obtained. Then, after removing the Boc protecting group according to the preparation method of Step 8 thereof, and following the preparation method of Step 9 thereof, Example 331 was prepared by reacting with 144c.

¹H NMR (400 MHz, DMSO) δ 8.66 (t, J = 1.0 Hz, 2H), 8.48 (d, J = 2.0 Hz, 1H), 7.66 (d, J = 4.5 Hz, 1H), 5.25 (s, 1H), 4.96 (d, J = 2.7 Hz, 2H), 4.74 (s, 1H), 4.29 (t, J = 8.7 Hz, 2H), 3.90 - 3.82 (m, 2H), 3.19 - 3.04 (m, 1H), 2.99 - 2.88 (m, 2H), 2.40 (d, J = 5.7 Hz, 3H). MS m/z (ESI): 418.2.

### Example 333

### 1-(5-Methyl-4-vinyl-6,8-dihydro-7H-pyrrolo[3,4-e][1,2,4]triazolo[1,5-a]pyridin-7-yl)-2-(1-(5-(trifluoromethyl)pyrimidin-2-yl)azetidin-3-yl)ethan-1-one

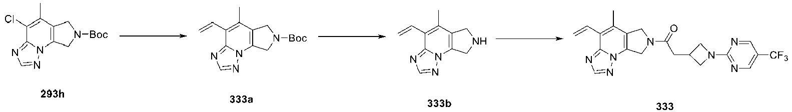

### tert-Butyl 5-methyl-4-vinyl-6,8-dihydro-7H-pyrrolo[3,4-e][1,2,4]triazolo[1,5-a]pyridine-7-carboxylate

tert-Butyl 4-chloro-5-methyl-6,8-dihydro-7*H*-pyrrolo[3,4-e][1,2,4]triazolo[1,5-a]pyridine-7-carboxylate **293h** (500 mg, 1.62 mmol) was dissolved in a mixed solvent of *1,4*-dioxane and water (V/V = 4:1, 5.0 mL). Potassium vinyltrifluoroborate (325 mg, 2.43 mmol), 1,1'-bis(diphenylphosphino)ferrocene palladium(II) dichloride (136 mg, 0.16 mmol) and caesium carbonate (1.58 g, 4.86 mmol) were added sequentially to the reaction system. The resulting mixture was heated to 110°C and reacted for 5 hours. Water (20 mL) was added to the reaction solution, and the mixture was extracted with ethyl acetate (10 mL×2). The organic phases were combined, washed with a saturated sodium chloride solution (10 mL×2), dried over anhydrous sodium sulphate, and filtered. The filtrate was concentrated under reduced pressure, and the resulting residue was purified by silica gel column chromatography using eluent system B, to afford title product **333a** (410 mg, yield: 84.4%). MS m/z (ESI): 301.2 [M+1]

### 5-Methyl-4-vinyl-7,8-dihydro-6H-pyrrolo[3,4-e][1,2,4]triazolo[1,5-a]pyridine

Compound 333**a** (100 mg, 0.33 mmol) was dissolved in hydrochloric acid/methanol (4.0 M, 2.0 mL), and the mixture was stirred and reacted at 25°C for 1 hour. The methanol solvent was removed by rotary evaporation, to afford title product 333**b** (67 mg), which was used directly in the next step. MS m/z (ESI): 200.1 [M+1]

### 1-(5-Methyl-4-vinyl-6,8-dihydro-7H-pyrrolo[3,4-e][1,2,4]triazolo[1,5-a]pyridin-7-yl)-2-(1-(5-(trifluoromethyl)pyrimidin-2-yl)azetidin-3-yl)ethan-1-one

Compound 5-methyl-4-vinyl-7,8-dihydro-6*H*-pyrrolo[3,4-e][1,2,4]triazolo[1,5-a]pyridine **333b** (67 mg, 0.33 mmol) was dissolved in *N,N-*dimethylformamide (2.0 mL). 2-(1-(5-(Trifluoromethyl)pyrimidin-2-yl)azetidin-3-yl)acetic acid (87 mg, 0.33 mmol), 2-(7-azabenzotriazol-1-yl)-*N*,*N*,*N',N*'-tetramethyluronium hexafluorophosphate (188 mg, 0.5 mmol) and diisopropylethylamine (129 mg, 1.0 mmol) were added sequentially to the reaction system. The mixture was reacted at 25°C for 1 hour. Water (10 mL) was added to the reaction solution, and the mixture was extracted with dichloromethane (10 mL×2). The organic phases were combined, washed with a saturated sodium chloride solution (10 mL×2), dried over anhydrous sodium sulphate, and filtered. The filtrate was concentrated under reduced pressure, and the resulting residue was purified by preparative HPLC, to afford title product 333 (95 mg, yield: 64.2%). MS m/z (ESI): 444.2 [M+1]

### Example 334

### 1-(4-Ethyl-5-methyl-6,8-dihydro-7H-pyrrolo[3,4-e][1,2,4]triazolo[1,5-a]pyridin-7-yl)-2-(1-(5-(trifluoromethyl)pyrimidin-2-yl)azetidin-3-yl)ethan-1-one

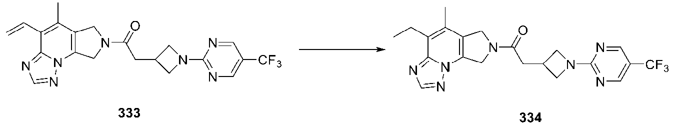

### 1-(4-Ethyl-5-methyl-6,8-dihydro-7H-pyrrolo[3,4-e][1,2,4]triazolo[1,5-a]pyridin-7-yl)-2-(1-(5-(trifluoromethyl)pyrimidin-2-yl)azetidin-3-yl)ethan-1-one

Compound **333** (50 mg, 0.11 mmol) was dissolved in methanol (2.0 mL). Palladium on carbon (20 mg, 10%) was added, and the mixture was purged three times with hydrogen, and stirred and reacted for 1 hour. The system was filtered. The filtrate was concentrated under reduced pressure, and the resulting residue was purified by preparative HPLC, to afford title product 334 (35 mg, yield: 69.7%). MS m/z (ESI): 446.2 [M+1]

### Example 346

### 1-(4-Chloro-5-methyl-6,8-dihydro-7H-pyrrolo[3,4-e][1,2,4]triazolo[1,5-a]pyridin-7-yl)-2-(1-(5-fluoropyrimidin-2-yl)azetidin-3-yl)ethan-1-one

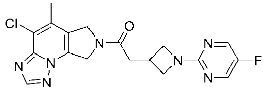

Referring to the preparation method of Step 1 to Step 8 in Example 293, 293i was obtained. Then, referring to the preparation method of Step 9 thereof, Example 346 was prepared by reacting with

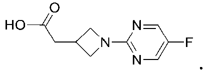

¹H NMR (400 MHz, DMSO-d6) δ 8.59-8.58 (m, 1H), 8.44-8.42 (m, 2H), 5.26 (s, 1H), 5.01-4.97 (m, 2H), 4.78 (s, 1H), 4.21-4.16 (m, 2H), 3.77-3.74 (m, 2H), 3.06 (brs, 1H), 2.94-2.89 (m, 2H), 2.44 (d, *J*=8.0 Hz, 3H). MS m/z (ESI): 402.2 [M+1]

### Example 348

### (4-Ethyl-5-methyl-6,8-dihydro-7H-pyrrolo[3,4-e][1,2,4]triazolo[1,5-a]pyridin-7-yl)(1-(2-(trifluoromethyl)pyridin-4-yl)pyrrolidin-3-yl)methanone

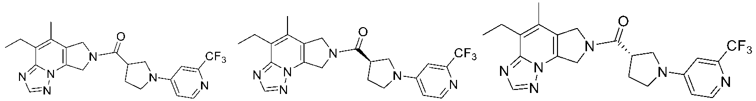

Referring to the synthesis method of Example **334,** Example 348 was obtained. MS m/z (ESI): 445.2.

### Example 349

### 1-(4-Ethyl-5-methyl-6,8-dihydro-7H-pyrrolo[3,4-e][1,2,4]triazolo[1,5-a]pyridin-7-yl)-2-(1-(5-fluoro-2-methoxypyridin-4-yl)azetidin-3-yl)ethan-1-one

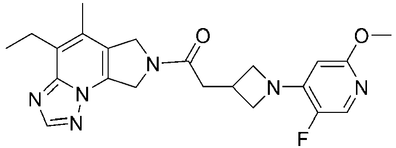

Referring to the synthesis method of Example **334,** Example 349 was obtained.

¹H NMR (400 MHz, DMSO-d6) δ 8.46-8.45 (m, 1H), 7.74-7.73 (m, 1H), 5.78-5.75 (m, 1H), 5.22 (s, 1H), 4.97-4.95 (m, 2H), 4.74 (s, 1H), 4.23-4.19 (m, 2H), 3.79-3.75 (m, 2H), 3.73 (s, 3H), 3.11-3.08 (m, 1H), 3.05-2.99 (m, 2H), 2.93-2.89 (m, 2H), 2.34 (d, *J*=4.0 Hz, 3H), 1.20-1.16 (t, *J*=8.0 Hz, 3H).

MS m/z (ESI): 425.2 [M+1]

### Example 351

### 1-(5-Ethyl-6-methyl-7,9-dihydro-8H-pyrrolo[3,4-f]quinoxalin-8-yl)-2-(1-(2-(trifluoromethyl)pyridin-4-yl)azetidin-3-yl)ethan-1-one

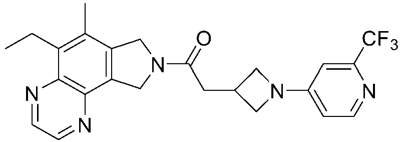

Referring to the synthesis method of Example **330,** Example 351 was obtained. MS m/z (ESI): 456.2.

¹H NMR (400 MHz, DMSO) δ 1H NMR (400 MHz, DMSO) δ 9.53 (s, 1H), 8.51 (dd, J = 10.9, 1.7 Hz, 1H), 8.18 (dd, J = 8.5, 4.5 Hz, 1H), 7.79 (ddd, J = 8.4, 3.8, 1.7 Hz, 1H), 4.91 - 4.75 (m, 4H), 2.85 (ddt, J = 20.5, 14.2, 7.6 Hz, 4H), 2.22 (s, 1H), 2.11 - 2.00 (m, 4H).

MS m/z (ESI): 336.1.

| The following examples were synthesised by referring to the method of Reference Example 1. | | | |
|---|---|---|---|
| 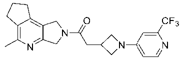 Example 1 | 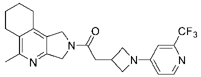 Example 2 | 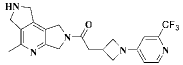 Example 3 | 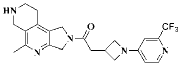 Example 4 |
| Example 1 was synthesised using 5-methyl-1,2,3,6,7,8-hexahydrocyclopenta[3, 4-b]pyridine in place of 2-chloro-4-methyl-6,7-dihydro-5H-pyrrolo[3,4-d]pyrimidine. | Example 2 was synthesised using 5-methyl-1,3,6,7,8,9-hexahydro-2H-pyrrolo[3,4-c]isoquinoline in place of 2-chloro-4-methyl-6,7-dihydro-5H-pyrrolo[3,4-d]pyrimidine. | Example 3 was synthesised using 5-methyl-3,6,7,8-tetrahydrodipyrrolo[3,4-b:3', 4'-d]pyridine in place of 2-chloro-4-methyl-6,7-dihydro-5H-pyrrolo[3,4-d]pyrimidine. | Example 4 was synthesised using 5-methyl-1,3,6,7,8,9-hexahydro-2H-pyrrolo[3,4-c][2,7]naphthyridine in place of 2-chloro-4-methyl-6,7-dihydro-5H-pyrrolo[3,4-d]pyrimidine. |
| MS m/z (ESI): 417.4 [M+1] | MS m/z (ESI): 431.5 [M+1] | MS m/z (ESI): 418.4 [M+1] | MS m/z (ESI): 432.4 [M+1] |
| 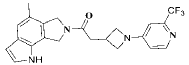 Example 5 | 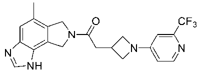 Example 6 | 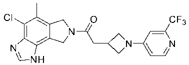 Example 7 | 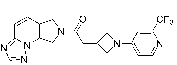 Example 8 |
| Example 5 was synthesised using 5-methyl-6,8-dihydropyrrolo[3,4-g]indole in place of 2-chloro-4-methyl-6,7-dihydro-5H-pyrrolo[3,4-d]pyrimidine. | Example 6 was synthesised using 5-methyl-6,8-dihydroimidazo[4,5-e]isoindole in place of 2-chloro-4-methyl-6,7-dihydro-5H-pyrrolo[3,4-d]pyrimidine. | Example 7 was synthesised using 4-chloro-5-methyl-6,8-dihydroimidazo[4,5-e]isoindole in place of 2-chloro-4-methyl-6,7-dihydro-5H-pyrrolo[3,4-d]pyrimidine. | Example 8 was synthesised using 5-methyl-6,8-dihydro-7H-pyrrolo[3,4-e][1,2,4]triazolo[1,5-a]pyridine in place of 2-chloro-4-methyl-6,7-dihydro-5H-pyrrolo[3,4-d]pyrimidine. |
| MS m/z (ESI): 415.4 [M+1] | MS m/z (ESI): 416.4 [M+1] | MS m/z (ESI): 450.9 [M+1] | MS m/z (ESI): 417.4 [M+1] |
| 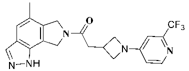 Example 9 | 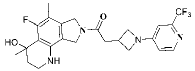 Example 10 | 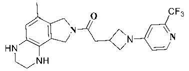 Example 11 | 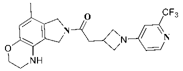 Example 12 |
| Example 9 was synthesised using 5-methyl-6,8-dihydropyrrolo[3,4-g]indazole in place of 2-chloro-4-methyl-6,7-dihydro-5H-pyrrolo[3,4-d]pyrimidine. | | | |
| MS m/z (ESI): 416.4 [M+1] | MS m/z (ESI): 479.5 [M+1] | MS m/z (ESI): 432.5 [M+1] | MS m/z (ESI): 433.5 [M+1] |
| 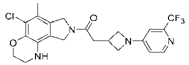 Example 13 | 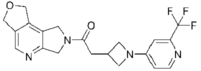 Example 14 | 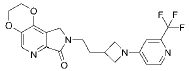 Example 15 | 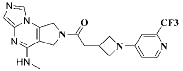 Example 16 |
| MS m/z (ESI): 467.9 [M+1] | MS m/z (ESI): 411.5 [M+1] | MS m/z (ESI): 421.4 [M+1] | MS m/z (ESI): 432.4 [M+1] |
| | | MS m/z (ESI): 419.5 [M+1] | |
| 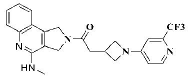 Example 17 | 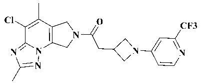 Example 18 | 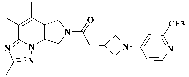 Example 19 | 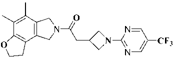 Example 337 |
| MS m/z (ESI): 442.5 [M+1] | MS m/z (ESI):465.9 [M+1] | MS m/z (ESI): 445.5 [M+1] | MS m/z (ESI): 433.2 |
| 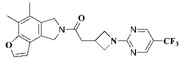 Example 338 | 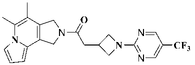 Example 339 | 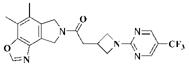 Example 340 | 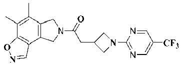 Example 341 |
| MS m/z (ESI): 431.2 | MS m/z (ESI): 430.2 | MS m/z (ESI): 432.2 | MS m/z (ESI): 432.2 |
| 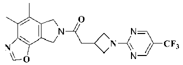 Example 342 | 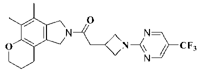 Example 343 | 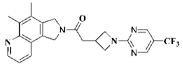 Example 344 | 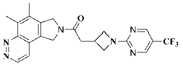 Example 345 |
| MS m/z (ESI): 432.2 | MS m/z (ESI): 447.2 | MS m/z (ESI): 442.2 | MS m/z (ESI): 443.2 |

| The following examples were synthesised by referring to the method of Example 20. | | | |
|---|---|---|---|
| 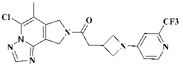 Example 21 | 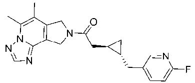 Example 22 | 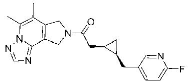 Example 23 | 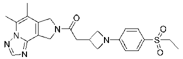 Example 24 |
| MS m/z (ESI): 451.9 | MS m/z (ESI): 380.4 | MS m/z (ESI): 380.4 | MS m/z (ESI): 454.6 |
| 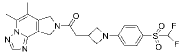 Example 25 | 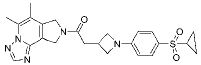 Example 26 | 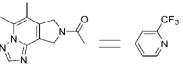 Example 27 | 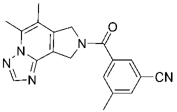 Example 28 |
| MS m/z (ESI): 476.5 | MS m/z (ESI): 466.6 | MS m/z (ESI): 400.4 | MS m/z (ESI): 332.4 |
| 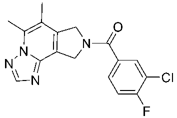 Example 29 | 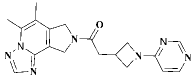 Example 30 | 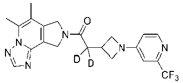 Example 31 | 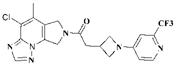 Example 33 |
| MS m/z (ESI): 345.8 | MS m/z (ESI): 364.4 | MS m/z (ESI): 433.4 | MS m/z (ESI): 451.9 |
| 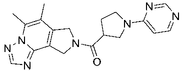 Example 34 | 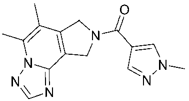 Example 35 | 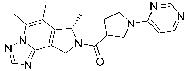 Example 36 | 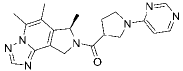 Example 37 |
| MS m/z (ESI): 364.4 | MS m/z (ESI): 297.3 | MS m/z (ESI): 378.4 | MS m/z (ESI): 378.4 |
| 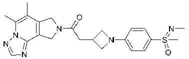 Example 38 | 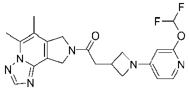 Example 52 | 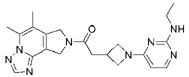 Example 53 | 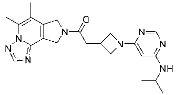 Example 54 |
| MS m/z (ESI): 453.6 | MS m/z (ESI): 429.4 | MS m/z (ESI): 407.5 | MS m/z (ESI): 421.5 |
| 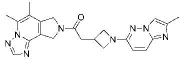 Example 55 | 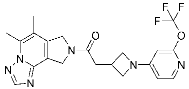 Example 56 | 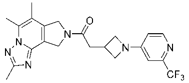 Example 57 | 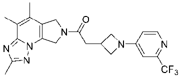 Example 58 |
| MS m/z (ESI): 417.5 | MS m/z (ESI): 447.4 | MS m/z (ESI): 445.5 | MS m/z (ESI): 445.5 |
| 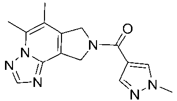 Example 59 | 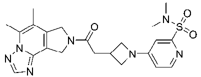 Example 61 | 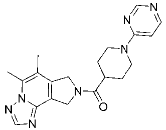 Example 64 | 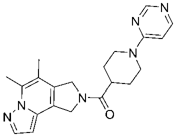 Example 65 |
| MS m/z (ESI): 297.3 | MS m/z (ESI): 470.6 | MS m/z (ESI): 378.5 | MS m/z (ESI): 377.5 |
| 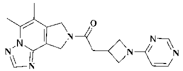 Example 66 | 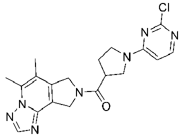 Example 67 | 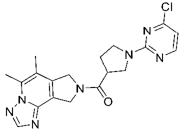 Example 68 | 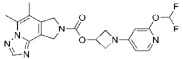 Example 73 |
| MS m/z (ESI):432.4 | MS m/z (ESI): 398.9 | MS m/z (ESI): 398.9 | MS m/z (ESI): 431.4 |
| 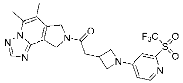 Example 90 | 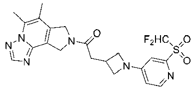 Example 91 | 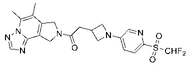 Example 92 | 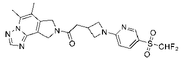 Example 93 |
| MS m/z (ESI): 495.5 | MS m/z (ESI): 477.5 | MS m/z (ESI): 477.5 | MS m/z (ESI): 477.5 |
| 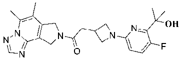 Example 94 | 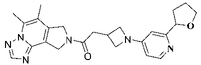 Example 95 | 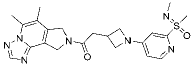 Example 96 | 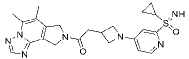 Example 97 |
| MS m/z (ESI): 439.5 | MS m/z (ESI): 433.5 | MS m/z (ESI): 454.6 | MS m/z (ESI): 466.6 |
| 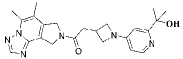 Example 98 | 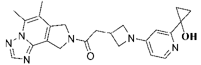 Example 99 | 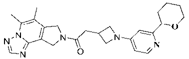 Example 100 | 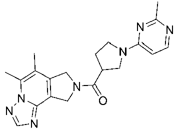 Example 102 |
| MS m/z (ESI): 421.5 | MS m/z (ESI): 419.5 | MS m/z (ESI): 447.6 | MS m/z (ESI): 412.5 |
| 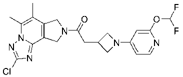 Example 128 | 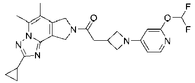 Example 130 | 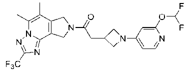 Example 131 | 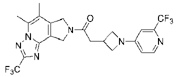 Example 132 |
| MS m/z (ESI): 463.9 | MS m/z (ESI): 469.5 | MS m/z (ESI): 497.4 | MS m/z (ESI): 499.4 |
| 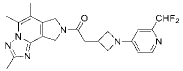 Example 133 | 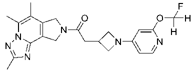 Example 134 | 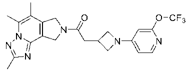 Example 135 | 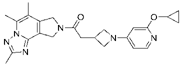 Example 136 |
| MS m/z (ESI): 427.5 | MS m/z (ESI): 425.5 | MS m/z (ESI): 461.5 | MS m/z (ESI): 433.5 |
| 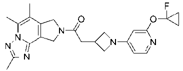 Example 137 | 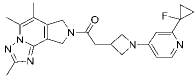 Example 138 | 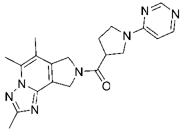 Example 140 | 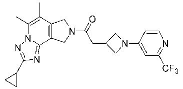 Example 143 |
| MS m/z (ESI): 451.5 | MS m/z (ESI): 435.5 | MS m/z (ESI): 378.4 | MS m/z (ESI): 471.5 |
| 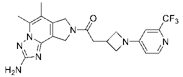 Example 145 | 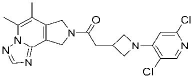 Example 146 | 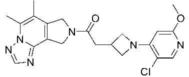 Example 147 | 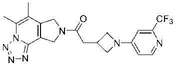 Example 148 |
| MS m/z (ESI): 446.4 | MS m/z (ESI): 432.3 | MS m/z (ESI): 427.9 | MS m/z (ESI): 432.4 |
| 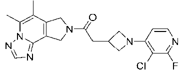 Example 149 | 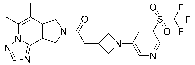 Example 151 | 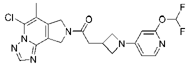 Example 152 | 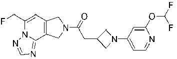 Example 153 |
| MS m/z (ESI): 415.9 | MS m/z (ESI): 495.5 | MS m/z (ESI): 449.9 | MS m/z (ESI): 447.4 |
| 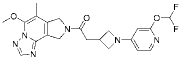 Example 154 | 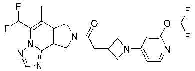 Example 155 | 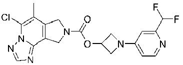 Example 156 | 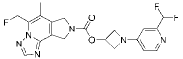 Example 157 |
| MS m/z (ESI): 445.4 | MS m/z (ESI): 465.4 | MS m/z (ESI): 435.8 | MS m/z (ESI): 433.4 |
| 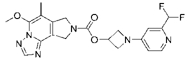 Example 158 | 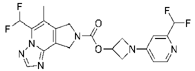 Example 159 | 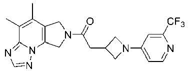 Example 163 | 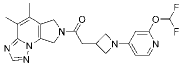 Example 164 |
| MS m/z (ESI): 431.4 | MS m/z (ESI): 451.4 | MS m/z (ESI): 431.4 | MS m/z (ESI): 429.4 |
| 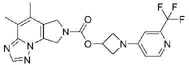 Example 165 | 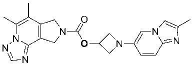 Example 178 | 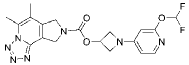 Example 179 | 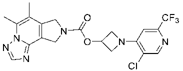 Example 180 |
| MS m/z (ESI): 433.4 | MS m/z (ESI): 418.5 | MS m/z (ESI): 432.4 | MS m/z (ESI): 467.8 |
| 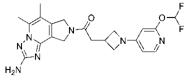 Example 181 | 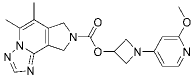 Example 182 | 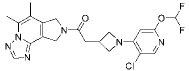 Example 183 | 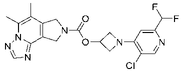 Example 184 |
| MS m/z (ESI): 444.5 | MS m/z (ESI): 395.4 | MS m/z (ESI): 463.9 | MS m/z (ESI): 449.9 |
| 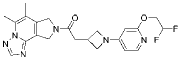 Example 185 | 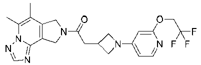 Example 186 | 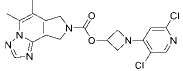 Example 187 | 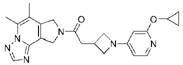 Example 188 |
| MS m/z (ESI): 443.5 | MS m/z (ESI): 461.5 | MS m/z (ESI): 434.3 | MS m/z (ESI): 419.5 |
| 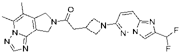 Example 189 | 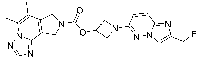 Example 190 | 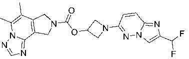 Example 191 | 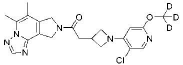 Example 192 |
| MS m/z (ESI): 453.5 | MS m/z (ESI): 437.5 | MS m/z (ESI): 455.4 | MS m/z (ESI): 430.9 |
| 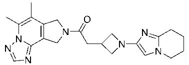 Example 193 | 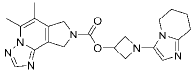 Example 194 | 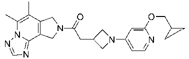 Example 195 | 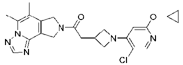 Example 196 |
| MS m/z (ESI): 406.5 | MS m/z (ESI): 408.5 | MS m/z (ESI): 433.5 | MS m/z (ESI): 453.9 |
| 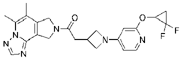 Example 197 | 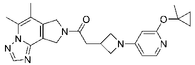 Example 198 | 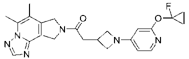 Example 199 | 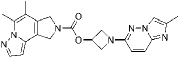 Example 200 |
| MS m/z (ESI): 455.5 | MS m/z (ESI): 433.5 | MS m/z (ESI): 437.5 | MS m/z (ESI): 418.5 |
| 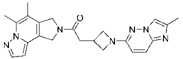 Example 201 | 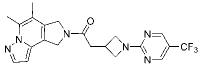 Example 202 | 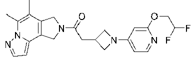 Example 203 | 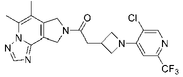 Example 204 |
| MS m/z (ESI): 416.5 | MS m/z (ESI): 431.4 | MS m/z (ESI): 442.5 | MS m/z (ESI): 465.9 |
| 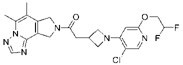 Example 205 | 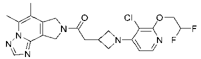 Example 206 | 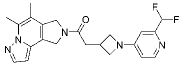 Example 208 | 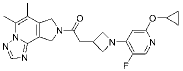 Example 209 |
| MS m/z (ESI): 477.9 | MS m/z (ESI): 477.9 | MS m/z (ESI): 412.5 | MS m/z (ESI): 437.5 |
| 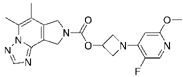 Example 210 | 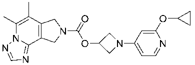 Example 211 | 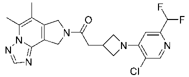 Example 212 | 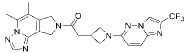 Example 213 |
| MS m/z (ESI): 413.4 | MS m/z (ESI): 421.5 | MS m/z (ESI): 447.9 | MS m/z (ESI): 471.5 |
| 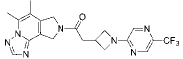 Example 214 | 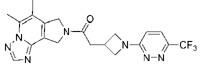 Example 215 | 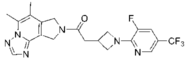 Example 216 | 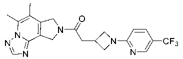 Example 217 |
| MS m/z (ESI): 432.4 | MS m/z (ESI): 432.4 | MS m/z (ESI): 449.4 | MS m/z (ESI): 431.4 |
| 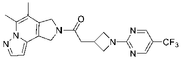 Example 218 | 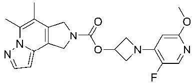 Example 219 | 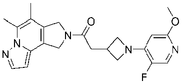 Example 220 | 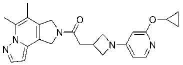 Example 221 |
| MS m/z (ESI): 431.4 | MS m/z (ESI): 412.4 | MS m/z (ESI): 410.5 | MS m/z (ESI): 418.5 |
| 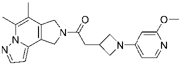 Example 222 | 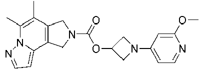 Example 223 | 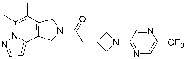 Example 224 | 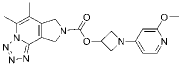 Example 225 |
| MS m/z (ESI): 392.5 | MS m/z (ESI): 394.5 | MS m/z (ESI): 431.4 | MS m/z (ESI): 396.4 |
| 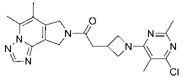 Example 226 | 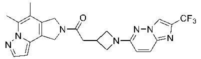 Example 227 | 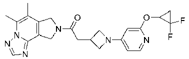 Example 228 | 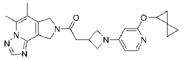 Example 229 |
| MS m/z (ESI): 426.9 | MS m/z (ESI): 470.5 | MS m/z (ESI): 455.5 | MS m/z (ESI): 445.5 |
| 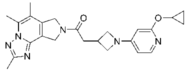 Example 230 | 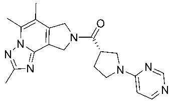 Example 231 | 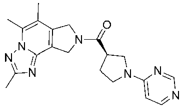 Example 232 | 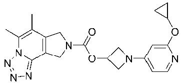 Example 233 |
| MS m/z (ESI): 433.5 | MS m/z (ESI): 378.5 | MS m/z (ESI): 378.5 | MS m/z (ESI): 422.5 |
| 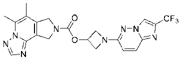 Example 234 | 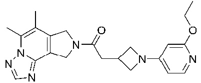 Example 235 | 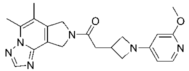 Example 236 | 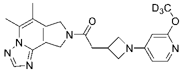 Example 237 |
| MS m/z (ESI): 473.4 | MS m/z (ESI): 407.5 | MS m/z (ESI): 393.5 | MS m/z (ESI): 396.5 |
| 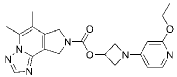 Example 238 | 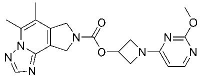 Example 239 | 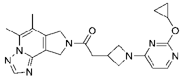 Example 240 | 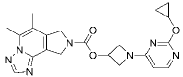 Example 241 |
| MS m/z (ESI): 409.5 | MS m/z (ESI): 396.4 | MS m/z (ESI): 420.5 | MS m/z (ESI): 422.5 |
| 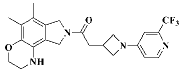 Example 242 | 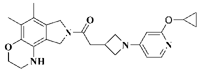 Example 243 | 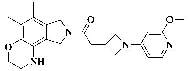 Example 244 | 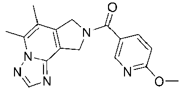 Example 245 |
| MS m/z (ESI): 447.5 | MS m/z (ESI): 435.5 | MS m/z (ESI): 409.5 | MS m/z (ESI): 324.4 |
| 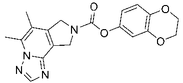 Example 246 | 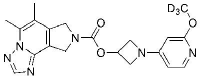 Example 247 | 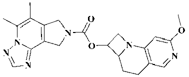 Example 248 | 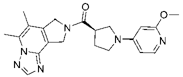 Example 249 |
| MS m/z (ESI): 367.4 | MS m/z (ESI): 398.5 | MS m/z (ESI): 421.5 | MS m/z (ESI): 393.5 |
| 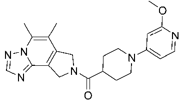 Example 250 | 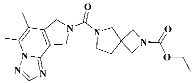 Example 251 | 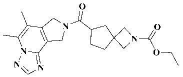 Example 252 | 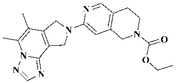 Example 253 |
| MS m/z (ESI): 407.5 | MS m/z (ESI): 399.5 | MS m/z (ESI): 398.5 | MS m/z (ESI): 393.5 |
| 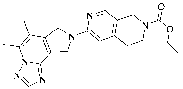 Example 254 | 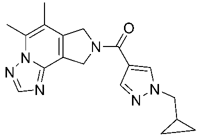 Example 255 | 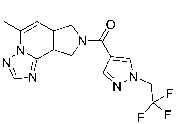 Example 256 | 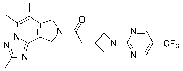 Example 257 |
| MS m/z (ESI): 393.5 | MS m/z (ESI): 337.4 | MS m/z (ESI): 365.3 | MS m/z (ESI): 446.5 |
| 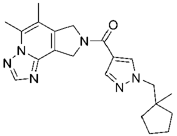 Example 258 | 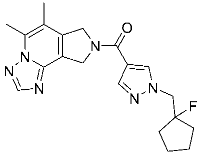 Example 259 | 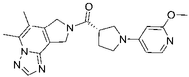 Example 260 | 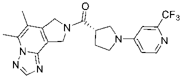 Example 261 |
| MS m/z (ESI): 379.5 | MS m/z (ESI): 383.4 | MS m/z (ESI): 393.5 | MS m/z (ESI): 431.4 |
| 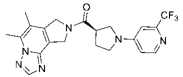 Example 262 | 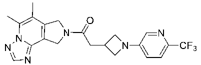 Example 263 | 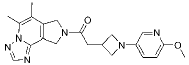 Example 264 | 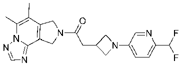 Example 265 |
| MS m/z (ESI): 431.4 | MS m/z (ESI): 431.4 | MS m/z (ESI): 393.5 | MS m/z (ESI): 413.4 |
| 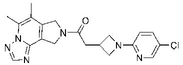 Example 266 | 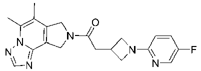 Example 267 | 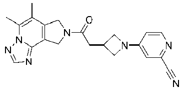 Example 268 | 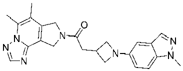 Example 269 |
| MS m/z (ESI): 397.8 | MS m/z (ESI): 381.4 | MS m/z (ESI): 388.4 | MS m/z (ESI): 416.5 |
| 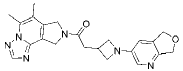 Example 270 | 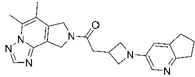 Example 271 | 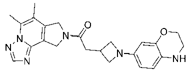 Example 272 | 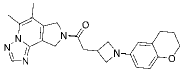 Example 273 |
| MS m/z (ESI): 405.5 | MS m/z (ESI): 403.5 | MS m/z (ESI): 419.5 | MS m/z (ESI): 418.5 |
| 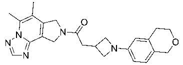 Example 274 | 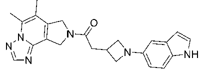 Example 275 | 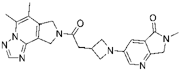 Example 276 | 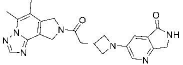 Example 277 |
| MS m/z (ESI): 418.5 | MS m/z (ESI): 401.5 | MS m/z (ESI): 432.5 | MS m/z (ESI): 418.5 |
| 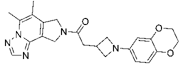 Example 278 | 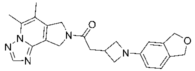 Example 279 | 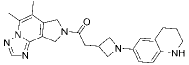 Example 280 | 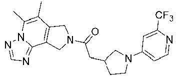 Example 281 |
| MS m/z (ESI): 420.5 | MS m/z (ESI): 404.5 | MS m/z (ESI): 417.5 | MS m/z (ESI): 445.5 |
| 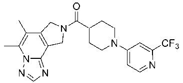 Example 282 | 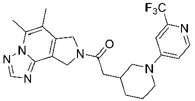 Example 283 | 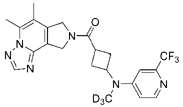 Example 285 | 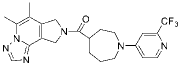 Example 286 |
| MS m/z (ESI): 445.5 | MS m/z (ESI): 459.5 | MS m/z (ESI): 448.5 | MS m/z (ESI): 459.5 |
| 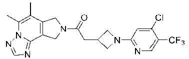 Example 287 | 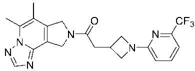 Example 288 | 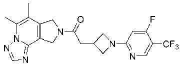 Example 289 | 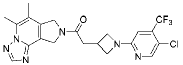 Example 290 |
| MS m/z (ESI): 465.8 | MS m/z (ESI): 431.4 | MS m/z (ESI): 449.4 | MS m/z (ESI): 465.8 |
| 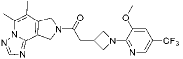 Example 291 | 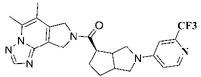 Example 294 | 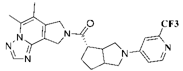 Example 295 | 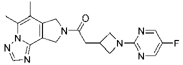 Example 300 |
| MS m/z (ESI): 461.5 | MS m/z (ESI): 471.5 | MS m/z (ESI): 471.5 | MS m/z (ESI): 382.4 |
| 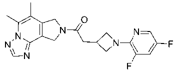 Example 301 | 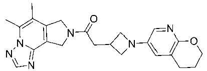 Example 302 | 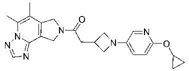 Example 303 | 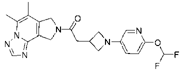 Example 305 |
| MS m/z (ESI): 399.4 | MS m/z (ESI): 419.5 | MS m/z (ESI): 419.5 | MS m/z (ESI): 429.4 |
| 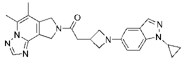 Example 306 | 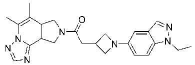 Example 307 | 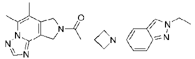 Example 308 | 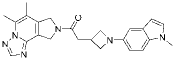 Example 309 |
| MS m/z (ESI): 442.5 | MS m/z (ESI): 430.5 | MS m/z (ESI): 430.5 | MS m/z (ESI): 415.5 |
| 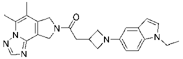 Example 310 | 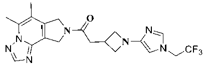 Example 311 | 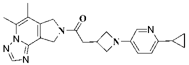 Example 312 | 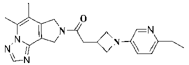 Example 313 |
| MS m/z (ESI): 429.5 | MS m/z (ESI):434.4 | MS m/z (ESI): 403.5 | MS m/z (ESI): 391.5 |
| 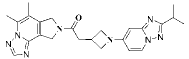 Example 314 | 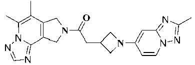 Example 315 | 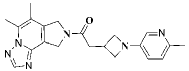 Example 318 | 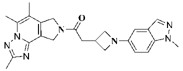 Example 319 |
| MS m/z (ESI): 445.5 | MS m/z (ESI): 417.5 | MS m/z (ESI): 377.5 | MS m/z (ESI): 430.5 |
| 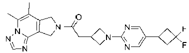 Example 320 | | | |
| MS m/z (ESI): 454.5 | | | |

| Examples 40 and 41 were synthesised by referring to the method of Reference Example 2; Examples 44 and 49 were synthesised by referring to the method of Example 39. | | | |
|---|---|---|---|
| 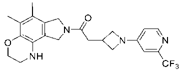 Example 40 | 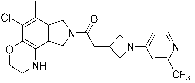 Example 41 | 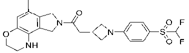 Example 44 | 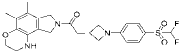 Example 49 |
| MS m/z (ESI): 447.5 | MS m/z (ESI): 467.9 | MS m/z (ESI): 478.5 | MS m/z (ESI): 492.6 |

| The following examples were synthesised by referring to the method of Example 42. | | | |
|---|---|---|---|
| 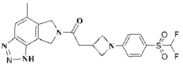 Example 45 | 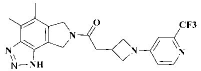 Example 47 | 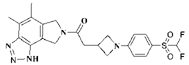 Example 50 | |
| MS m/z (ESI): 462.5 | MS m/z (ESI): 431.4 | MS m/z (ESI): 476.5 | |

| Examples 46, 48, and 51 were synthesised by referring to the method of Example 43; Example 336 was synthesised by referring to the method of Example 330; | | | |
|---|---|---|---|
| 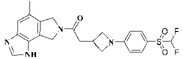 Example 46 | 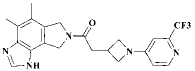 Example 48 | 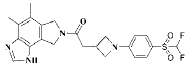 Example 51 | 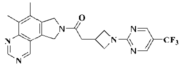 Example 336 |
| MS m/z (ESI): 461.5 | MS m/z (ESI): 430.4 | MS m/z (ESI): 475.5 | MS m/z (ESI): 443.2 |

| The following examples were synthesised by referring to the method of Example 60. | | | |
|---|---|---|---|
| 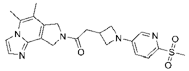 Example 62 | 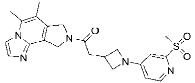 Example 63 | 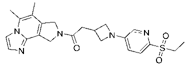 Example 69 | 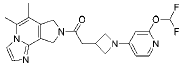 Example 70 |
| MS m/z (ESI): 440.5 | MS m/z (ESI): 440.5 | MS m/z (ESI): 454.6 | MS m/z (ESI): 428.5 |
| 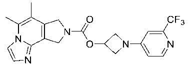 Example 71 | 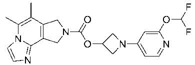 Example 72 | 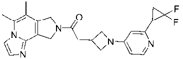 Example 74 | 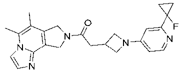 Example 75 |
| MS m/z (ESI): 432.4 | MS m/z (ESI): 430.4 | MS m/z (ESI): 438.5 | MS m/z (ESI): 420.5 |
| 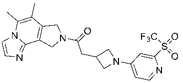 Example 79 | 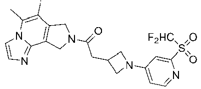 Example 80 | 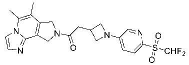 Example 81 | 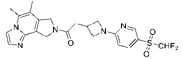 Example 82 |
| MS m/z (ESI): 494.5 | MS m/z (ESI): 476.5 | MS m/z (ESI): 476.5 | MS m/z (ESI): 476.5 |
| 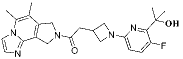 Example 83 | 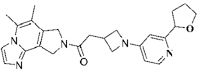 Example 84 | 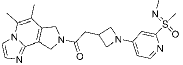 Example 85 | 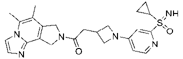 Example 86 |
| MS m/z (ESI): 438.5 | MS m/z (ESI): 432.5 | MS m/z (ESI): 453.6 | MS m/z (ESI): 465.6 |
| 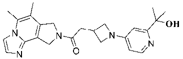 Example 87 | 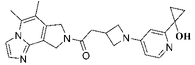 Example 88 | 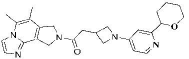 Example 89 | 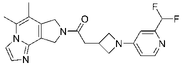 Example 101 |
| MS m/z (ESI): 420.5 | MS m/z (ESI): 418.5 | MS m/z (ESI): 446.6 | MS m/z (ESI): 412.5 |
| 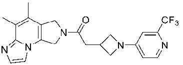 Example 160 | 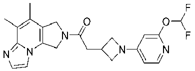 Example 161 | 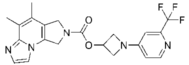 Example 162 | |
| MS m/z (ESI): 430.4 | MS m/z (ESI): 428.5 | MS m/z (ESI): 432.4 | |

| The following examples were synthesised by referring to the method of Example 103. | | | |
|---|---|---|---|
| 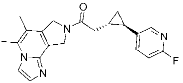 Example 104 | 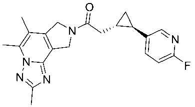 Example 139 | 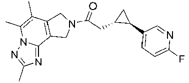 Example 169 | 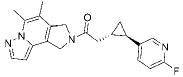 Example 207 |
| MS m/z (ESI): 365.4 | MS m/z (ESI): 380.4 | MS m/z (ESI): 380.4 | MS m/z (ESI): 365.4 |

| The following examples were synthesised by referring to the method of Example 32. | | | |
|---|---|---|---|
| 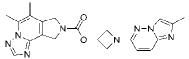 Example 105 | 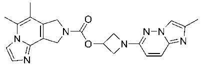 Example 106 | 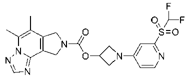 Example 107 | 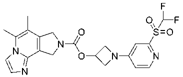 Example 108 |
| MS m/z (ESI): 419.4 | MS m/z (ESI): 418.4 | MS m/z (ESI): 479.4 | MS m/z (ESI): 478.4 |
| 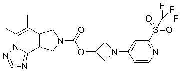 Example 109 | 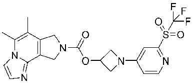 Example 110 | 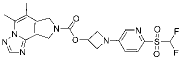 Example 111 | 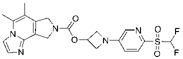 Example 112 |
| MS m/z (ESI): 497.4 | MS m/z (ESI): 496.4 | MS m/z (ESI): 479.4 | MS m/z (ESI): 478.4 |
| 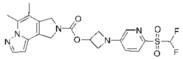 Example 113 | 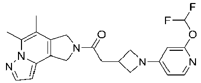 Example 114 | 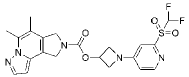 Example 115 | 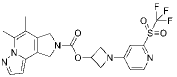 Example 116 |
| MS m/z (ESI): 478.4 | MS m/z (ESI): 428.4 | MS m/z (ESI): 478.4 | MS m/z (ESI): 496.4 |
| 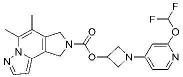 Example 117 | 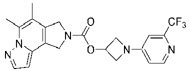 Example 118 | 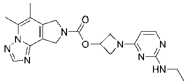 Example 119 | 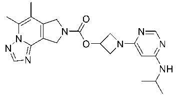 Example 120 |
| MS m/z (ESI): 430.4 | MS m/z (ESI): 432.4 | MS m/z (ESI): 409.4 | MS m/z (ESI): 423.4 |
| 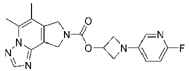 Example 121 | 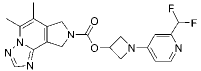 Example 122 | 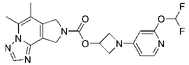 Example 123 | 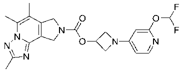 Example 125 |
| MS m/z (ESI): 383.4 | MS m/z (ESI): 415.4 | MS m/z (ESI): 431.4 | MS m/z (ESI): 445.4 |
| 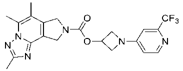 Example 126 | 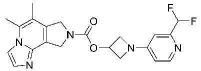 Example 141 | 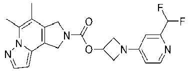 Example 142 | 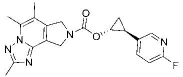 Example 170 |
| MS m/z (ESI): 447.4 | MS m/z (ESI): 414.4 | MS m/z (ESI): 414.4 | MS m/z (ESI): 382.4 |
| 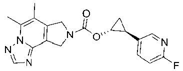 Example 171 | | | |
| MS m/z (ESI): 368.4 | | | |

| Examples 124, 127, and 129 were synthesised by referring to the method of Example 57; Example 316 was synthesised by referring to the method of Example 292; | | | |
|---|---|---|---|
| 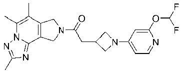 Example 124 | 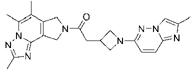 Example 127 | 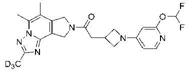 Example 129 | 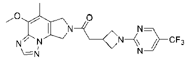 Example 316 |
| MS m/z (ESI): 443.5 | MS m/z (ESI): 431.5 | MS m/z (ESI): 446.5 | MS m/z (ESI): 448.4 |

| Examples 150, 166, 167, 168, and 172-177 were synthesised by referring to the method of Example 76; Examples 335 and 350 were synthesised by referring to the method of Example 334. | | | |
|---|---|---|---|
| 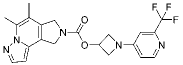 Example 150 | 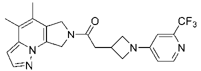 Example 166 | 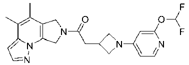 Example 167 | 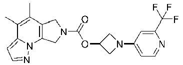 Example 168 |
| MS m/z (ESI): 432.4 | MS m/z (ESI): 430.5 | MS m/z (ESI): 428.5 | MS m/z (ESI): 432.4 |
| 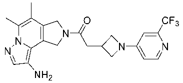 Example 172 | 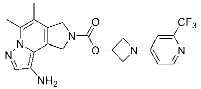 Example 173 | 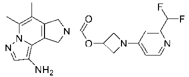 Example 174 | 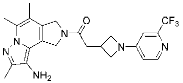 Example 175 |
| MS m/z (ESI): 445.5 | MS m/z (ESI): 447.4 | MS m/z (ESI): 429.4 | MS m/z (ESI): 459.5 |
| 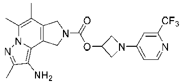 Example 176 | 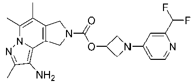 Example 177 | 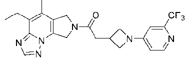 Example 335 | 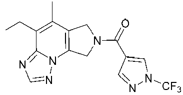 Example 350 |
| MS m/z (ESI): 461.5 | MS m/z (ESI): 443.5 | MS m/z (ESI): 445.2 | MS m/z (ESI): 365.2 |

| Examples 321, 322, 323, 329, 332, and 347 were synthesised by referring to the method of Example 293; Examples 324 and 325 were synthesised by referring to the method of Example 284. | | | |
|---|---|---|---|
| 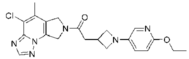 Example 321 | 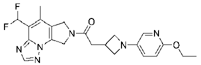 Example 322 | 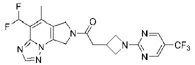 Example 323 | 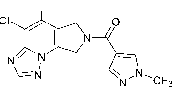 Example 329 |
| MS m/z (ESI): 427.9 | MS m/z (ESI): 443.5 | MS m/z (ESI): 468.4 | MS m/z (ESI): 371.1 |
| 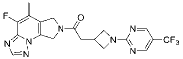 Example 332 | 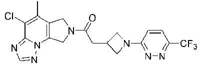 Example 347 | 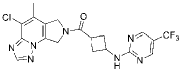 Example 324 | 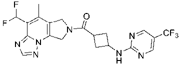 Example 325 |
| MS m/z (ESI): 436.2 | MS m/z (ESI): 452.2 | MS m/z (ESI): 452.8 | MS m/z (ESI): 468.4 |

### Biological assay and evaluation

The present invention will be further described and explained below in conjunction with test examples, but these examples are not meant to limit the scope of the present invention.

### I. Cell function experiment

### Test example 1. Determination of the effect of the compounds of the present invention on calcium current in cells stably expressing M1-M5 receptors

### 1. Experimental objective:

To detect the potentiating effect of compounds on calcium flux activity in CHO-K1/human M1/3/5 and CHO-K1/Gα15 M2/4 cells.

### 2. Experimental instruments and reagents:

### 2.1 Instruments:

384-well assay plate, 384-well plate, FLIPR Tip, FLIPR Penta plate reader, Dispendix non-contact nanoliter dispensing system (liquid handling workstation), liquid handler.

### 2.2 Reagents:

Ham's F-12 Nutrient Mix, foetal bovine serum, P/S (Gibco: 15140122), HBSS (Gibco: 14025076), HEPES (Gibco: 15630080), FLIPR calcium 6 assay kit (Molecular Devices: R8190), Probenecid (PBA) (Invitrogen: P36400), Acetylcholine chloride (Sigma: A6625-25G)
Complete culture medium: DMEM + 10% FBS + 1X P/S; Cell seeding medium: DMEM + 10% FBS + 1X PS; Assay buffer 1: 1X HBSS + 20 mM HEPES +2.5 mM Probenecid; Assay buffer 2: 1X HBSS + 20 mM HEPES; Cell line: CHO-K1/M1; CHO-K1/M2/Gα15; CHO-K1/M3; CHO-K1/M4/Gα15; CHO-K1/M5.

FLIPR calcium 6 assay kit: Component A was dissolved in Assay buffer 1, and the mixture was shaken for 1-2 min, sub-packaged and stored at -20°C.

### 3. Experimental methods:

1) CHO-K1/M1; CHO-K1/M2/Gα15; CHO-K1/M3; CHO-K1/M4/Gα15; and CHO-K1/M5 cell lines were cultured separately in complete culture medium at 37°C, 5% CO₂ to 70%-90% confluency.
2) The cells were digested and resuspended in cell seeding medium, 5,000 cells/well/20 µL were seeded into a 384-well cell culture plate, and the cells were cultured at 37°C, 5% CO₂ for 24 hours.
3) The cell culture plate was taken out of the CO2 incubator and equilibrated at room temperature for 10 minutes.
4) Component A solution was removed and allowed to equilibrate to room temperature. 20 µL of Component A solution was added to each well of the cell culture plate, and incubated at room temperature in the dark for 1 hour.
5) Acetylcholine chloride (ACh), a muscarinic receptor agonist, was prepared in Assay buffer 2 at EC₂₀/EC₁₀₀ concentrations; subsequently, 30 µL of the prepared muscarinic receptor agonist was added to a 384-well agonist plate (PE: 6008590) using a Multidrop Combi; the working solutions of the positive control compound and the test compound were serially diluted at a 3.16-fold gradient and dispensed (450 nL) into a 384-well compound plate (PE: 6008590) using the Dispendix liquid handling workstation; 30 µL of Assay buffer 2 was added to the 384-well compound plate (PE: 6008590) using the Multidrop Combi; the positive control compound and the test compound were diluted to 6X, mixed by shaking, and placed at room temperature until use.
6) 10 µL of the diluted 6X compound was added to the assay wells of the corresponding 384-well cell plate using the FLIPR Penta, followed by a 2-minute incubation.
7) 10 µL of the prepared muscarinic receptor agonist was added to the assay wells of the corresponding 384-well cell plate using the FLIPR Penta, with simultaneous data acquisition.

### 4. Experimental data processing method:

FLIPR Penta was used to read and collect the fluorescence signal value (RFU). The maximum RFU value was used to calculate the percent activation data based on the readings of the Low control (DMSO control group) and High control (ACh EC₁₀₀) experimental groups {% activation rate = (RFUsample - RFUlow control) / (RFUhigh control - RFUlow control)×100}. The test compound was diluted 3.16X in the reaction system to generate 11 concentration points ranging from 30 µM to 0.095 nM. XLFit was used to fit the percentage activation rate and 11-point concentration data to a parametric nonlinear logic formula to calculate the EC₅₀ value of the compound.

### 5. Experimental results:

| Example | M4 FLIPR EC₅₀ (nM) | Example | M4 FLIPR EC₅₀ (nM) |
|---|---|---|---|
| Example 20 | 14.70 | Example 196 | 33.9 |
| Example 25 | 9.5 | Example 200 | 39.4 |
| Example 29 | 37.0 | Example 201 | 56.4 |
| Example 32 | 72.4 | Example 203 | 90.0 |
| Example 34 | 40.4 | Example 204 | 33.0 |
| Example 38 | 37.9 | Example 208 | 42.6 |
| Example 52 | 13.9 | Example 213 | 12.4 |
| Example 53 | 8.0 | Example 227 | 90.4 |
| Example 54 | 9.6 | Example 226 | 29.9 |
| Example 55 | 18.1 | Example 232 | 36.4 |
| Example 57 | 18.0 | Example 230 | 16.3 |
| Example 60 | 8.8 | Example 238 | 35.1 |
| Example 61 | 10.6 | Example 239 | 42.6 |
| Example 70 | 73.6 | Example 235 | 35.3 |
| Example 71 | 71.9 | Example 236 | 49.1 |
| Example 72 | 87.5 | Example 247 | 96.3 |
| Example 76 | 40.4 | Example 220 | 53.5 |
| Example 100 | 18.4 | Example 221 | 94.3 |
| Example 101 | 33.5 | Example 217 | 99.7 |
| Example 103 | 24.4 | Example 264 | 47.8 |
| Example 104 | 53.8 | Example 269 | 87.1 |
| Example 105 | 19.9 | Example 270 | 23.2 |
| Example 127 | 15.2 | Example 284-P2 | 59.7 |
| Example 140 | 51.7 | Example 292 | 53.5 |
| Example 141 | 39.3 | Example 293 | 99.7 |
| Example 143 | 30.9 | Example 299 | 26.8 |
| Example 144 | 85.8 | Example 300 | 35.7 |
| Example 145 | 27.4 | Example 301 | 12.4 |
| Example 146 | 12.4 | Example 302 | 23.2 |
| Example 147 | 40.9 | Example 303 | 13.8 |
| Example 148 | 37.7 | Example 304 | 26.9 |
| Example 178 | 83.8 | Example 305 | 6.8 |
| Example 179 | 57.1 | Example 306 | 11.1 |
| Example 180 | 69.5 | Example 307 | 17.1 |
| Example 181 | 25.9 | Example 309 | 28.1 |
| Example 182 | 37.0 | Example 310 | 91.2 |
| Example 184 | 44.1 | Example 312 | 15.1 |
| Example 185 | 11.1 | Example 313 | 10.4 |
| Example 186 | 28.8 | Example 317 | 16.9 |
| Example 187 | 28.2 | Example 318 | 11.2 |
| Example 188 | 20.6 | Example 320 | 34.1 |
| Example 189 | 13.3 | Example 331 | 100.7 |
| Example 190 | 73.2 | Example 346 | 32.6 |
| Example 191 | 31.0 | | |

The results showed that the compound of the present invention exhibited a good potentiating effect in calcium flux assays using cells stably expressing the M4 receptor, had no activity against the M2 target (EC₅₀ ≥ 30 uM), and displayed better selectivity for M4.

### II. Pharmacokinetic determination in Balb/c mice

### 1. Study objective:

To investigate the pharmacokinetic behaviours of the example compounds in both plasma and brain of Balb/c mice (test animals) following oral administration at a dose of 5 mg/kg.

### 2. Test scheme

2.1 Test drugs: The example compounds of the present invention, made in house.

2.2 Test animals: Balb/c Mouse, male.

### 2.3 Administration:

24 Balb/c mice, male; p.o. respectively at a dose of 5 mg/kg and an administration volume of 10 mL/kg after the mice were fasted overnight.

### 2.4 Drug formulation:

Formulation of a drug for oral administration: 0.5% CMC-Na (1% Tween 80).

0.50 g of sodium carboxymethyl cellulose (CMC-Na, viscosity: 800-1200 Cps) was weighed and dissolved in 99 mL of purified water, and 1 ml of Tween 80 was added. The components were mixed thoroughly under stirring to form a clear solution.

The example compound was weighed into a 4-mL glass vial, 2.4 mL of the solution was added, and the mixture was subjected to ultrasound treatment for 10 minutes to obtain a homogeneous suspension with a concentration of 0.5 mg/mL.

### 2.5 Sample collection:

After the test animals were euthanised by CO2, blood samples were collected from the heart and brain tissues were harvested at 0.25 h, 0.5 h, 1 h, 2 h, 4 h, 6 h, 8 h, and 24 h post-dose. Blood was placed in EDTA-2K tubes, and centrifuged at 6000 rpm for 6 min at 4°C to separate the plasma. Brain tissues were rinsed with pre-cold PBS, blotted dry, and weighed. Both plasma and brain samples were stored at -80°C. The animals were fed 4 h after administration.

### 2.6 Sample treatment:

1) 40 uL of plasma sample was precipitated with 160 uL of acetonitrile, mixed, and centrifuged at 3500 × g for 5-20 min. Brain tissues were diluted, homogenised and centrifuged.
2) 100 uL of the treated supernatant solution was taken for LC/MS/MS analysis to determine the concentration of the test compounds.

### 2.7 Liquid phase analysis:

• Liquid phase conditions: Shimadzu LC-20AD pump
• Mass spectrometry conditions: AB Sciex API 4000 mass spectrometer
• Chromatographic column: Phenomenex Gemiu 5 um C18 50 × 4.6 mm
• Mobile phase: solution A: 0.1% formic acid in water, solution B: acetonitrile
• Flow rate: 0.8 mL/min
• Elution time: 0-4.0 min, with eluents as follows:

| Time/min | Solution A | Solution B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Experimental results and analysis

The main pharmacokinetic parameters were calculated using WinNonlin 8.1. The results of pharmacokinetic experiments in mice are as shown in the table below:

| No. | AUC_{0-∞} (ng/mL*h) | Cₘₐₓ (ng/mL) | Tₘₐₓ (h) | MRT _{0-∞} (h) |
|---|---|---|---|---|
| Example 32 | 7369 | 2563 | 0.5 | 2.5 |
| Example 52 | 2535 | 1947 | 0.25 | 1.2 |
| Example 105 | 10217 | 1573 | 0.5 | 4.3 |
| Example 122 | 6134 | 1547 | 0.5 | 2.6 |
| Example 123 | 4160 | 1045 | 0.5 | 3.8 |
| Example 144 | 4791 | 1887 | 0.5 | 1.4 |

### 4. Experimental conclusion:

The data in the table showed that in the mouse pharmacokinetic evaluation experiment, the example compounds of the present invention exhibited high exposure after oral administration.

### III. Pharmacokinetic determination in SD rats

### 1. Study objective:

To investigate the pharmacokinetic behaviours of the example compounds in both plasma and brain of SD rats (test animals) following oral administration at a dose of 5 mg/kg.

### 2. Test scheme

2.1 Test drugs: The example compounds of the present invention, made in house.

2.2 Test animals: SD Rat, male.

### 2.3 Administration:

24 SD Rats, male; p.o. respectively at a dose of 5 mg/kg and an administration volume of 10 mL/kg after the rats were fasted overnight.

### 2.4 Drug formulation:

Formulation of a drug for oral administration: 0.5% CMC-Na (1% Tween 80).

0.50 g of sodium carboxymethyl cellulose (CMC-Na, viscosity: 800-1200 Cps) was weighed and dissolved in 99 mL of purified water, and 1 ml of Tween 80 was added. The components were mixed thoroughly under stirring to form a clear solution.

The example compound was weighed into a 100 mL glass vial, 80 mL of the solution was added, and the mixture was subjected to ultrasound treatment for 10 minutes to obtain a homogeneous suspension with a concentration of 0.5 mg/mL.

### 2.5 Sample collection:

After the test animals were euthanised by CO2, blood samples were collected from the heart and brain tissues were harvested at 0.25 h, 0.5 h, 1 h, 2 h, 4 h, 6 h, 8 h, and 24 h post-dose. Blood was placed in EDTA-2K tubes, and centrifuged at 6000 rpm for 6 min at 4°C to separate the plasma. Brain tissues were rinsed with pre-cold PBS, blotted dry, and weighed. Both plasma and brain samples were stored at -80°C. The animals were fed 4 h after administration.

### 2.6 Sample treatment:

3) 40 uL of plasma sample was precipitated with 160 uL of acetonitrile, mixed, and centrifuged at 3500 × g for 5-20 min. Brain tissues were diluted, homogenised and centrifuged.
4) 100 uL of the treated supernatant solution was taken for LC/MS/MS analysis to determine the concentration of the test compounds.

### 2.7 Liquid phase analysis:

• Liquid phase conditions: Shimadzu LC-20AD pump
• Mass spectrometry conditions: AB Sciex API 4000 mass spectrometer
• Chromatographic column: Phenomenex Gemiu 5 um C18 50 × 4.6 mm
• Mobile phase: solution A: 0.1% formic acid in water, solution B: acetonitrile
• Flow rate: 0.8 mL/min
• Elution time: 0-4.0 min, with eluents as follows:

| Time/min | Solution A | Solution B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Experimental results and analysis:

The main pharmacokinetic parameters were calculated using WinNonlin 8.1. The results of pharmacokinetic experiments in rats are as shown in the table below:

| No. | AUC_{0-∞} (ng/mL*h) | Cₘₐₓ (ng/mL) | Tₘₐₓ (h) | MRT _{0-∞} (h) |
|---|---|---|---|---|
| Example 32 | 17455 | 4520 | 2.0 | 2.7 |
| Example 52 | 10788 | 2450 | 2.0 | 2.6 |
| Example 76 | 6153 | 1094 | 2.0 | 3.9 |
| Example 105 | 16301 | 2677 | 1.0 | 4.3 |
| Example 122 | 34812 | 3107 | 2.0 | 5.8 |
| Example 123 | 12212 | 2530 | 2.0 | 3.5 |
| Example 141 | 8543 | 3207 | 0.5 | 1.7 |
| Example 142 | 9321 | 1383 | 2.0 | 4.4 |
| Example 147 | 3264 | 914 | 1.0 | 2.4 |
| Example 182 | 7047 | 1707 | 1.0 | 2.8 |
| Example 188 | 3170 | 967 | 1.0 | 2.6 |
| Example 189 | 3223 | 1041 | 1.0 | 2.6 |
| Example 191 | 16071 | 2840 | 2.0 | 3.9 |
| Example 144 | 10997 | 1713 | 2.0 | 4.6 |
| Example 236 | 6712 | 1086 | 1.5 | 4.6 |
| Example 269 | 3642 | 1329 | 0.83 | 2.0 |
| Example 270 | 11973 | 3497 | 0.83 | 2.5 |
| Example 304 | 4078 | 2377 | 0.50 | 1.32 |
| Example 307 | 5295 | 2110 | 0.75 | 2.02 |
| Example 317 | 26653 | 2137 | 4.0 | 11.2 |
| Example 331 | 10697 | 944 | 2.0 | 6.8 |
| Example 346 | 11428 | 1457 | 1.00 | 5.02 |

### 4. Experimental conclusion:

The above results showed that, in the rat pharmacokinetic evaluation experiment, the example compounds of the present invention exhibited high exposure after oral administration, along with a higher plasma protein unbound fraction and higher brain exposure. The plasma protein unbound fraction was more than 8-fold, or even over 20-fold, and the ratio of total compound concentration in brain to that in plasma reached above 0.2, or even above 0.3.

## Claims

1. A compound represented by general formula (II-B), (II-C), (II-D) or (II-E), or a stereoisomer or pharmaceutically acceptable salt thereof: 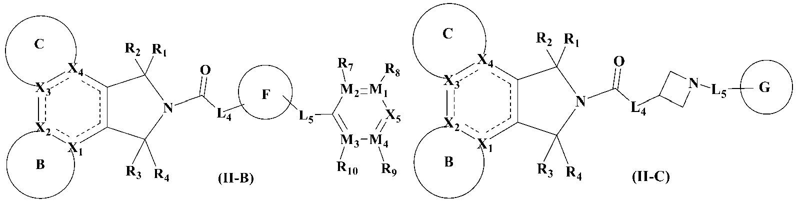 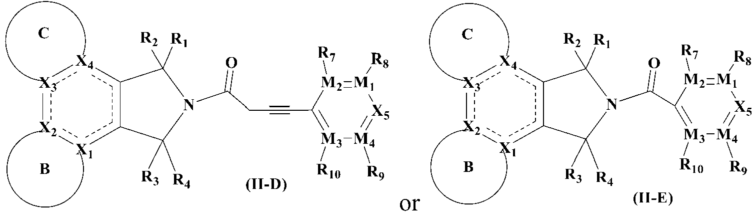 **characterised in that**
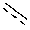 is a single bond or a double bond;
each X₁ is independently selected from a bond, C, CR₁₁, CR₁₁R₂₂ or N;
each X₂ is independently selected from a bond, C, CR₂₂, CR₁₁R₂₂ or N;
each X₃ is independently selected from a bond, C, CR₃₃, CR₁₁R₂₂ or N;
each X₄ is independently selected from a bond, C, CR₄₄, CR₁₁R₂₂ or N;
each L₄ or L₅ is independently selected from a bond, C(R₅R₆), N(R₅), O or S;
each X₅ is independently selected from CR₅₅ or N;
each M₁, M₂, M₃ or M₄ is independently selected from C or N;
when M₁, M₂, M₃ or M₄ is N, R₇, R₈, R₉ or R₁₀ is absent;
each R₁₁, R₂₂, R₃₃ or R₄₄ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
ring B or ring C is present or absent, and when present, each is independently selected from cycloalkyl, heterocyclyl, aryl or heteroaryl, wherein the cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
at least one of ring B and ring C is present;
each ring F or ring G is independently selected from cycloalkyl, heterocyclyl, aryl or heteroaryl, wherein the cycloalkyl, heterocyclyl, aryl or heteroaryl may optionally be further substituted with one or more substituents selected from R₅₅, R₇, R₈, R₉ or R₁₀;
each R₁, R₂, R₃, R₄, R₅ or R₆ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, oxo, thio, halogen, amino, hydroxyl, cyano, nitro, alkyl, alkenyl, alkynyl, deuteroalkyl, haloalkyl, alkoxy, haloalkoxy, hydroxyalkyl or cyano-substituted alkyl;
alternatively, R₁ and R₂, R₃ and R₄, or R₅ and R₆, together with the carbon atom to which they are each attached, form cycloalkyl or heterocyclyl, wherein the cycloalkyl or heterocyclyl is optionally substituted with one or more substituents selected from deuterium, oxo, thio, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
each R₅₅, R₇, R₈, R₉ or R₁₀ is independently selected from hydrogen, deuterium, halogen, amino, hydroxyl, cyano, nitro, alkyl, alkenyl, alkynyl, deuteroalkyl, haloalkyl, alkoxy, haloalkoxy, deuterated alkoxy, hydroxyalkyl, cyano-substituted alkyl, cycloalkyl, heterocyclyl, aryl, heteroaryl, -O(CRₐₐR_{bb})ₙ₁R₃₁, -N(CRₐₐR_{bb})ₙ₁R₃₁, -S(CRₐₐR_{bb})ₙ₁R₃₁, -OCR₃₁R₃₂R₃₃, -(CRₐₐR_{bb})ₙ₁C(O)R₃₁, N=S=OR₃₁R₃₂, N(R₃₁)=S=OR₃₂-, -(CH₂)ₐ₁C(O)NR₃₁R₃₂, -(CH₂)ₙ₁P(O)R₃₁R₃₂, -(CH₂)ₙ₁P(O)₂R₃₁R₃₂, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
each R₃₁, R₃₂, R₃₃, Rₐₐ, R_{bb} or R_{cc} is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
each m1 is independently 0, 1 or 2;
each n1 is independently 0, 1 or 2.

2. The compound, or the stereoisomer or pharmaceutically acceptable salt thereof according to claim 1, **characterised in that** the compound is further represented by general formula (II-A-1), (II-A-2), (II-A-3), (II) or (II-1): 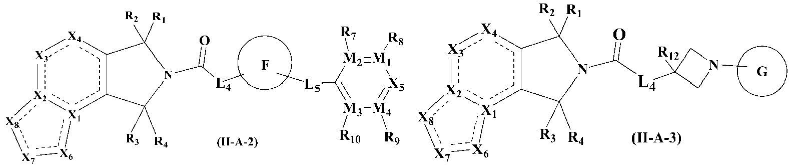 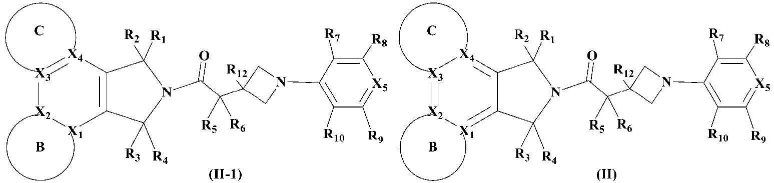 or 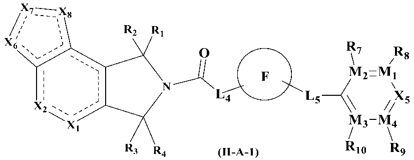 wherein:
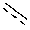 is a single bond or a double bond;
each X₁ is independently selected from a bond, C, CR₁₁, CR₁₁R₂₂ or N;
each X₂ is independently selected from a bond, C, CR₂₂, CR₁₁R₂₂ or N;
each X₃ is independently selected from a bond, C, CR₃₃, CR₁₁R₂₂ or N;
each X₄ is independently selected from a bond, C, CR₄₄, CR₁₁R₂₂ or N;
each X₅ is independently selected from CR₅₅ or N;
each M₁, M₂, M₃ or M₄ is independently selected from C or N;
when M₁, M₂, M₃ or M₄ is N, R₇, R₈, R₉ or R₁₀ is absent;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, (CR₆₆)ₙ₂, N or O;
each L₄ or L₅ is independently selected from a bond, C(R₅R₆), N(R₅), O or S;
each R₁₁, R₂₂, R₃₃, R₄₄, R₆₆, R₇₇, R₈₈, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, hydroxyl, halogen, cyano, amino, C₁₋₆ alkyl, C₁₋₆ haloalkyl or C₁₋₆ deuteroalkyl;
each ring F is independently selected from C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
each ring G is independently selected from C₃₋₁₂ cycloalkyl, 3- to 12-membered heterocyclyl, C₆₋₁₂ aryl or 5- to 14-membered heteroaryl, wherein the C₃₋₁₂ cycloalkyl, 3- to 12-membered heterocyclyl, C₆₋₁₂ aryl or 5- to 14-membered heteroaryl may optionally be further substituted with one or more substituents selected from R₅₅, R₇, R₈, R₉ or R₁₀;
alternatively, R₁ and R₂, R₃ and R₄, or R₅ and R₆, together with the carbon atom to which they are each attached, form C₃₋₁₂ cycloalkyl or 3- to 12-membered heterocyclyl, wherein the C₃₋₁₂ cycloalkyl or 3- to 12-membered heterocyclyl is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₅₅, R₇, R₈, R₉, R₁₀ or R₁₂ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, deuterated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -O(CRₐₐR_{bb})ₙ₁R₃₁, -N(CRₐₐR_{bb})ₙ₁R₃₁, -S(CRₐₐR_{bb})ₙ₁R₃₁, -OCR₃₁R₃₂R₃₃, - (CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, - (CH₂)ₙ₁P(O)₂R₃₁R₃₂, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl;
each m1 is independently 0, 1 or 2;
each n1 is independently 0, 1 or 2; and
each n2 is independently 1 or 2.

3. The compound, or the stereoisomer or pharmaceutically acceptable salt thereof according to claim 1 or 2, **characterised in that** the compound is not selected from the following structures:

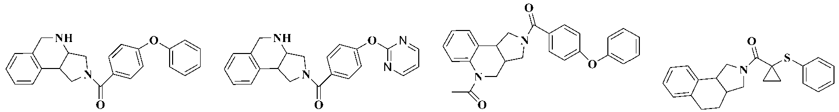

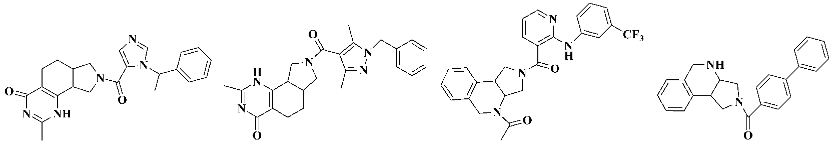

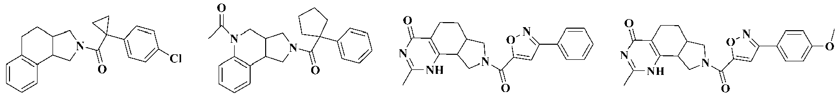

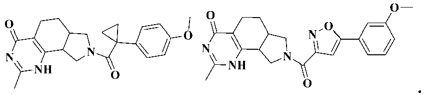

4. The compound, or the stereoisomer or pharmaceutically acceptable salt thereof according to claim 2, **characterised in that** the compound is further represented by general formula (II-B-1), (II-B-2) or (II-B-5): 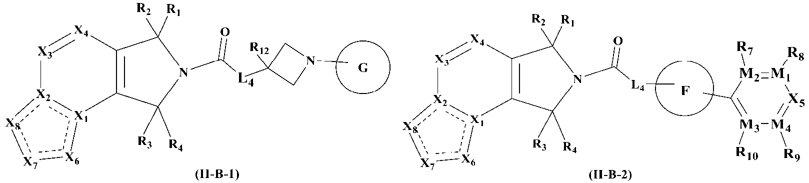 or 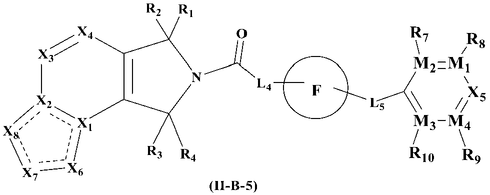
each X₁ is independently selected from a bond, C, CR₁₁ or N;
each X₂ is independently selected from a bond, C, CR₂₂ or N;
each X₃ is independently selected from a bond, C, CR₃₃ or N;
each X₄ is independently selected from a bond, C, CR₄₄ or N.

5. The compound, or the stereoisomer or pharmaceutically acceptable salt thereof according to claim 4, **characterised in that** the compound is further represented by general formula (II-A-a2), (II-A-a3), (II-A-a4), (II-A-a5), (II-A-a6), (II-A-a7), (II-A-a8) or (II-A-a9):

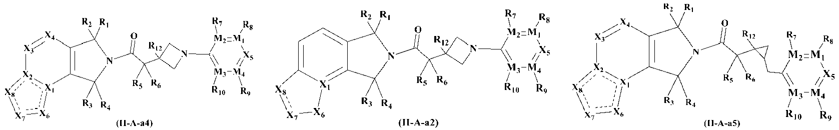

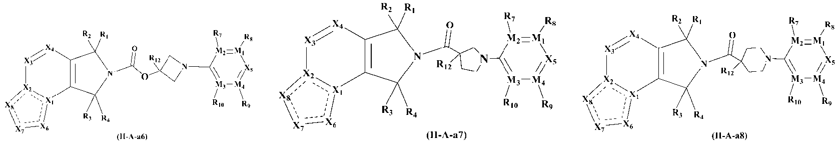

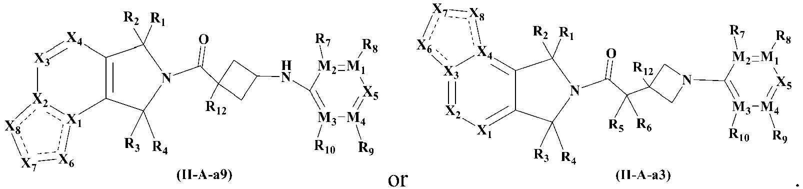

6. The compound, or the stereoisomer or pharmaceutically acceptable salt thereof according to claim 4, **characterised in that** the compound is further represented by general formula (II-A-a1), (II-B-a1), (II-B-a2), (II-B-a3), (II-B-a4), (II-B-a5), (II-B-a6), (II-B-a7), (II-B-a8), (II-B-a9) or (II-B-a10): 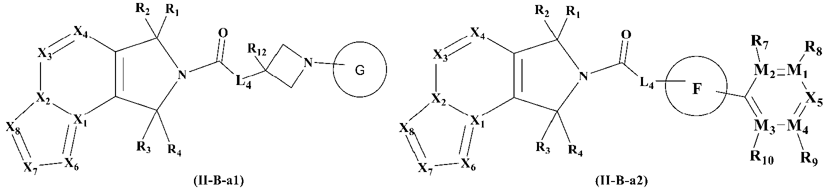 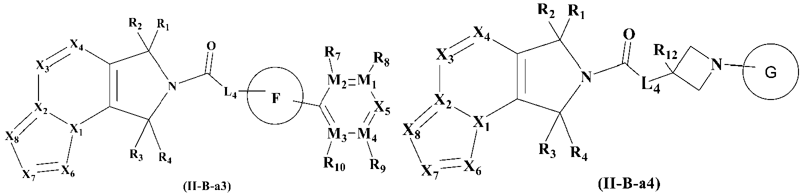 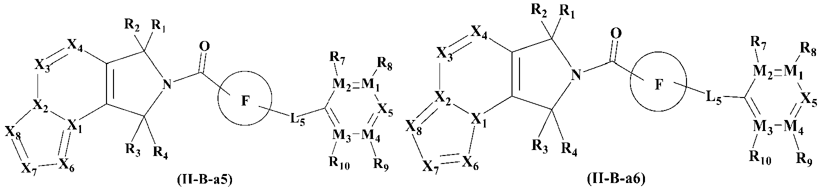 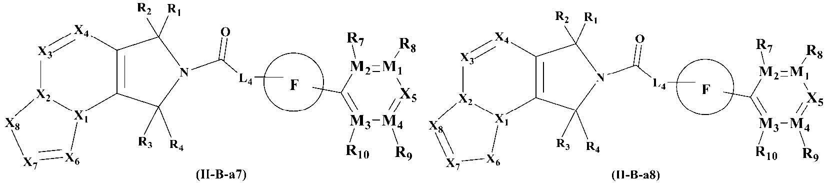 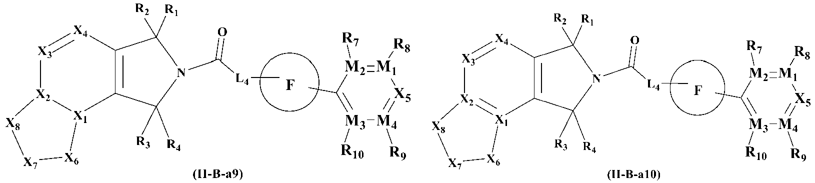 or 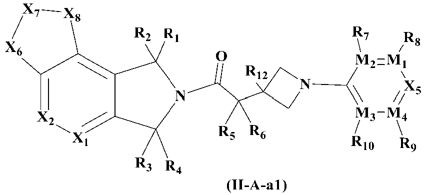
each ring F is independently selected from C₃₋₈ cycloalkyl, 3- to 8-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
each ring G is independently selected from C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S may optionally be further substituted with one or more substituents selected from R₅₅, R₇, R₈, R₉ or R₁₀.

7. The compound, or the stereoisomer or pharmaceutically acceptable salt thereof according to claim 6, **characterised in that** the compound is further represented by general formula (II-B-3), (II-B-4), (II-6), (II-7), (II-8), (II-9), (II-10), (II-11), (II-12), (II-13), (II-14), (II-15), (II-16), (II-17) or (II-18): 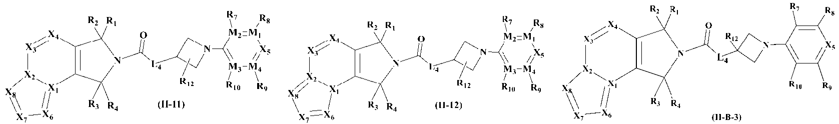 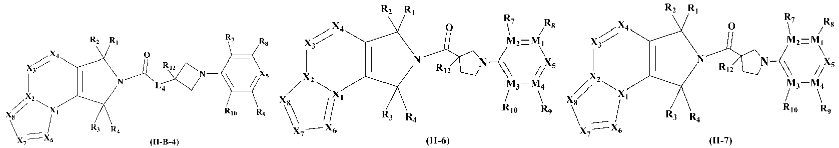 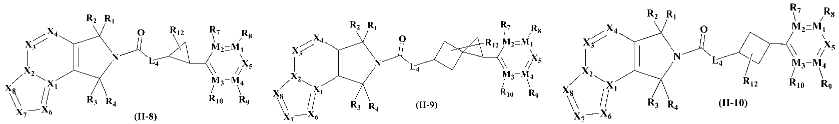 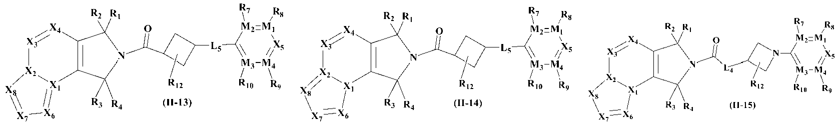 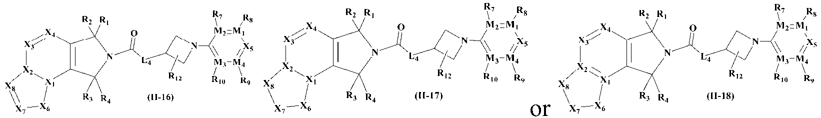 wherein:
each X₁ is independently selected from C, CR₁₁ or N;
each X₂ is independently selected from C, CR₂₂ or N;
each X₃ is independently selected from CR₃₃ or N;
each X₄ is independently selected from CR₄₄ or N;
each X₅ is independently selected from CR₅₅ or N;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, (CR₆₆)ₙ₂, N or O;
each L₄ is independently selected from a bond, C(R₅R₆), N(R₅), O or S;
each R₁₁, R₂₂, R₃₃, R₄₄, R₆₆, R₇₇, R₈₈, R₁, R₂, R₃, R₄, R₅ or R₆ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S is optionally substituted with one or more substituents selected from deuterium, hydroxyl, halogen, cyano, amino, C₁₋₆ alkyl, C₁₋₆ haloalkyl or C₁₋₆ deuteroalkyl;
alternatively, R₁ and R₂, R₃ and R₄, or R₅ and R₆, together with the carbon atom to which they are each attached, form C₃₋₁₀ cycloalkyl or 3- to 10-membered heterocyclyl containing 1 to 3 heteroatoms selected from N, O or S, wherein the C₃₋₁₀ cycloalkyl or 3- to 10-membered heterocyclyl containing 1 to 3 heteroatoms selected from N, O or S is optionally substituted with one or more substituents selected from deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
each R₅₅, R₇, R₈, R₉ or R₁₀ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, deuterated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, -O(CRₐₐR_{bb})ₙ₁R₃₁, -N(CRₐₐR_{bb})ₙ₁R₃₁, -S(CRₐₐR_{bb})ₙ₁R₃₁, -OCR₃₁R₃₂R₃₃, - (CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, - (CH₂)ₙ₁P(O)₂R₃₁R₃₂, -(CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S;
each R₃₁, R₃₂, R₃₃, Rₐₐ, R_{bb} or R_{cc} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
each m1 is independently selected from 0, 1 or 2; and
each n1 is independently selected from 0, 1 or 2.

8. The compound, or the stereoisomer or pharmaceutically acceptable salt thereof according to claim 7, **characterised in that** the compound is further represented by general formula (II-1b), (II-2b), (II-3b), (II-4b), (II-5b), (II-6b), (II-7b), (II-8b), (II-9b), (II-10b), (II-11b), (II-12b), (II-13b), (II-14b), (II-15b), (II-16b), (II-17b), (II-18b) or (II-19b): 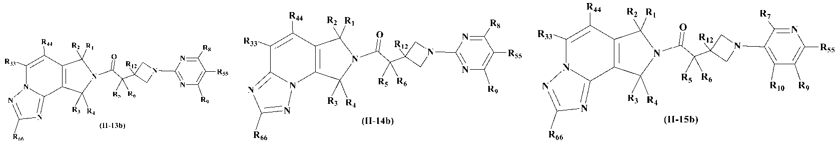 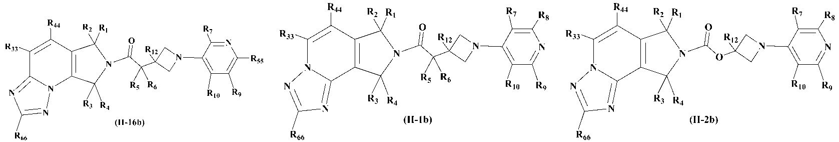 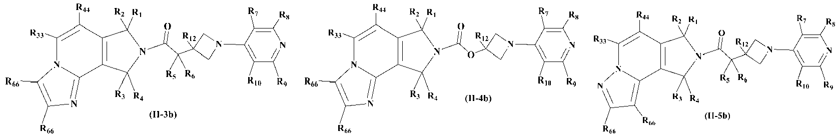 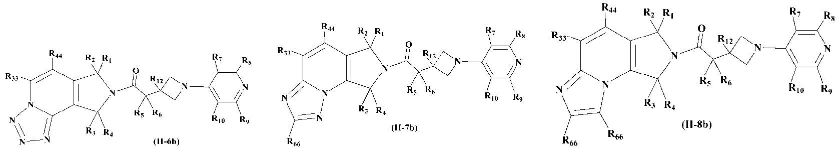 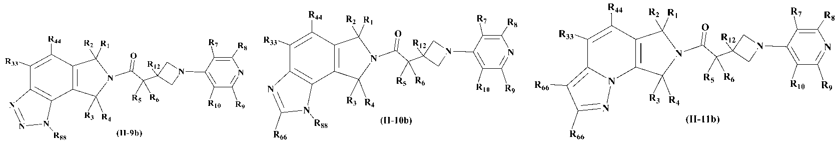 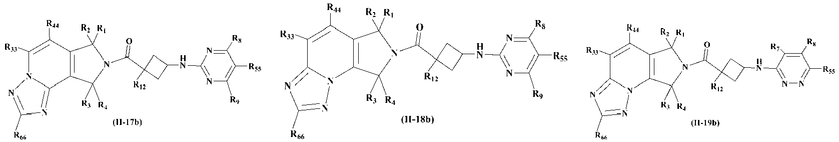 or

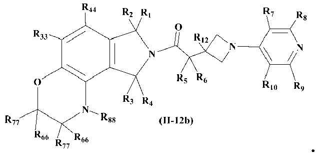

9. The compound, or the stereoisomer or pharmaceutically acceptable salt thereof according to any one of claims 1-7, **characterised in that**
each R₁₁, R₂₂, R₃₃ or R₄₄ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each R₁₁, R₂₂, R₃₃ or R₄₄ is independently selected from hydrogen, deuterium, fluorine, chlorine, bromine, amino, hydroxyl, cyano, nitro, methyl, ethyl, propyl, isopropyl, fluoromethyl, difluoromethyl, trifluoromethyl, cyclopropyl, cyclobutyl, epoxyethyl, epoxypropyl or epoxybutyl, wherein the amino, methyl, ethyl, propyl, isopropyl, fluoromethyl, difluoromethyl, trifluoromethyl, cyclopropyl, cyclobutyl, epoxyethyl, epoxypropyl or epoxybutyl is optionally substituted with one or more substituents selected from deuterium, hydroxyl, halogen, cyano, amino, C₁₋₃ alkyl, C₁₋₃ haloalkyl or C₁₋₃ deuteroalkyl.

10. The compound, or the stereoisomer or pharmaceutically acceptable salt thereof according to any one of claims 1-8, **characterised in that**
each R₅₅ or R₈ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, deuterated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl, 5- to 10-membered heteroaryl containing 1 to 3 heteroatoms selected from N, O or S, - O(CRₐₐR_{bb})ₙ₁R₃₁, -N(CRₐₐR_{bb})ₙ₁R₃₁, -S(CRₐₐR_{bb})ₙ₁R₃₁, -(CRₐₐR_{bb})ₙ₁C(O)R₃₁, N=S=OR₃₁R₃₂, -(CH₂)ₙ₁C(O)NR₃₁R₃₂, -(CH₂)ₙ₁P(O)R₃₁R₃₂, -(CH₂)ₙ₁P(O)₂R₃₁, - (CRₐₐR_{bb})ₙ₁(NR₃₁)C(O)R₃₂, -(CRₐₐR_{bb})ₙ₁C(O)NR₃₁R₃₂, - (CRₐₐR_{bb})ₙ₁(NR₃₁)S(O)ₘ₁R₃₂, -(CRₐₐR_{bb})ₙ₁S(O)ₘ₁NR₃₁R₃₂, - (CRₐₐR_{bb})ₙ₁S(O)(=NR_{cc})R₃₂ or -(CRₐₐR_{bb})ₙ₁S(O)ₘ₁R₃₂, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, deuterated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
each R₃₁, R₃₂ or R₃₃ is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₆ cycloalkyl, 3- to 6-membered heterocyclyl containing 1 to 3 heteroatoms selected from N, O or S, C₆₋₁₀ aryl or 5- to 8-membered heteroaryl containing 1 to 3 heteroatoms selected from N, O or S, wherein the amino, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₆ cycloalkyl, 3- to 6-membered heterocyclyl containing 1 to 3 heteroatoms selected from N, O or S, C₆₋₁₀ aryl or 5- to 8-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each R₃₁, R₃₂ or R₃₃ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, cyclopropyl, cyclobutyl, cyclopentyl, aminomethyl, methoxy, ethoxy or propoxy, wherein the amino, methylamino, dimethylamino, methyl, ethyl, isopropyl, fluoromethyl, fluoroethyl, difluoromethyl, difluoroethyl, trifluoroethyl, cyclopropyl, cyclobutyl, cyclopentyl, aminomethyl, methoxy, ethoxy or propoxy is optionally substituted with one or more substituents selected from deuterium, hydroxyl, fluorine, chlorine, bromine, cyano, amino, C₁₋₃ alkyl, C₁₋₃ haloalkyl or C₁₋₃ deuteroalkyl;
each Rₐₐ, R_{bb} or R_{cc} is independently selected from hydrogen, deuterium, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl, wherein the amino, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each Rₐₐ, R_{bb} or R_{cc} is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy.

11. The compound, or the stereoisomer or pharmaceutically acceptable salt thereof according to any one of claims 1-8, **characterised in that**
each R₁, R₂, R₃, R₄, R₅ or R₆ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each R₁, R₂, R₃, R₄, R₅ or R₆ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy;
alternatively, R₁ and R₂, R₃ and R₄, or R₅ and R₆, together with the carbon atom to which they are each attached, form C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S, wherein the C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
preferably, R₁ and R₂, R₃ and R₄, or R₅ and R₆, together with the carbon atom to which they are each attached, form C₃₋₈ cycloalkyl, 3- to 8-membered heterocyclyl containing 1 to 3 heteroatoms selected from N, O or S, C₆₋₁₀ aryl or 5- to 8-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 8-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, R₁ and R₂, R₃ and R₄, or R₅ and R₆, together with the carbon atom to which they are attached, form cyclopropyl, cyclobutyl, cyclopentyl or cyclohexyl.

12. The compound, or the stereoisomer or pharmaceutically acceptable salt thereof according to any one of claims 1-8, **characterised in that**
each R₇, R₉ or R₁₀ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
preferably, each R₇, R₉ or R₁₀ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 8-membered heterocyclyl containing 1 to 3 heteroatoms selected from N, O or S, C₆₋₁₀ aryl or 5-to 8-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 8-membered heterocyclyl containing 1 to 3 heteroatoms selected from N, O or S, C₆₋₁₀ aryl or 5- to 8-membered heteroaryl containing 1 to 3 heteroatoms selected from N, O or S is optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, or cyano-substituted C₁₋₃ alkyl;
more preferably, each R₇, R₉ or R₁₀ is independently selected from hydrogen, deuterium, fluorine, chlorine, bromine, amino, hydroxyl, cyano, nitro, methyl, ethyl, propyl, vinyl, allyl, propenyl, ethynyl, propynyl, propargyl, deuterated methyl, deuterated ethyl, deuterated propyl, fluoromethyl, fluoroethyl, trifluoromethyl, difluoromethyl, methoxy, ethoxy, propoxy, fluoro-substituted methoxy, fluoro-substituted ethoxy, trifluoromethoxy, hydroxymethyl, hydroxyethyl, hydroxypropyl, 2-hydroxypropyl, cyano-substituted methyl, cyano-substituted ethyl, cyano-substituted propyl, cyclopropyl, cyclobutyl, cyclopentyl or cyclohexyl.

13. The compound, or the stereoisomer or pharmaceutically acceptable salt thereof according to any one of claims 3-8, **characterised in that**
R₁₂ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S is optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S;
preferably, R₁₂ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 8-membered heterocyclyl containing 1 to 3 heteroatoms selected from N, O or S, C₆₋₁₀ aryl or 5- to 8-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 8-membered heterocyclyl containing 1 to 3 heteroatoms selected from N, O or S, C₆₋₁₀ aryl or 5-to 8-membered heteroaryl containing 1 to 3 heteroatoms selected from N, O or S is optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, or cyano-substituted C₁₋₃ alkyl;
more preferably, R₁₂ is selected from hydrogen, deuterium, fluorine, chlorine, bromine, amino, hydroxyl, cyano, nitro, methyl, ethyl, propyl, vinyl, allyl, propenyl, ethynyl, propynyl, propargyl, deuterated methyl, deuterated ethyl, deuterated propyl, fluoromethyl, fluoroethyl, trifluoromethyl, difluoromethyl, methoxy, ethoxy, propoxy, fluoro-substituted methoxy, fluoro-substituted ethoxy, trifluoromethoxy, hydroxymethyl, hydroxyethyl, hydroxypropyl, 2-hydroxypropyl, cyano-substituted methyl, cyano-substituted ethyl, cyano-substituted propyl, cyclopropyl, cyclobutyl, cyclopentyl or cyclohexyl.

14. The compound, or the stereoisomer or pharmaceutically acceptable salt thereof according to any one of claims 1-3, **characterised in that**
ring B is selected from C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
preferably, ring B is selected from C₃₋₆ cycloalkyl, 5- to 6-membered heterocyclyl containing 1 to 3 heteroatoms selected from N, O or S, C₆₋₁₀ aryl or 5-to 6-membered heteroaryl containing 1 to 3 heteroatoms selected from N, O or S, wherein the C₃₋₆ cycloalkyl, 5- to 6-membered heterocyclyl containing 1 to 3 heteroatoms selected from N, O or S, C₆₋₁₀ aryl or 5- to 6-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, ring B is selected from cyclopropyl, cyclobutyl, cyclopentyl, cyclohexyl, 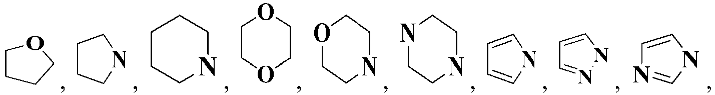 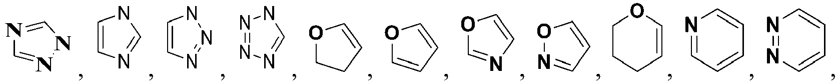 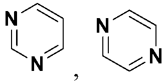 or phenyl, which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl;
further preferably, ring B is selected from 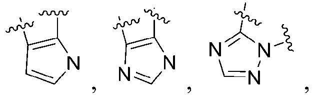 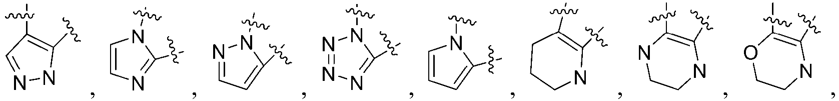 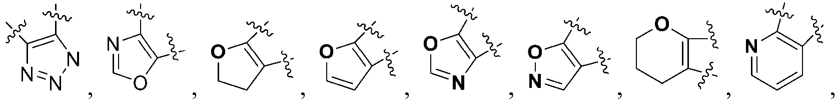 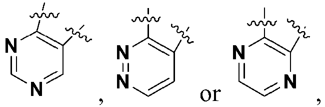 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl;
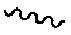 indicates the point of attachment to a fused ring.

15. The compound, or the stereoisomer or pharmaceutically acceptable salt thereof according to any one of claims 1-3, **characterised in that**
ring C is selected from C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
preferably, ring C is selected from C₃₋₆ cycloalkyl, 5- to 6-membered heterocyclyl containing 1 to 3 heteroatoms selected from N, O or S, C₆₋₁₀ aryl or 5-to 6-membered heteroaryl containing 1 to 3 heteroatoms selected from N, O or S, wherein the C₃₋₆ cycloalkyl, 5- to 6-membered heterocyclyl containing 1 to 3 heteroatoms selected from N, O or S, C₆₋₁₀ aryl or 5- to 6-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, ring C is selected from cyclopropyl, cyclobutyl, cyclopentyl, cyclohexyl, 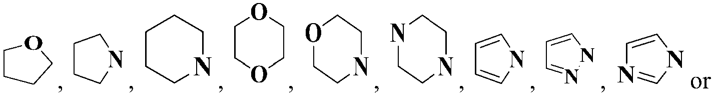 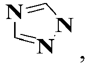 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl.

16. The compound, or the stereoisomer or pharmaceutically acceptable salt thereof according to any one of claims 2-7, **characterised in that**
each R₆₆, R₇₇ or R₈₈ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl;
preferably, each R₆₆, R₇₇ or R₈₈ is independently selected from hydrogen, deuterium, fluorine, chlorine, bromine, amino, hydroxyl, cyano, nitro, methyl, ethyl, propyl, isopropyl, fluoromethyl, difluoromethyl, trifluoromethyl, cyclopropyl, cyclobutyl, epoxyethyl, epoxypropyl or epoxybutyl, wherein the amino, methyl, ethyl, propyl, isopropyl, fluoromethyl, difluoromethyl, trifluoromethyl, cyclopropyl, cyclobutyl, epoxyethyl, epoxypropyl or epoxybutyl is optionally substituted with one or more substituents selected from deuterium, hydroxyl, halogen, cyano, amino, C₁₋₃ alkyl, C₁₋₃ haloalkyl or C₁₋₃ deuteroalkyl.

17. The compound, or the stereoisomer or pharmaceutically acceptable salt thereof according to any one of claims 1-4 or 6, **characterised in that**
ring F is selected from C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
preferably, ring F is selected from C₃₋₆ cycloalkyl, 5- to 6-membered heterocyclyl containing 1 to 3 heteroatoms selected from N, O or S, C₆₋₁₀ aryl or 5-to 6-membered heteroaryl containing 1 to 3 heteroatoms selected from N, O or S, wherein the C₃₋₆ cycloalkyl, 5- to 6-membered heterocyclyl containing 1 to 3 heteroatoms selected from N, O or S, C₆₋₁₀ aryl or 5- to 6-membered heteroaryl containing 1 to 3 heteroatoms selected from N, O or S is optionally substituted with one or more substituents selected from deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl;
more preferably, ring F is selected from cyclopropyl, cyclobutyl, cyclopentyl, cyclohexyl, 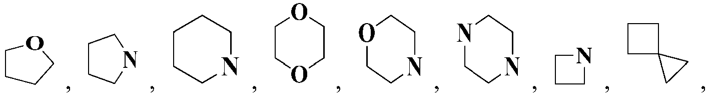 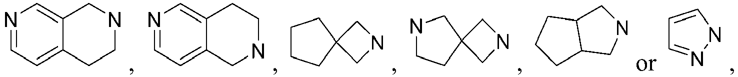 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl;
further preferably, ring F is selected from 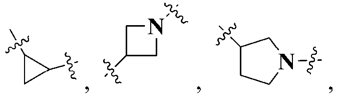 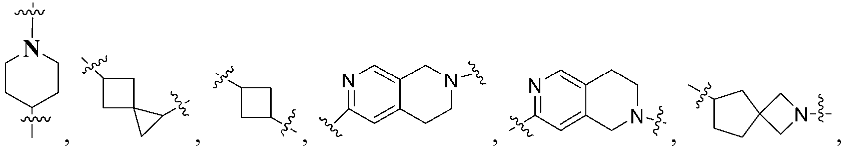 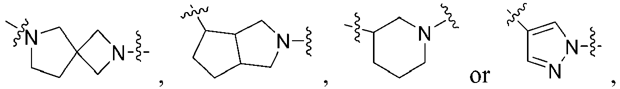 which is optionally substituted with one or more substituents selected from hydrogen, deuterium, fluorine, chlorine, bromine, amino, hydroxyl, cyano, nitro, methyl, ethyl, isopropyl, fluoromethyl, fluoroethyl, difluoromethyl, difluoroethyl, trifluoromethyl or trifluoroethyl;
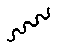 indicates the point of attachment to L₄ or L₅; when L₄ or L₅ is a bond, it indicates the point of attachment to carbonyl or ring G.

18. The compound, or the stereoisomer or pharmaceutically acceptable salt thereof according to any one of claims 1-4 or 6, **characterised in that**
ring G is selected from C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the C₃₋₈ cycloalkyl, 3- to 8-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from R₅₅, R₇, R₈, R₉ or R₁₀;
preferably, ring G is selected from C₃₋₈ cycloalkyl, 5- to 6-membered monocyclic heterocyclyl containing 1 to 3 heteroatoms selected from N, O or S, 6-to 10-membered fused heterocyclyl containing 1 to 3 heteroatoms selected from N, O or S, 5- to 10-membered spiro heterocyclyl containing 1 to 3 heteroatoms selected from N, O or S, C₆₋₁₀ aryl, 5- to 6-membered monocyclic heteroaryl containing 1 to 3 heteroatoms selected from N, O or S, or 8- to 10-membered fused heteroaryl containing 1 to 3 heteroatoms selected from N, O or S, which is optionally substituted with one or more substituents selected from R₅₅, R₇, R₈, R₉ or R₁₀;
more preferably, ring G is selected from a benzene ring, a naphthalene ring, pyridine, 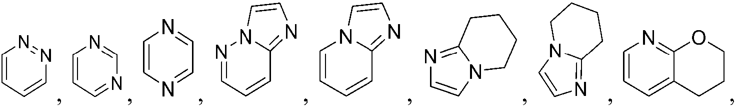 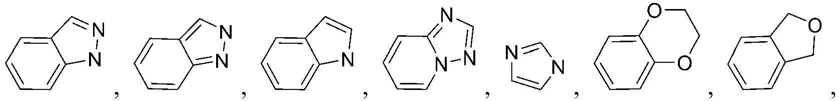 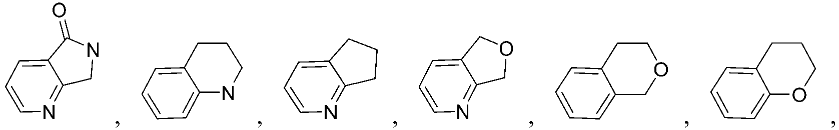 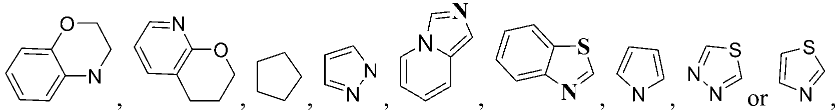 which is optionally substituted with one or more substituents selected from R₅₅, R₇, R₈, R₉ or R₁₀;
further preferably, ring G is selected from 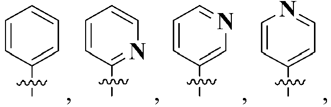 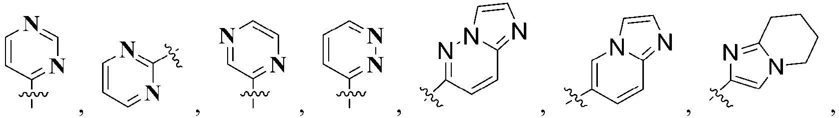 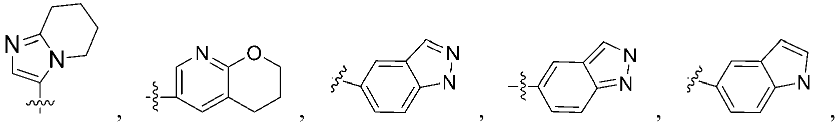 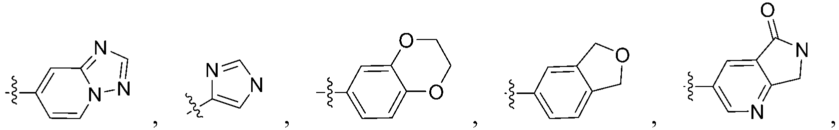 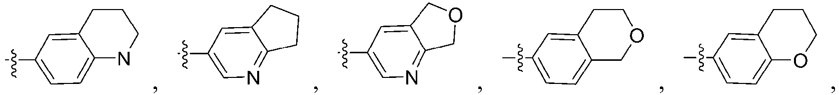 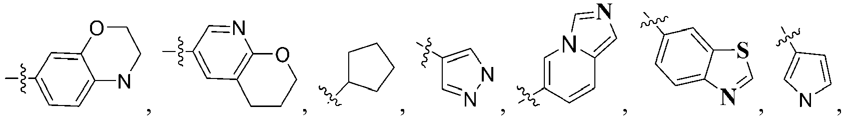 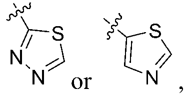 which is optionally substituted with one or more substituents selected from R₅₅, R₇, R₈, R₉ or R₁₀;
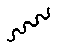 indicates the point of attachment to L₅; when L₅ is a bond, it indicates the point of attachment to ring F;when ring F is absent, it indicates the point of attachment to L₄; when L₄ is a bond, it indicates the point of attachment to C(O).

19. The compound, or the stereoisomer or pharmaceutically acceptable salt thereof according to claim 1, **characterised in that** the compound has the following structure:

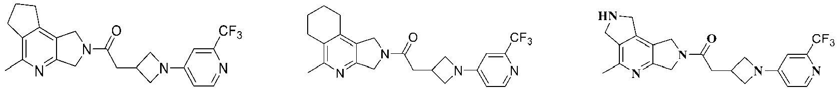

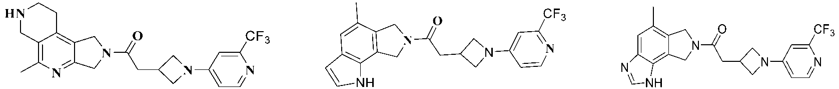

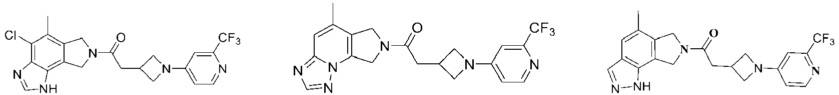

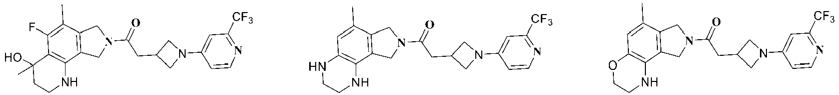

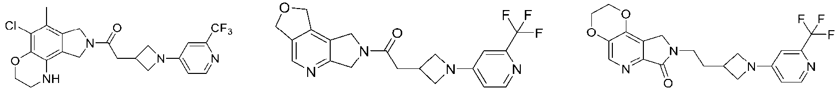

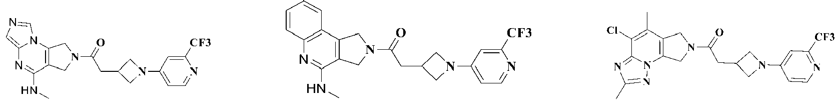

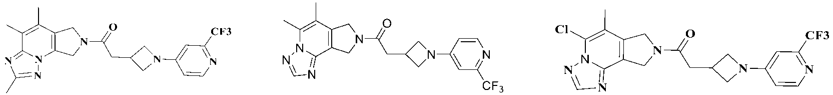

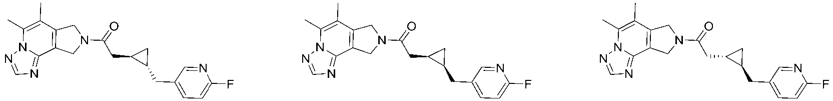

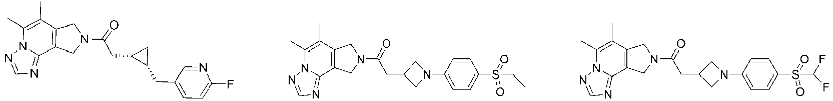

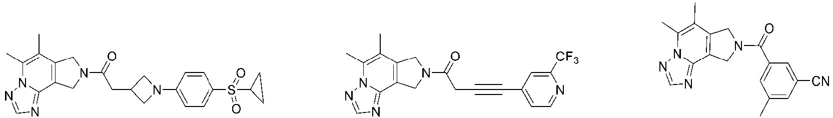

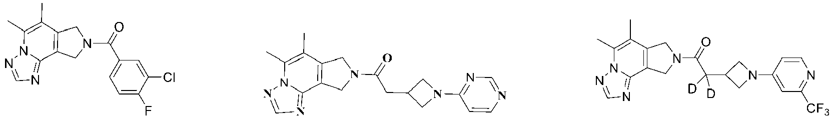

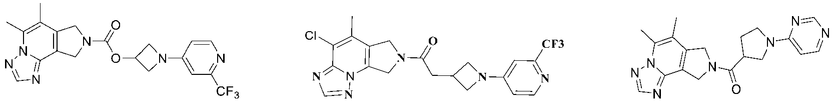

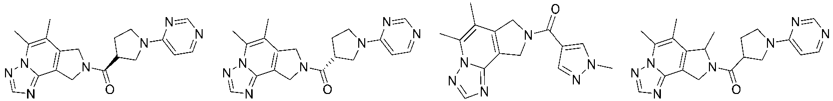

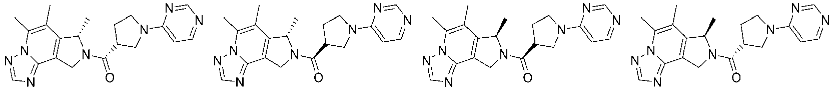

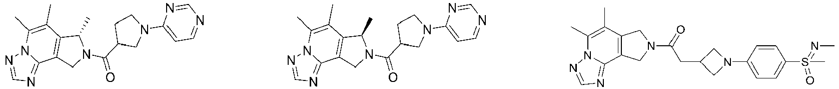

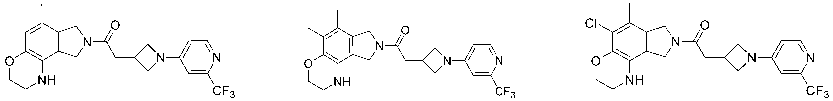

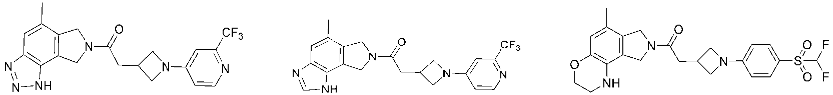

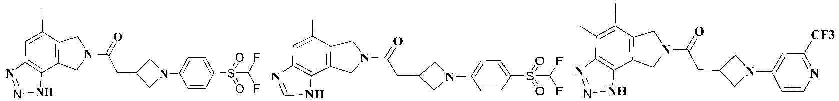

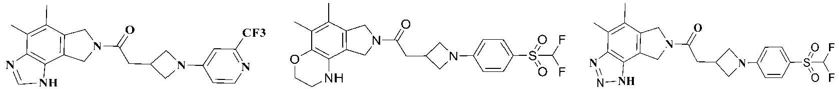

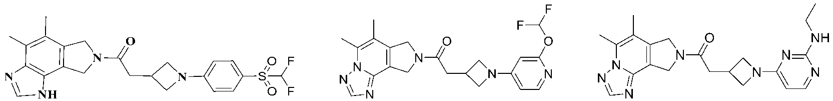

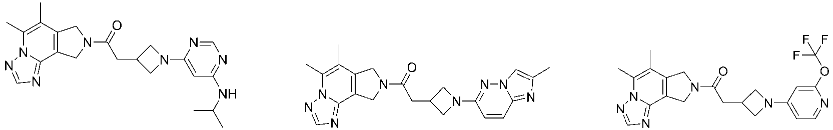

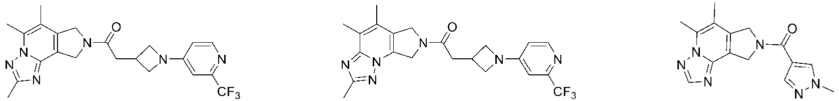

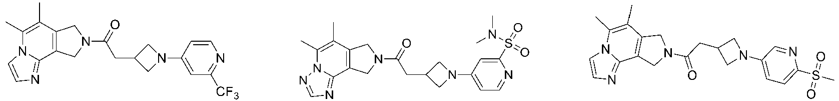

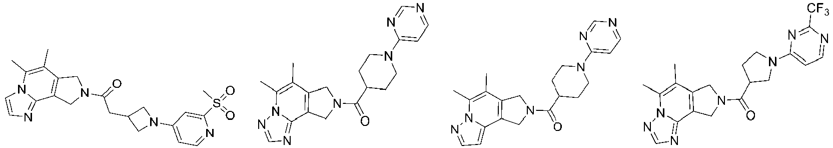

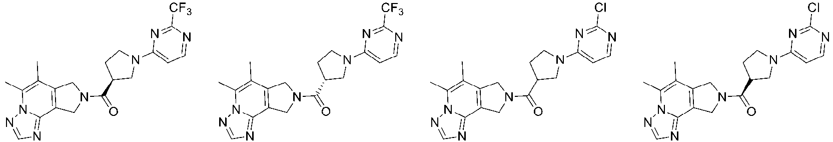

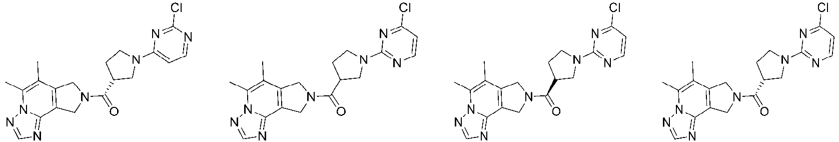

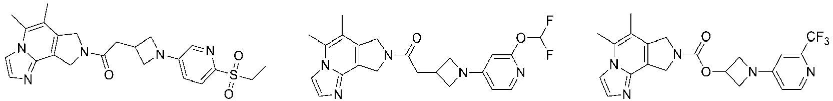

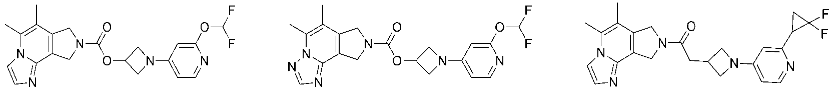

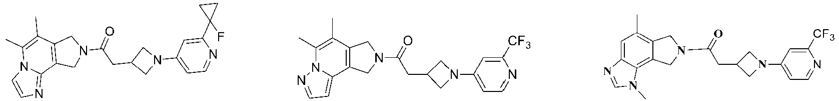

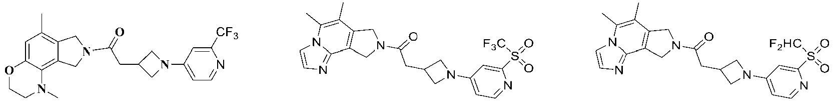

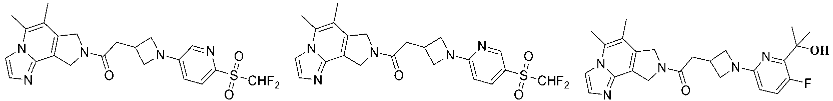

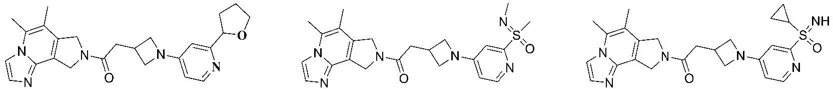

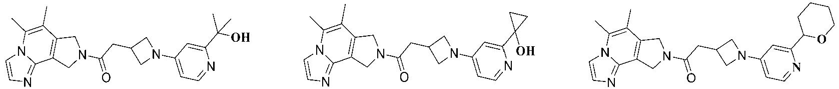

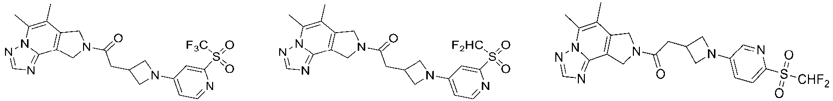

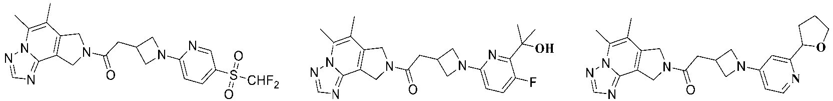

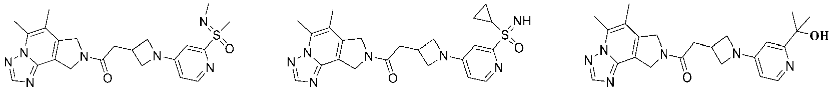

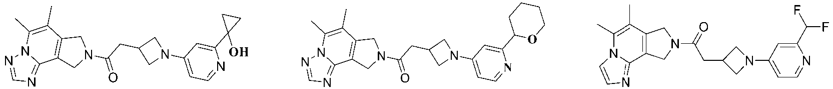

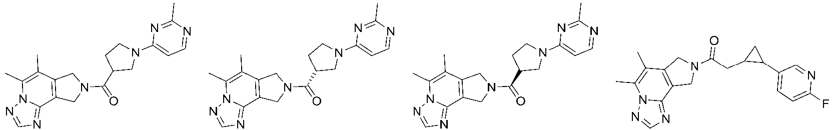

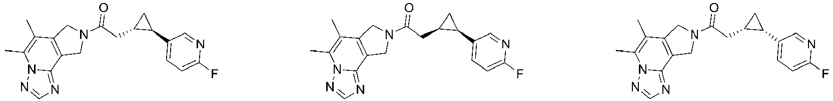

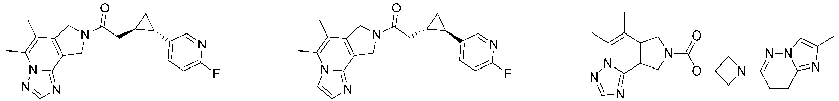

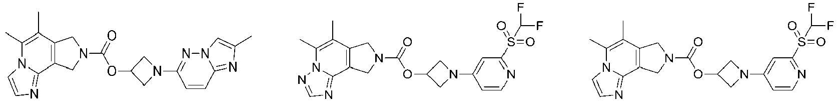

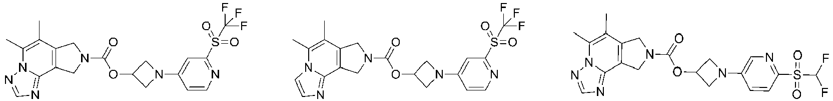

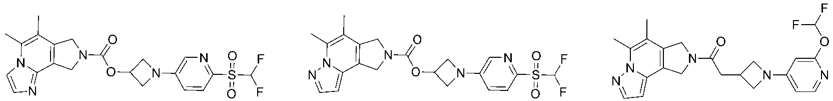

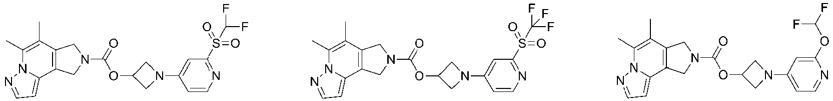

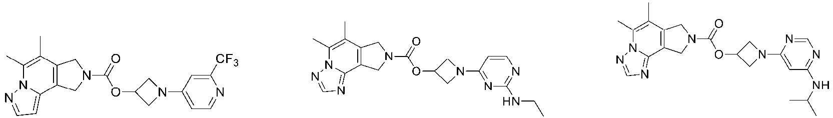

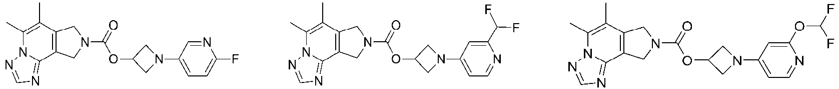

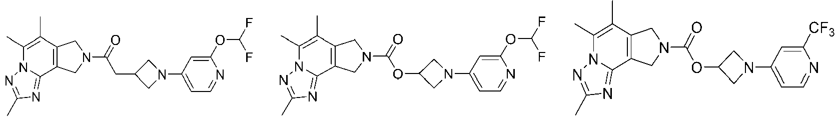

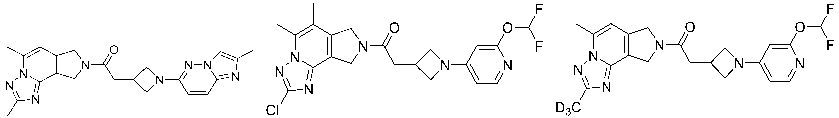

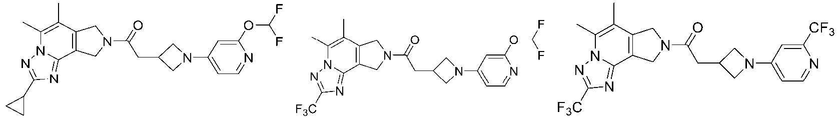

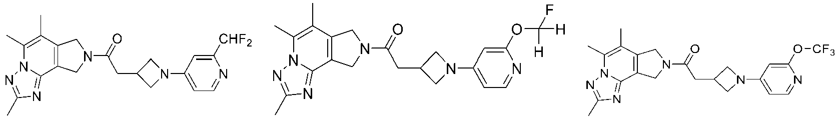

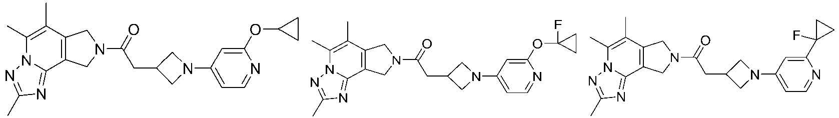

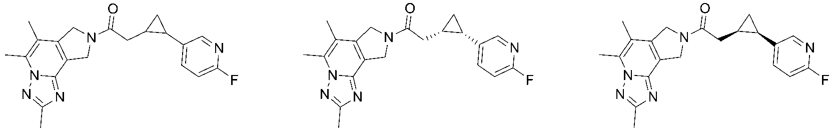

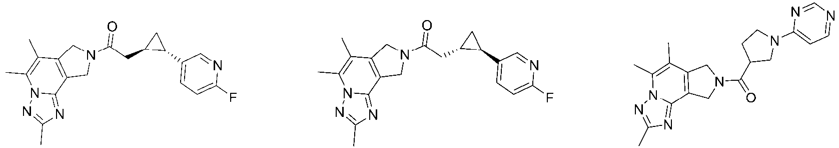

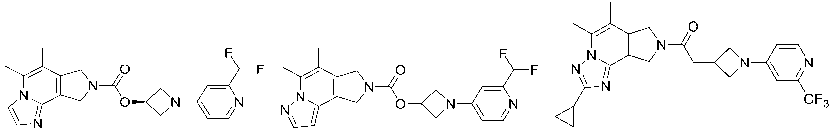

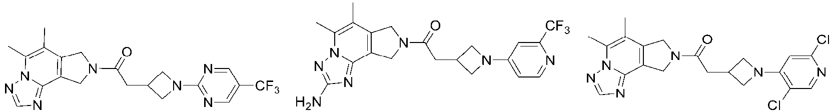

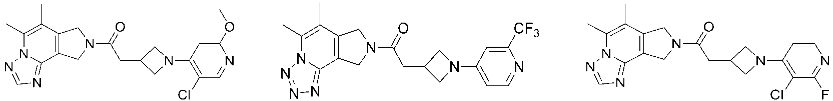

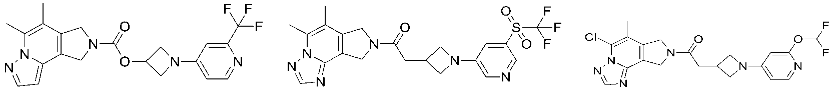

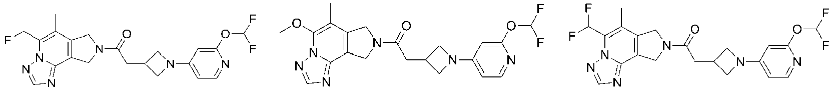

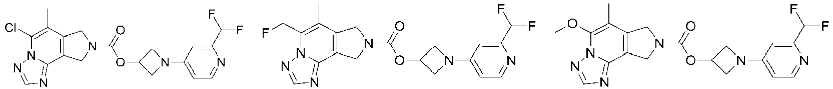

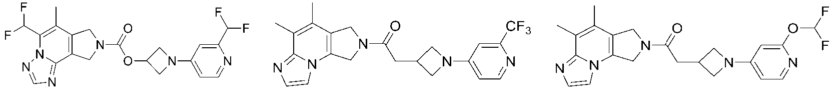

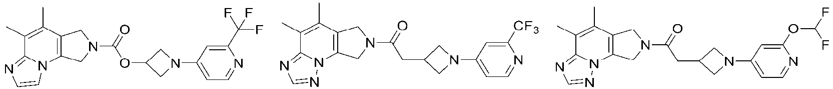

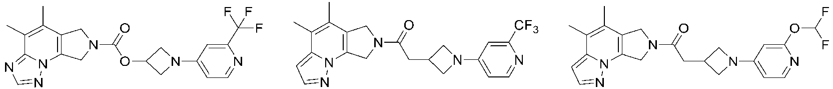

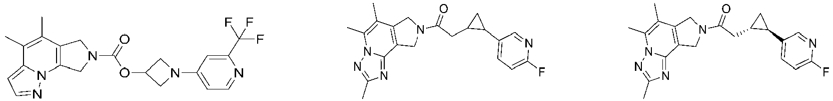

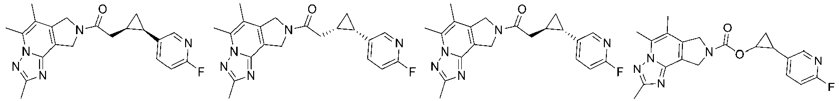

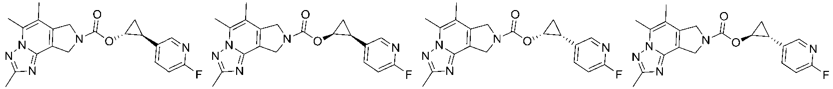

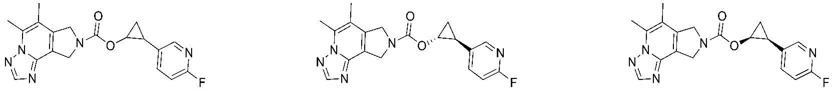

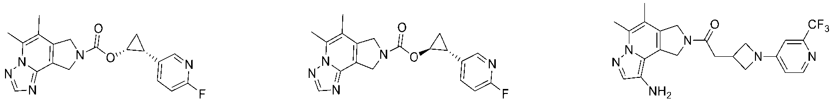

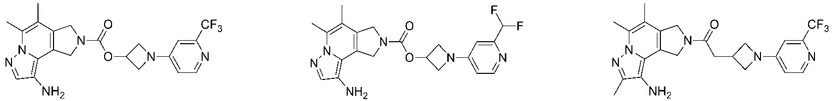

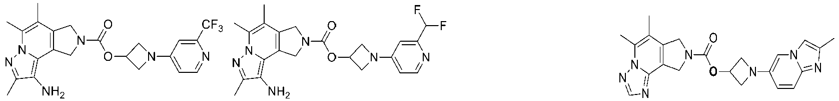

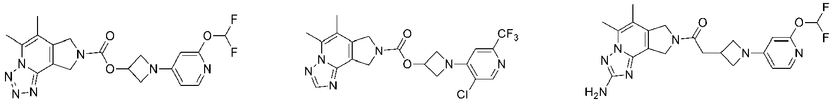

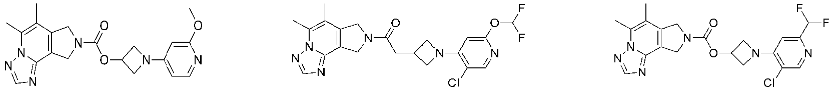

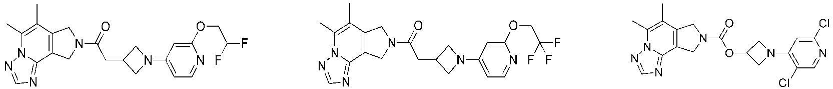

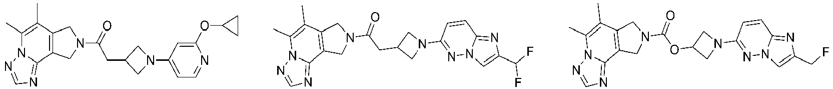

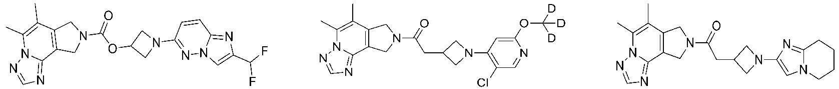

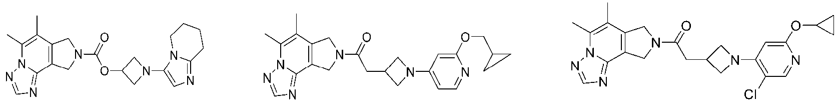

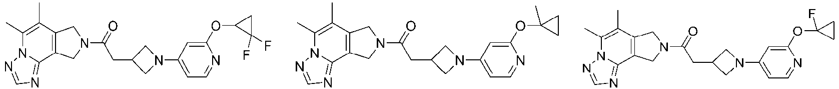

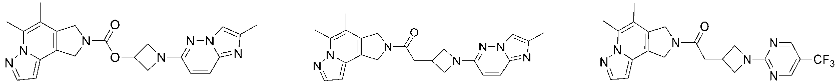

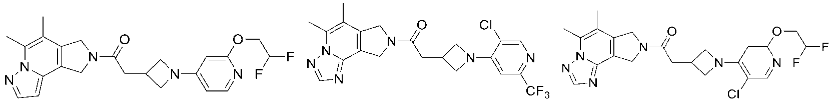

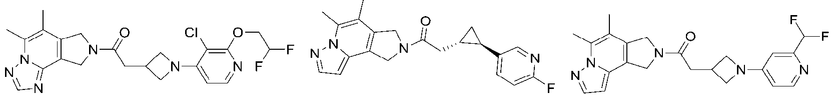

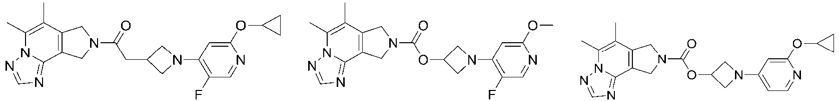

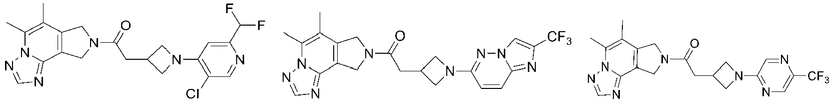

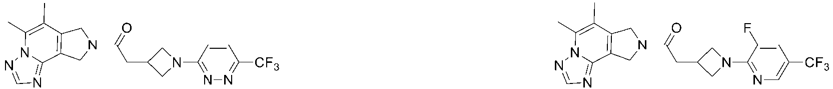

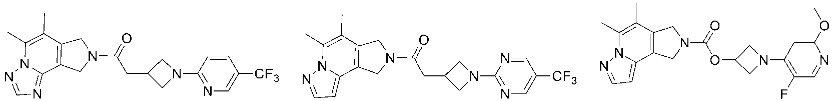

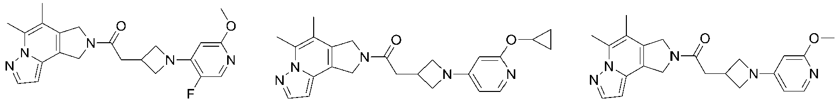

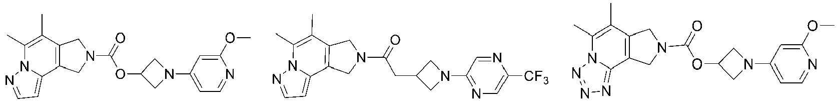

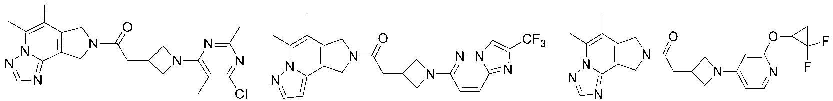

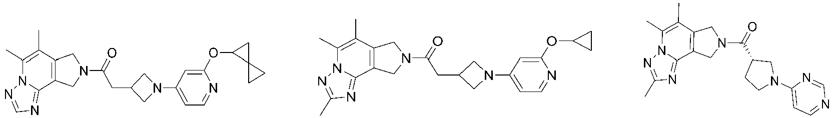

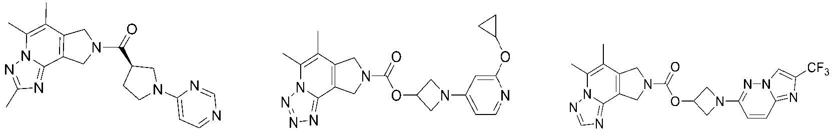

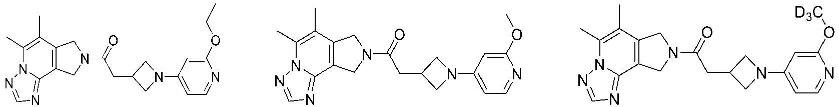

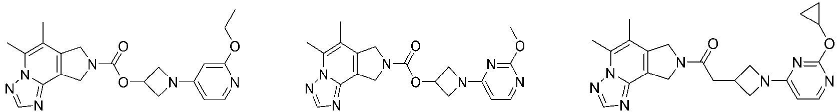

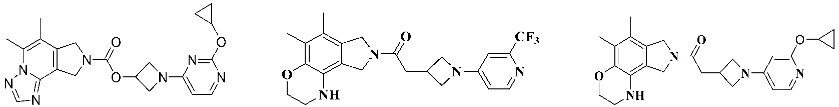

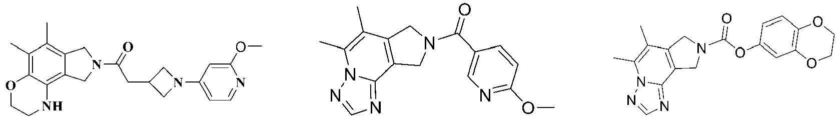

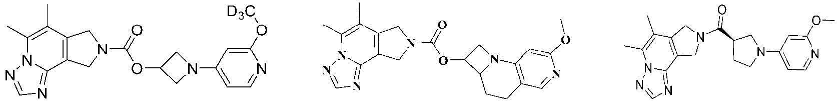

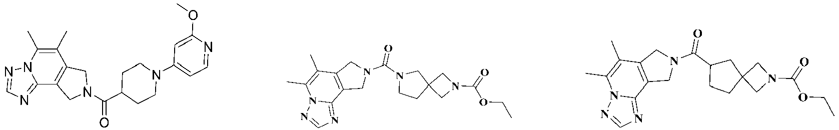

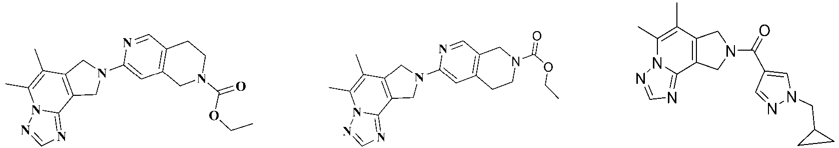

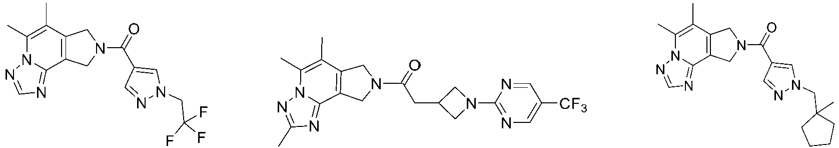

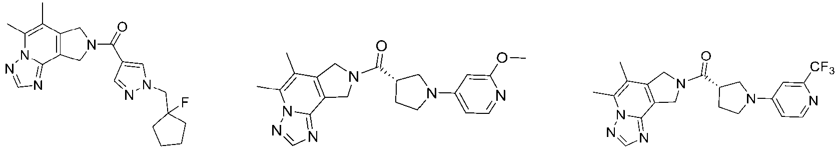

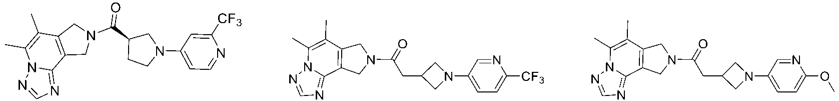

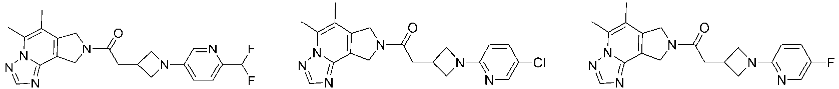

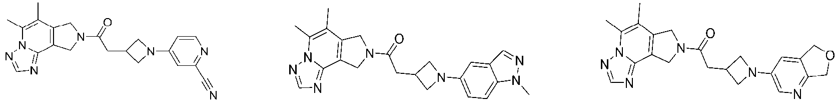

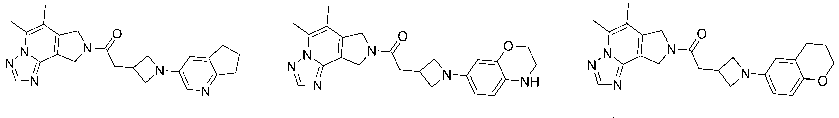

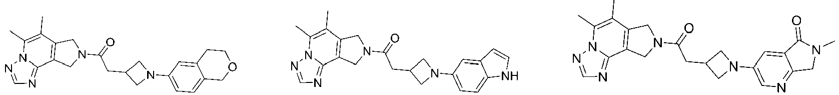

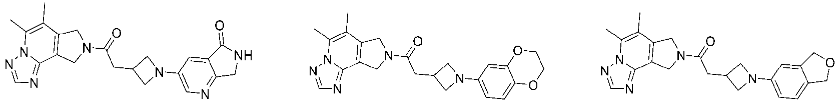

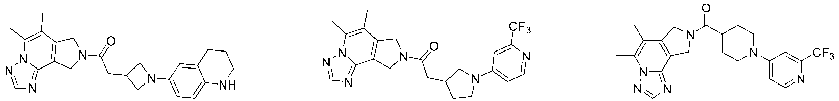

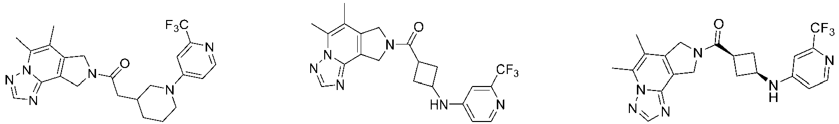

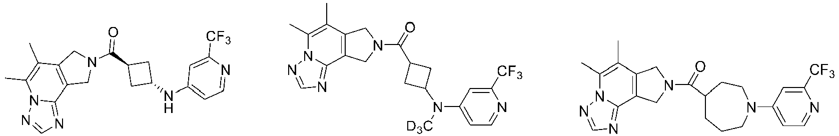

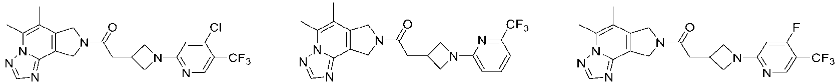

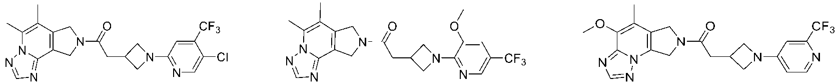

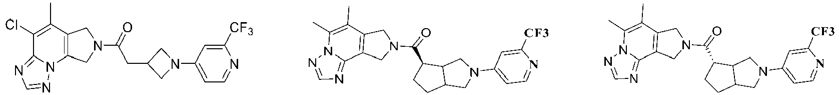

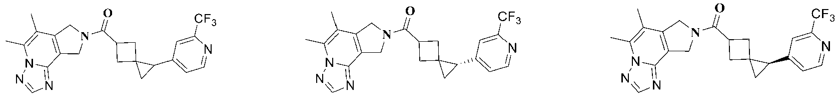

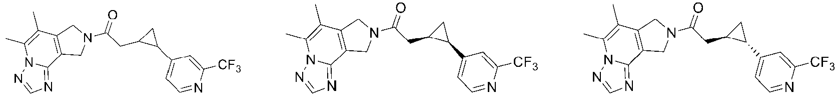

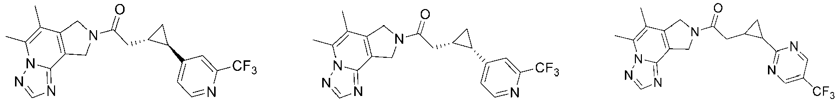

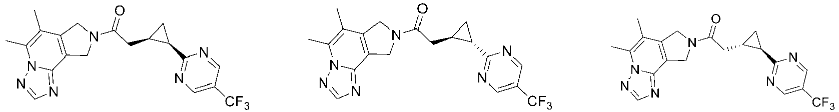

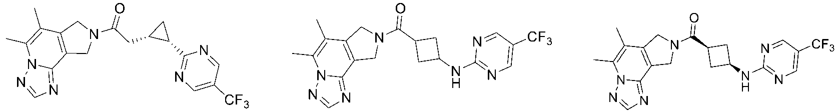

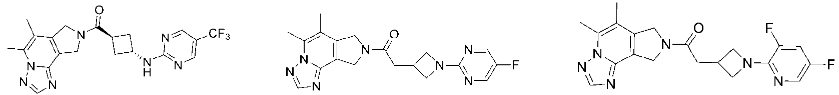

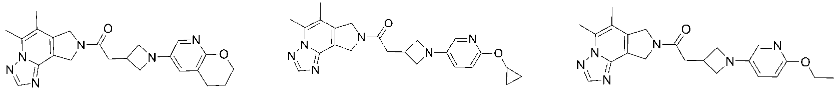

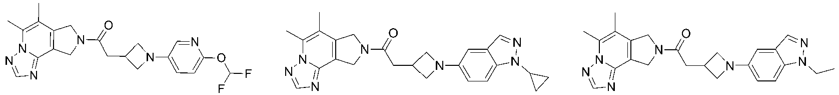

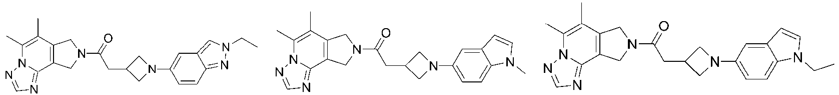

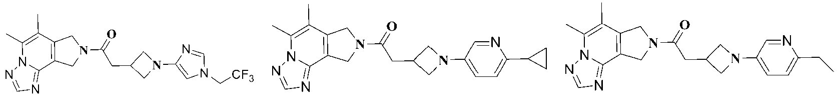

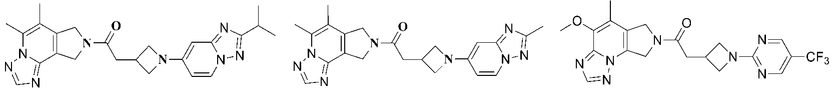

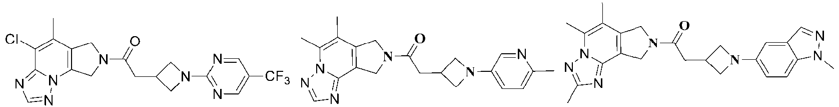

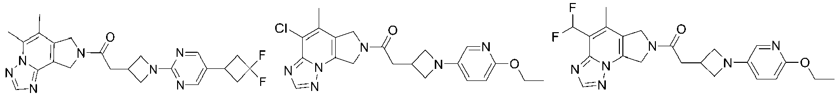

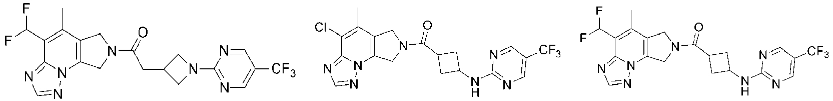

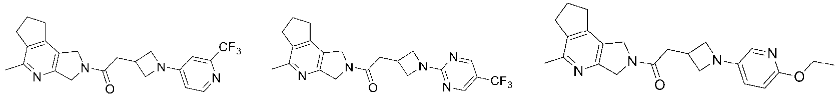

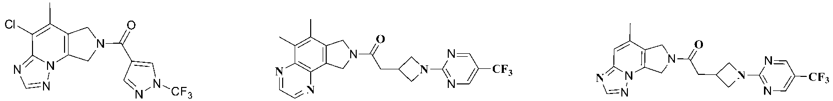

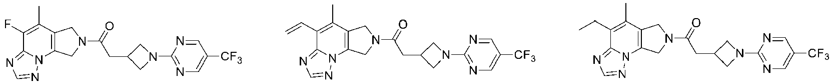

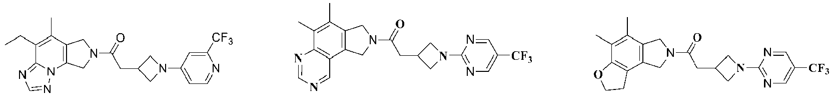

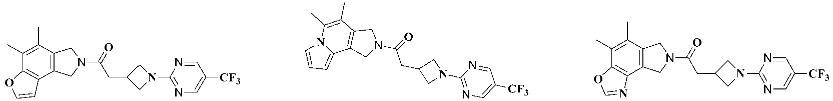

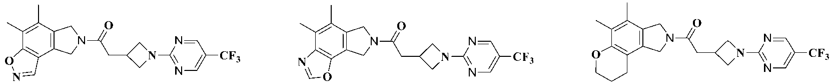

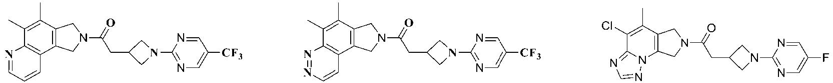

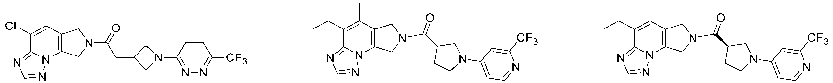

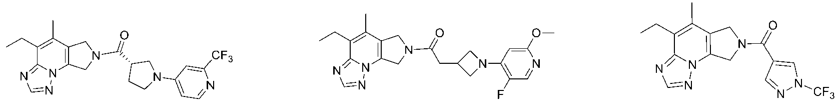

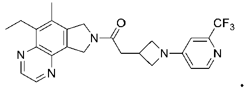

20. A method for preparing the compound represented by general formula (II-A-a4), or the stereoisomer or pharmaceutically acceptable salt thereof according to claim 5, **characterised by** comprising the following step: 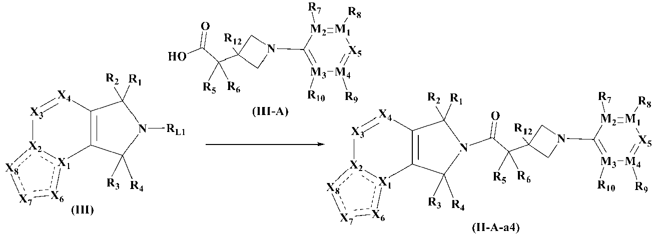
reacting a compound represented by general formula (III), or a stereoisomer or pharmaceutically acceptable salt thereof, with a compound represented by general formula (III-A), to afford general formula (II-A-a4);
wherein preferably, the reaction is carried out in a condensing reagent, wherein the condensing reagent is preferably 2-(7-azabenzotriazol-1-yl)-N,N,N',N'-tetramethyluronium hexafluorophosphate, 1-(3-dimethylaminopropyl)-3-ethylcarbodiimide hydrochloride, benzotriazol-1-yl-N,N,N',N'-tetramethyluronium hexafluorophosphate, 1-propylphosphonic anhydride, O-(benzotriazol-1-yl)-N,N,N',N'-tetramethyluronium tetrafluoroborate, N,N'-carbonyldiimidazole, N,N'-dicyclohexylcarbodiimide, N,N'-diisopropylcarbodiimide, 1H-benzotriazol-1-yloxytripyrrolidinophosphonium hexafluorophosphate, benzotriazol-1-yloxytris(dimethylamino)phosphonium hexafluorophosphate, (ethyl 2-cyano-2-(hydroxyimino)acetate)-N,N-dimethyl-morpholinourea hexafluorophosphate, N,N,N',N'-tetramethylchloroformamidinium hexafluorophosphate, 2-hydroxypyridine-N-oxide, tripyrrolidinophosphonium bromide hexafluorophosphate, bis(2-oxo-3-oxazolidinyl)phosphinic chloride, 2-chloro-1-methylpyridinium iodide, diethyl cyanophosphonate, 4-(4,6-dimethoxy-1,3,5-triazin-2-yl)-4-methylmorpholinium chloride, 2-succinimido-1,1,3,3-tetramethyluronium tetrafluoroborate, 2-ethoxy-1-ethoxycarbonyl-1,2-dihydroquinoline, 2-chloro-4,6-dimethoxy-1,3,5-triazine, ethyl 2-cyano-2-(hydroxyimino)acetate, diphenyl phosphorazidate, bis(pentafluorophenyl)carbonate, or 3-(diethoxyphosphoryloxy)-1,2,3-benzotriazin-4-one;
more preferably, the reaction is carried out in a base, wherein the base is preferably diisopropylethylamine, N-methylmorpholine, N-methylimidazole, 4-dimethylaminopyridine, 2,6-dimethylpyridine, potassium carbonate, potassium tert-butoxide, sodium tert-butoxide, 1,8-diazabicyclo[5.4.0]undec-7-ene, trimethylamine, triethylamine, pyridine, piperidine, morpholine, lithium diisopropylamide, lithium diethylamide, lithium bis(trimethylsilyl)amide, lithium isopropylcyclohexylamide, LiHMDS, potassium phosphate, tripotassium phosphate trihydrate, tripotassium phosphate dihydrate, tripotassium phosphate monohydrate, potassium acetate, sodium acetate, sodium bicarbonate, potassium bicarbonate, sodium carbonate, caesium carbonate, potassium hydroxide, sodium hydroxide, potassium hydride, sodium hydride, lithium hydroxide or 2-tert-butyl-1,1,3,3-tetramethylguanidine and mixtures thereof.

21. A compound represented by general formula (III), or a stereoisomer or pharmaceutically acceptable salt thereof, 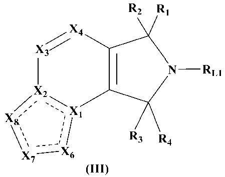 **characterised in that**
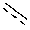 is a single bond or a double bond;
each X₁ is independently selected from a bond, C, CR₁₁ or N;
each X₂ is independently selected from a bond, C, CR₂₂ or N;
each X₃ is independently selected from a bond, C, CR₃₃ or N;
each X₄ is independently selected from a bond, C, CR₄₄ or N;
each X₆, X₇ or X₈ is independently selected from (CR₆₆R₇₇)ₙ₂, NR₈₈, (CR₆₆R₇₇)ₙ₂NR₈₈, NR₈₈(CR₆₆R₇₇)ₙ₂, (CR₆₆)ₙ₂, N or O;
R_{L1} is selected from hydrogen, deuterium or an amino protecting group;
each R₁₁, R₂₂, R₃₃, R₄₄, R₆₆, R₇₇, R₈₈, R₁, R₂, R₃ or R₄ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl is optionally substituted with one or more substituents selected from deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl;
n2 is 1 or 2.

22. The compound, or the stereoisomer or pharmaceutically acceptable salt thereof according to claim 21, **characterised in that** the compound is further represented by general formula (III-1), (III-2), (III-3), (III-4) or (III-5): 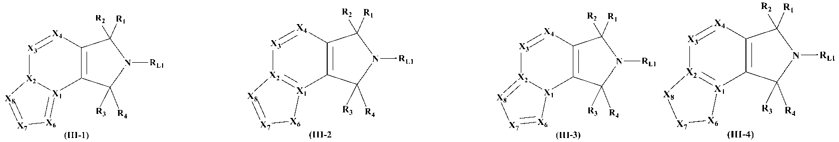 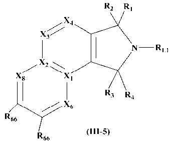
R_{L1} is selected from hydrogen, deuterium or an amino protecting group, wherein the amino protecting group is selected from a benzyl-type protecting group, a carboxylic ester-type protecting group, an acyl protecting group or a silyl protecting group;
preferably, the benzyl-type protecting group is selected from benzyl, 4-methoxybenzyl, 2,4-dimethoxybenzyl, 3,4-dimethoxybenzyl, 3-methoxybenzyl, 3,5-dimethoxybenzyl or 2,4,6-trimethoxybenzyl; the carboxylic ester-type protecting group is selected from allyloxycarbonyl, tert-butoxycarbonyl, benzyloxycarbonyl, trimethylsilylethoxycarbonyl, trichloroethoxycarbonyl, 9-fluorenylmethoxycarbonyl, methoxycarbonyl or ethoxycarbonyl; the acyl protecting group is selected from acetyl, benzoyl, p-methoxybenzylcarbonyl, acetyl, tosyl, p-nitrobenzenesulphonyl, phthaloyl, p-toluenesulphonyl or trifluoroacetyl; the silyl protecting group is selected from tert-butyldimethylsilyl or (trimethylsilyl)ethoxymethyl;
each R₁₁, R₂₂, R₃₃, R₄₄, R₆₆, R₇₇, R₈₈, R₁, R₂, R₃ or R₄ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, hydroxyl, halogen, cyano, amino, C₁₋₆ alkyl, C₁₋₆ haloalkyl or C₁₋₆ deuteroalkyl;
preferably, each R₁₁, R₂₂, R₃₃, R₄₄, R₆₆, R₇₇, R₈₈, R₁, R₂, R₃ or R₄ is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5-to 10-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl;
more preferably, each R₁₁, R₂₂, R₃₃, R₄₄, R₆₆, R₇₇ or R₈₈ is independently selected from hydrogen, deuterium, fluorine, chlorine, bromine, amino, hydroxyl, cyano, nitro, methyl, ethyl, propyl, isopropyl, fluoromethyl, difluoromethyl, trifluoromethyl, vinyl, ethynyl, cyclopropyl, cyclobutyl, epoxyethyl, epoxypropyl or epoxybutyl, wherein the amino, methyl, ethyl, propyl, isopropyl, fluoromethyl, difluoromethyl, trifluoromethyl, vinyl, ethynyl, cyclopropyl, cyclobutyl, epoxyethyl, epoxypropyl or epoxybutyl is optionally substituted with one or more substituents selected from deuterium, hydroxyl, halogen, cyano, amino, C₁₋₃ alkyl, C₁₋₃ haloalkyl or C₁₋₃ deuteroalkyl;
each R₁, R₂, R₃ or R₄ is independently selected from hydrogen, deuterium, fluorine, chlorine, bromine, amino, methylamino, dimethylamino, hydroxyl, cyano, nitro, methyl, ethyl, isopropyl, fluoromethyl, fluoroethyl, difluoromethyl, difluoroethyl, trifluoromethyl, trifluoroethyl, aminomethyl, methoxy, ethoxy or propoxy.

23. A method for preparing the compound represented by general formula (III-1), or the stereoisomer or pharmaceutically acceptable salt thereof according to claim 22, **characterised by** comprising the following step: 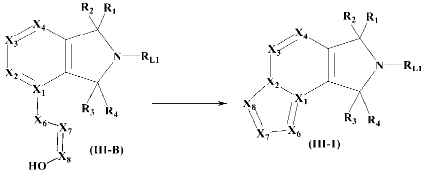
cyclising a compound represented by general formula (III-B), or a stereoisomer or pharmaceutically acceptable salt thereof to afford general formula (III-1);
preferably, the reaction is carried out in a cyclising reagent, wherein the cyclising reagent is preferably trifluoroacetic anhydride, p-toluenesulphonyl chloride, methanesulphonyl chloride, p-trifluoromethylbenzenesulphonyl chloride, 1-propylphosphonic anhydride, acetic anhydride, polyphosphoric acid, Eaton's reagent or boron trifluoride diethyl etherate;
alternatively, a method for preparing the compound represented by general formula (III-3), or the stereoisomer or pharmaceutically acceptable salt thereof, comprising the following step: 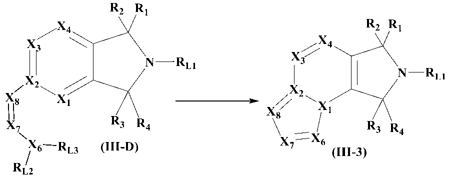
reacting a compound represented by general formula (III-D), or a stereoisomer or pharmaceutically acceptable salt thereof to afford general formula (III-3);
optionally, a method for preparing the compound represented by general formula (III-B), or the stereoisomer or pharmaceutically acceptable salt thereof, comprising the following step: 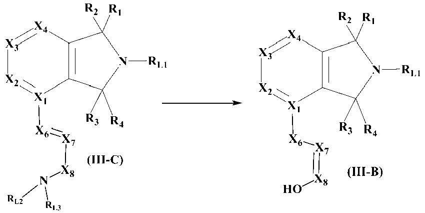
reacting a compound represented by general formula (III-C), or a stereoisomer or pharmaceutically acceptable salt thereof to afford general formula (III-B);
each R_{L2} or R_{L3} is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl is optionally substituted with one or more substituents selected from deuterium, hydroxyl, halogen, cyano, amino, C₁₋₆ alkyl, C₁₋₆ haloalkyl or C₁₋₆ deuteroalkyl;
preferably, each R_{L2} or R_{L3} is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl containing 1 to 3 heteroatoms selected from N, O or S, C₆₋₁₀ aryl or 5- to 10-membered heteroaryl containing 1 to 3 heteroatoms selected from N, O or S is optionally substituted with one or more substituents selected from deuterium, hydroxyl, halogen, cyano, amino, C₁₋₃ alkyl, C₁₋₃ haloalkyl or C₁₋₃ deuteroalkyl.

24. The compound, or the stereoisomer or pharmaceutically acceptable salt thereof according to claim 21 or 22, **characterised in that** the compound has the following structure:

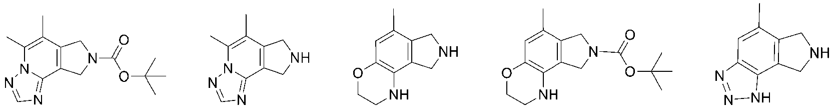

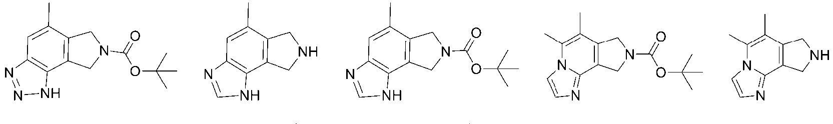

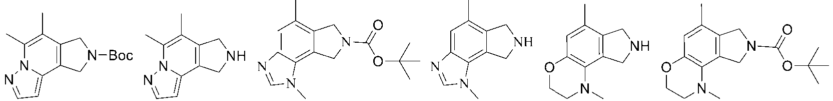

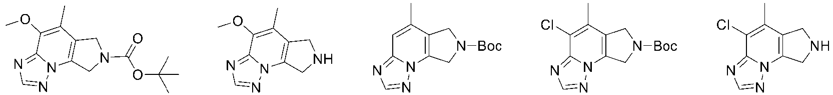

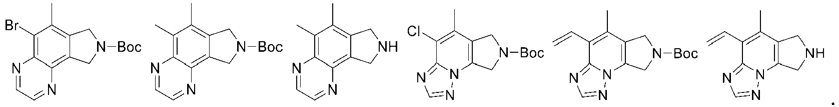

25. A pharmaceutical composition, **characterised by** comprising a therapeutically effective dose of the compound, or the stereoisomer or pharmaceutically acceptable salt thereof according to any one of claims 1-19, and one or more pharmaceutically acceptable carriers or excipients.

26. Use of the compound, or the stereoisomer or pharmaceutically acceptable salt thereof according to any one of claims 1-19 or the pharmaceutical composition according to claim 25 in the preparation of a medicament for treating a disease associated with M4, **characterised in that** preferably, the disease associated with M4 is selected from Alzheimer's disease, schizophrenia or psychosis, pain, addiction, sleep disorder, cognitive impairment, Parkinson's disease or condition, levodopa-induced dyskinesia in Parkinson's disease, Huntington's disease, xerostomia, pulmonary arterial hypertension, chronic obstructive pulmonary disease, asthma, urinary incontinence, glaucoma, Down syndrome, cerebral amyloid angiopathy, dementia, hereditary cerebral hemorrhage with amyloidosis-Dutch type, Creutzfeldt-Jakob disease, prion disease, amyotrophic lateral sclerosis, progressive supranuclear palsy, head trauma, stroke, pancreatitis, inclusion body myositis, other peripheral amyloidosis, diabetes mellitus, autism, and atherosclerosis, more preferably Alzheimer's disease, schizophrenia, pain, addiction, and sleep disorder.
